# EUROPEAN PATENT APPLICATION

(11) **EP 2 033 962 A1**
(43) Date of publication of application: **11.03.2009**
(21) Application number: 07114794.6
(22) Date of filing: 22.08.2007
(51) Int. Cl.: C07D 471/14, A61K 31/4985, A61P 35/00

(54) **Tetracyclic indolopyridines as EG5 inhibitors**

(71) Applicant: 4SC AG, 82152 Planegg - Martinsried (DE)
(72) Inventor: The designation of the inventor has not yet been filed
(74) Representative: Vossius & Partner

(57) **Abstract**

Compounds of a certain formula I, in which R1, R2, R3 and R4 have the meanings indicated in the description, are effective Eg5-inhibiting compounds with anti-proliferative and/or apoptosis inducing activity.

## Description

### Field of application of the invention

The invention relates to tetracyclic indolopyridine derivatives, which can be used in the pharmaceutical industry for the production of pharmaceutical compositions.

### Known technical background

Eg5 (also called kinesin spindle protein) is a protein which is essential for the assembly and function of the bipolar mitotic spindle during cell division and which is a target for the discovery of novel cancer therapies.
In the document Hotha et al., Angew. Chem. 2003, 115, 2481-2484, the indolopyridine compound HR22C16 is described as inhibitor of cell division by targeting Eg5.
In WO9519978, tetracyclic derivatives are described that are PDE5 (phosphodiesterase 5) inhibitors.
In WO2007016361, WO2003000691, WO2002098877, WO2002098428, WO2002036593, WO2002010166, WO2002000658, WO2002000656, WO2001094345, WO199703985 WO199703675 and WO199519978, further PDE5 inhibitors are described.
In WO2006/018435, inter alia indolopyridines are described with Eg5 inhibiting activity.
In WO01/094347, WO2002/098428 and US6143746, tetracyclic compounds are described that are PDE5 inhibitors.
In J. Med. Chem. 2003, 46 (21), 4525-4532, and 4533-4542, indolopyridine derivatives are described with PDE5 inhibitory activity.
In Tetrahedron Lett. 1998, 39, 1291-1294, the solid support synthesis of tetrahydro-β-carboline,2,3-bis-lactams is described.

### Description of the invention

It has now been found, that the indolopyridine derivatives, which are described in detail below, have surprising and advantageous properties. Particularly, they act as inhibitors of the mitotic kinesin Eg5.

The invention relates to compounds of formula I 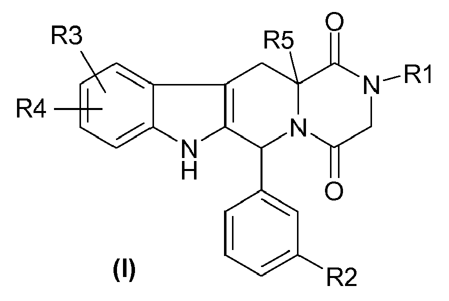 in which
- R1: is 1-4C-alkyl, 3-7C-cycloalkyl, 2-4C-alkenyl, 2-4C-alkinyl, 3-7C-cycloalkyl-1-4C-alkyl, or 2-7C-alkyl substituted by R11, in which
- R11: is -N(R111)R112, hydroxyl or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl, 3-7C-cycloalkyl, or 3-7C-cycloalkyl-1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
- R2: is hydrogen or hydroxyl,
- R3: is hydrogen, 1-4C-alkyl, halogen, 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy,
- R4: is hydrogen, 1-4C-alkyl or halogen,
- R5: is hydrogen or 1-4C-alkyl,
and the salts, stereoisomers and the salts of the stereoisomers of these compounds.

1-4C-Alkyl represents straight-chain or branched alkyl groups having 1 to 4 carbon atoms. Examples which may be mentioned are the n-butyl, isobutyl, sec-butyl, tert-butyl, n-propyl, isopropyl, ethyl and the methyl group, of which ethyl and methyl are preferred.

2-4C-Alkyl represents a straight-chain or branched alkyl group having 2 to 4 carbon atoms. Examples are the n-butyl, isobutyl, sec-butyl, tert-butyl, isopropyl, the n-propyl and ethyl group, of which ethyl and n-propyl are preferred.

2-7C-Alkyl represents a straight-chain or branched alkyl group having 2 to 7 carbon atoms. Examples are the n-heptyl, isoheptyl (5-methylhexyl), n-hexyl, isohexyl (4-methylpentyl), neohexyl (3,3-dimethylbutyl), n-pentyl, isopentyl (3-methylbutyl), neopentyl (2,2-dimethylpropyl), n-butyl, isobutyl, sec-butyl, tert-butyl, isopropyl, n-propyl and ethyl group, of which ethyl and n-propyl are preferred.

The abovementioned alkyl groups may be substituted by R11, e.g. the term 2-(R11)-ethyl represents ethyl which is substituted in 2-position by R11, the term 3-(R11)-n-propyl represents n-propyl which is substituted in 3-position by R11, and the term 4-(R11)-n-butyl represents n-butyl which is substituted in 4-position by R11.

3-7C-Cycloalkyl represents a monocyclic saturated aliphatic hydrocarbon group having 3 to 7 carbon atoms. Examples are the cyclopropyl, cyclobutyl, cyclopentyl, cyclohexyl and cycloheptyl, of which cyclopropyl, cyclobutyl and cyclopentyl are preferred.

3-7C-Cycloalkyl-1-4C-alkyl represents one of the aforementioned 1-4C-alkyl groups, which is substituted by one of the aforementioned 3-7C-cycloalkyl groups. Examples which may be mentioned are the cyclopropylmethyl, the cyclohexylmethyl and the cyclohexylethyl group, of which cyclopropylmethyl is preferred.

2-4C-Alkenyl represents a straight-chain or branched alkenyl group having 2 to 4 carbon atoms. Examples are the 2-butenyl, 3-butenyl (homoallyl), 1-propenyl, 2-propenyl (allyl) and the ethenyl (vinyl) group.

2-4C-Alkinyl represents a straight-chain or branched alkinyl group having 2 to 4 carbon atoms. Examples are the 2-butinyl, 3-butinyl (homopropargyl), 1-propinyl, 2-propinyl (propargyl), 1-methyl-2-propinyl (1-methyl-propargyl) and the ethinyl group.

Halogen within the meaning of the invention includes iodine, bromine, chlorine and fluorine, of which bromine, chlorine and fluorine are preferred.

Hydroxy-2-4C-alkyl denotes abovementioned 2-4C-alkyl groups which are substituted by a hydroxyl group. Examples which may be mentioned are the 2-hydroxyethyl and the 3-hydroxypropyl groups.

1-4C-Alkoxy represents groups, which in addition to the oxygen atom contain a straight-chain or branched alkyl group having 1 to 4 carbon atoms. Examples which may be mentioned are the n-butoxy, isobutoxy, sec-butoxy, tert-butoxy, n-propoxy, isopropoxy, ethoxy and methoxy group, of which methoxy and ethoxy are preferred.

1-4C-Alkoxy-2-4C-alkyl represents one of the aforementioned 2-4C-alkyl groups, which is substituted by one of the aforementioned 1-4C-alkoxy groups. Examples which may be mentioned are the 2-methoxyethyl, the 2-n-butoxyethyl and the 3-methoxypropyl groups.

1-4C-Alkylcarbonyl represents a group, which in addition to the carbonyl group contains one of the aforementioned 1-4C-alkyl groups. An example which may be mentioned is the acetyl group.

As completely or partially fluorine-substituted 1-4C-alkyl, for example, the 2,2,3,3,3-pentafluoropropyl, the perfluoroethyl, the 1,2,2-trifluoroethyl, the 1,1,2,2-tetrafluoroethyl, the 2,2,2-trifluoroethyl, the trifluoromethyl, the difluoromethyl, the monofluoromethyl, the 2-fluoroethyl and the 2,2-difluoroethyl groups may be mentioned, of which 2,2,2-trifluoroethyl, 2,2-difluoroethyl and 2-fluoroethyl is preferred. "Partially" in this connection means that at least one, but not all of the hydrogen atoms of the 1-4C-alkoxy groups is replaced by fluorine atoms. Preferred are the trifluoromethyl and the difluoromethyl group.

Completely or predominantly fluorine-substituted 1-4C-alkoxy is, for example, the 2,2,3,3,3-pentafluoropropoxy, the perfluoroethoxy, the 1,2,2-trifluoroethoxyand in particular the 1,1,2,2-tetrafluoroethoxy, the 2,2,2-trifluoroethoxy, the trifluoromethoxy and the difluoromethoxy group, of which the trifluoromethoxy and the difluoromethoxy groups are preferred. "Predominantly" in this connection means that more than half of the hydrogen atoms of the 1-4C-alkoxy groups are replaced by fluorine atoms.

1-4C-Alkoxy-2-4C-alkoxy represents one of the abovementioned 2-4C-alkoxy groups, which is substituted by one of the abovementioned 1-4C-alkoxy groups. Examples which may be mentioned are the 2-methoxyethoxy, 2-ethoxyethoxy and the 2-isopropoxyethoxy groups.

Hydroxy-2-4C-alkoxy represents one of the abovementioned 2-4C-alkoxy groups, which is substituted by a hydroxyl group. Examples which may be mentioned are the 2-hydroxyethoxy and the 3-hydroxypropoxy groups.

3-7C-Cycloalkoxy represents cyclopropyloxy, cyclobutyloxy, cyclopentyloxy, cyclohexyloxy and cycloheptyloxy, of which cyclopropyloxy, cyclobutyloxy and cyclopentyloxy are preferred.

3-7C-Cycloalkyl-1-4C-alkoxy represents one of the abovementioned 1-4C-alkoxy groups substituted by one of the abovementioned 3-7C-cycloalkyl groups. Examples which may be mentioned are the 3-7C-cycloalkylmethoxy groups, such as for example cyclopropylmethoxy, cyclobutylmethoxy or cyclopentylmethoxy, of which cyclopropylmethoxy is in particular to be mentioned.

In general and unless otherwise mentioned, the heterocyclic groups include all the possible isomeric forms thereof, e.g. the positional isomers thereof. Thus, for example, the term triazol-1-yl includes [1,2,3]triazol-1-yl, [1,3,4]triazol-1-yl and [1,2,4]triazol-1-yl, or the term isoxazolyl includes isoxazol-3-yl, isoxazol-4-yl and isoxazol-5-yl.

1N-(1-4C-alkyl)-pyrazolyl or 1N-(H)-pyrazolyl, respectively, represents a pyrazolyl group which is substituted on the ring nitrogen atom in 1-position with 1-4C-alkyl or hydrogen, respectively; such as especially the 1-methyl-pyrazol-5-yl or 1-methyl-pyrazol-3-yl group.

2N-(1-4C-alkyl)-pyrazolyl or 2N-(H)-pyrazolyl, respectively, represents a pyrazolyl group which is substituted on the ring nitrogen atom in 2-position with 1-4C-alkyl or hydrogen, respectively; such as especially the 2-methyl-pyrazol-5-yl or 2-methyl-pyrazol-3-yl group.

4N-(R113)-piperazin-1-yl or 4N-(R113)-homopiperazin-1-yl stands for a piperazin-1-yl or homopiperazin-1-yl group, respectively, which is substituted by R113 on the ring nitrogen atom in 4-position.

Constituents which are optionally substituted as stated herein, may be substituted, unless otherwise noted, at any possible position.

Unless otherwise noted, rings containing quaternizable amino- or imino-type ring nitrogen atoms (-N=) may be preferably not quaternized on these amino- or imino-type ring nitrogen atoms by the mentionned substituents or parent molecular groups.

When any variable occurs more than one time in any constituent, each definition is independent.

In a preferred embodiment, the invention relates to compounds of formula I, in which
- R1: is 2-7C-alkyl substituted by R11, in which
- R11: is -N(R111)R112, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl, 3-7C-cycloalkyl, or 3-7C-cycloalkyl-1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
- R2: is hydrogen or hydroxyl,
- R3: is 1-4C-alkoxy,
- R4: is hydrogen, 1-4C-alkyl or halogen,
- R5: is hydrogen or 1-4C-alkyl,
or a salt, stereoisomer or a salt of a stereoisomer thereof.

In a preferred embodiment, the invention relates to compounds of formula I, in which
- R1: is 1-4C-alkyl, 3-7C-cycloalkyl, 2-4C-alkenyl, 2-4C-alkinyl, 3-7C-cycloalkyl-1-4C-alkyl, or 2-7C-alkyl substituted by R11, in which
- R11: is -N(R111)R112, hydroxyl or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl, 3-7C-cycloalkyl, or 3-7C-cycloalkyl-1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
- R2: is hydrogen or hydroxyl,
- R3: is 1-4C-alkoxy,
- R4: is hydrogen, and
- R5: is hydrogen or 1-4C-alkyl,
and the salts, stereoisomers and the salts of the stereoisomers of these compounds.

In a further preferred embodiment, the invention relates to compounds of formula I, in which
- R1: is 3-7C-cycloalkyl, 2-4C-alkenyl, 2-4C-alkinyl, 3-7C-cycloalkyl-1-4C-alkyl, or 2-7C-alkyl substituted by R11, in which
- R11: is -N(R111)R112, hydroxyl or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl, 3-7C-cycloalkyl, or 3-7C-cycloalkyl-1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
- R2: is hydrogen or hydroxyl,
- R3: is hydrogen, 1-4C-alkyl, halogen, 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy,
- R4: is hydrogen, 1-4C-alkyl or halogen,
- R5: is 1-4C-alkyl,
or a salt, stereoisomer or a salt of a stereoisomer thereof.

In a further preferred embodiment, the invention relates to compounds of formula I, in which
- R1: is 1-4C-alkyl, 3-7C-cycloalkyl, 2-4C-alkenyl, 2-4C-alkinyl, 3-7C-cycloalkyl-1-4C-alkyl, or 2-7C-alkyl substituted by R11, in which
- R11: is -N(R111)R112, hydroxyl or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl, 3-7C-cycloalkyl, or 3-7C-cycloalkyl-1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
- R2: is hydrogen or hydroxyl,
- R3: is 1-4C-alkoxy,
- R4: is hydrogen, 1-4C-alkyl or halogen,
- R5: is 1-4C-alkyl,
or a salt, stereoisomer or a salt of a stereoisomer thereof.

In a further preferred embodiment, the invention relates to compounds of formula I, in which
- R1: is 2-7C-alkyl substituted by R11, in which
- R11: is -N(R111)R112, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl, 3-7C-cycloalkyl, or 3-7C-cycloalkyl-1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
- R2: is hydrogen or hydroxyl,
- R3: is hydrogen, 1-4C-alkyl, halogen, 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy,
- R4: is hydrogen, 1-4C-alkyl or halogen,
- R5: is 1-4C-alkyl,
or a salt, stereoisomer or a salt of a stereoisomer thereof.

In a further preferred embodiment, the invention relates to compounds of formula I, in which
- R1: is 2-7C-alkyl substituted by R11, in which
- R11: is -N(R111)R112, or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, or 1-4C-alkylcarbonyl, and
- R2: is hydrogen or hydroxyl,
- R3: is hydrogen, 1-4C-alkyl, halogen, 1-4C-alkoxy, or completely or predominantly fluorine-substituted 1-4C-alkoxy,
- R4: is hydrogen or halogen,
- R5: is 1-4C-alkyl,
or a salt, stereoisomer or a salt of a stereoisomer thereof.

In a further preferred embodiment, the invention relates to compounds of formula I, in which
- R1: is 2-7C-alkyl substituted by R11, in which
- R11: is -N(R111)R112, or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen or 1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl or 1-4C-alkylcarbonyl, and
- R2: is hydroxyl,
- R3: is hydrogen, 1-4C-alkyl, halogen, 1-4C-alkoxy, or completely or predominantly fluorine-substituted 1-4C-alkoxy,
- R4: is hydrogen or halogen,
- R5: is 1-4C-alkyl,
or a salt, stereoisomer or a salt of a stereoisomer thereof.

In a further preferred embodiment, the invention relates to compounds of formula I, in which
- R1: is 2-7C-alkyl substituted by R11, in which
- R11: is -N(R111)R112, or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen or 1-4C-alkyl
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl or 1-4C-alkylcarbonyl, and
- R2: is hydrogen,
- R3: is hydrogen, 1-4C-alkyl, halogen, 1-4C-alkoxy, or completely or predominantly fluorine-substituted 1-4C-alkoxy,
- R4: is hydrogen or halogen,
- R5: is 1-4C-alkyl,
or a salt, stereoisomer or a salt of a stereoisomer thereof.

In a further preferred embodiment, the invention relates to compounds of formula I, in which
- R1: is 3-7C-cycloalkyl, 2-4C-alkenyl, 2-4C-alkinyl, 3-7C-cycloalkyl-1-4C-alkyl, or 2-7C-alkyl substituted by R11, in which
- R11: is -N(R111)R112, hydroxyl or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl, 3-7C-cycloalkyl, or 3-7C-cycloalkyl-1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
- R2: is hydrogen or hydroxyl,
- R3: is hydrogen, 1-4C-alkyl, halogen, 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy,
- R4: is hydrogen, 1-4C-alkyl or halogen,
- R5: is hydrogen,
or a salt, stereoisomer or a salt of a stereoisomer thereof.

In a further preferred embodiment, the invention relates to compounds of formula I, in which
- R1: is 2-7C-alkyl substituted by R11, in which
- R11: is -N(R111)R112, or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen, 1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl, or 1-4C-alkylcarbonyl, and
- R2: is hydrogen or hydroxyl,
- R3: is hydrogen, 1-4C-alkyl, halogen, 1-4C-alkoxy, or completely or predominantly fluorine-substituted 1-4C-alkoxy,
- R4: is hydrogen or halogen,
- R5: is hydrogen,
or a salt, stereoisomer or a salt of a stereoisomer thereof.

In a further preferred embodiment, the invention relates to compounds of formula I, in which
- R1: is 2-7C-alkyl substituted by R11, in which
- R11: is -N(R111)R112, or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen or 1-4C-alkyl
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl or 1-4C-alkylcarbonyl, and
- R2: is hydroxyl,
- R3: is hydrogen, 1-4C-alkyl, halogen, 1-4C-alkoxy, or completely or predominantly fluorine-substituted 1-4C-alkoxy,
- R4: is hydrogen or halogen,
- R5: is hydrogen,
or a salt, stereoisomer or a salt of a stereoisomer thereof.

In a further preferred embodiment, the invention relates to compounds of formula I, in which
- R1: is 2-7C-alkyl substituted by R11, in which
- R11: is -N(R111)R112, or halogen, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
- R112: is hydrogen or 1-4C-alkyl
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
- R113: is hydrogen, 1-4C-alkyl or 1-4C-alkylcarbonyl, and
- R2: is hydrogen,
- R3: is hydrogen, 1-4C-alkyl, halogen, 1-4C-alkoxy, or completely or predominantly fluorine-substituted 1-4C-alkoxy,
- R4: is hydrogen or halogen,
- R5: is hydrogen,
or a salt, stereoisomer or a salt of a stereoisomer thereof.

In a further preferred embodiment, the invention relates to compounds of formula I, in which
- R1: is ethyl or n-propyl, which is substituted by -N(R111)R112, in which
- R111: is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, completely or partially fluorine-substituted 1-4C-alkyl,
- R112: is hydrogen, 1-4C-alkyl,
- or R111 and R112: together and with inclusion of the nitrogen atom, to which they are bonded, form a ring Het, in which
- Het: is optionally substituted by one or two substituents independently selected from 1-4C-alkyl and fluorine, and is piperidin-1-yl, morpholin-4-yl, pyrrolidin-1-yl, azetidin-1-yl, azepan-1-yl, 4N-(R113)-piperazin-1-yl, 2,5-dihydro-pyrrol-1-yl, imidazol-1-yl, in which
- R113: is 1-4C-alkyl, or 1-4C-alkylcarbonyl,
- R3: is hydrogen, halogen, 1-4C-alkoxy,
- R4: is hydrogen, and in which
either
- R2: is hydroxyl, and
- R5: is hydrogen,
or
- R2: is hydroxyl, and
- R5: is 1-4C-alkyl,
or
- R2: is hydrogen, and
- R5: is hydrogen,
or
- R2: is hydrogen, and
- R5: is 1-4C-alkyl,
or a salt, stereoisomer or a salt of a stereoisomer thereof.

In a further preferred embodiment, the invention relates to compounds of formula I, in which
- R1: is 2-7C-alkyl substituted by -N(R111)R112, in which
- R111: is 1-4C-alkyl,
- R112: is hydrogen or 1-4C-alkyl,
- or R111 and R112: together and with inclusion of the nitrogen atom, to which they are bonded, form a ring Het, in which
- Het: is piperidin-1-yl, morpholin-4-yl or pyrrolidin-1-yl,
- R3: is hydrogen, chloro or methoxy,
- R4: is hydrogen,
either
- R2: is hydroxyl, and
- R5: is hydrogen,
or
- R2: is hydroxyl, and
- R5: is 1-4C-alkyl,
or
- R2: is hydrogen, and
- R5: is hydrogen,
or
- R2: is hydrogen, and
- R5: is 1-4C-alkyl,
or a salt, stereoisomer or a salt of a stereoisomer thereof.

Salts of the compounds according to the invention include all inorganic and organic acid addition salts and salts with bases, depending on the substitution. Preferred are the pharmaceutically acceptable inorganic and organic acid addition salts and salts with bases, particularly all pharmaceutically acceptable inorganic and organic acid addition salts and salts with bases customarily used in pharmacy. Acids used for the preparation of the acid addition salts include, but are not limited to (1) inorganic acids, (2) carboxylic acids, (a) aliphatic, alicyclic, saturated or unsaturated carboxylic acids, (b) aromatic or heterocyclic carboxylic acids, (c) hydroxylated or carbohydrate-derived carboxylic acids, and (3) sulfonic acids.
Suitable salts include water-insoluble and, particularly, water-soluble acid addition salts and salts with bases.
Examples of acid addition salts include, but are not limited to, hydrochlorides, sulfates, phosphates, hydrobromides, nitrates, acetates, trifluoroacetates, succinates, oxalates, maleates, fumarates, butyrates, stearates, laurates, benzoates, embonates, salicylates, sulphosalicylates, 2-(4-hydroxybenzoyl)benzoates, 3-hydroxy-2-naphthoates, citrates, tartarates such as (+)-L-tartarates or (-)-D-tartarates or meso-tartarates, lactates such as D-lactates or L-lactates, malates such as (-)-L-malates or (+)-D-malates, D-gluconates, D-glucuronates, lactobionates (salts of 4-O-beta-D-galactopyranosyl-D-gluconic acid), galactarates, tosilates, mesilates, besilates, laurylsulfonates, and ascorbates.
Of these acid addition salts, hydrochlorides, sulfates, acetates, mesilates, citrates, maleates, oxalates, fumarates and tartarates are preferred. Most preferred are the salts selected from hydrochlorides, mesylates, tartrates and citrates.

In one embodiment of this invention, salts of compounds of formula I include a hydrochloride of a compound of formula I.

In another embodiment of this invention, salts of compounds of formula I include a hydrochloride, phosphate, citrate, tartrate, mesylate, tosylate and sulfate of a compound of formula I.

Examples of salts with bases include, but are not limited to, lithium, sodium, potassium, calcium, aluminium, magnesium, titanium, ammonium, meglumine and guanidinium salts.

The compounds of formula I according to this invention, and the salts, stereoisomers and the salts of the stereoisomers of these compounds, may contain, e.g. when isolated in crystalline form, varying amounts of solvents. Included within the scope of the invention are therefore all solvates of the compounds of formula I, and the salts, the stereoisomers and the salts thereof. Hydrates are a preferred example of said solvates.

The substituents R3 and R4 may be attached, unless otherwise noted, at any position of the benzene moiety of the scaffold, wherein preference is given to the attachement of none of R3 and R4 to the 8-position of the scaffold. In one embodiment, R3 is attached in the 5-position of the scaffold; in another embodiment, R3 is attached in the 7-position of the scaffold; and in yet another embodiment R3 is attached in the 6-position of the scaffold; wherein, especially, R4 is hydrogen, respectively; or wherein R4 is fluorine, respectively. In a particular embodiment, R3 is attached in the 6-position of the scaffold. In a more particular embodiment, R3 is attached in the 6-position of the scaffold, and R4 is hydrogen. In another embodiment, R3 is attached in the 6-position of the scaffold, and R4 is attached to the 7-position of the scaffold and is fluorine. In yet another embodiment, R3 is attached in the 6-position of the scaffold, and R4 is attached to the 5-position of the scaffold and is fluorine.
Numbering:

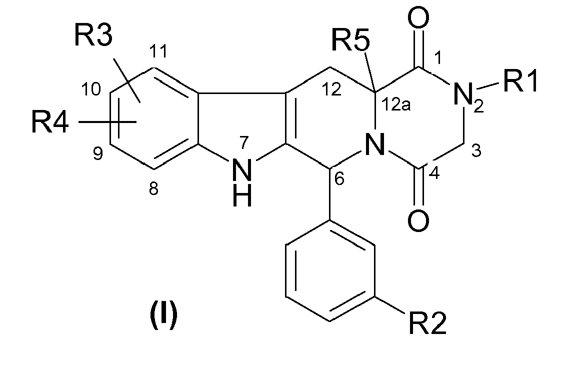

The compounds of formula I are chiral compounds having chiral centers at least in positions 12a and 6. Therefore, the compounds according to the invention and the salts thereof include stereoisomers. Each of the stereogenic centers present in said stereoisomers may have the absolute configuration R or the absolute configuration S (according to the rules of Cahn, Ingold and Prelog). Accordingly, the stereoisomers (6R,12aR), (6R,12aS), (16S,12aR), (6S,12S), wherein the numbers refer to the atoms indicated in formula above, and the salts thereof are part of the invention.

The invention includes all conceivable stereoisomers of compounds of formula I, like e.g. diastereomers and enantiomers. The invention further includes the stereoisomers in pure form as well as all mixtures of the stereoisomers mentioned above independent of the ratio, including the racemates, as well as the salts thereof.

Thus, stereoisomers of the compounds according to this invention, particularly stereoisomers of the following examples, are all part of the present invention and may be obtained according to procedures customary to the skilled person, e.g. by separation of corresponding mixtures, by using stereochemically pure starting materials and/or by stereoselective synthesis, as described in more detail below.

Preference is given hereby to those compounds of formula I, which have with respect to the positions 12a and 6 the same configuration as shown in formula I*:

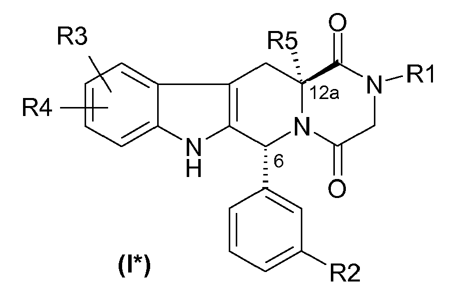

In compounds according to formula I*, in which R5 is hydrogen, methyl or ethyl, the configuration - according to the rules of Cahn, Ingold and Prelog - is S in the 12a position and R in the 6 position.

The enantiomers having the formula I* and the salts thereof are a preferred part of the invention.

Furthermore, also preferred compounds of the formula I are those which have, with respect to the positions 12a and 6, the same configuration as shown in formula I**:

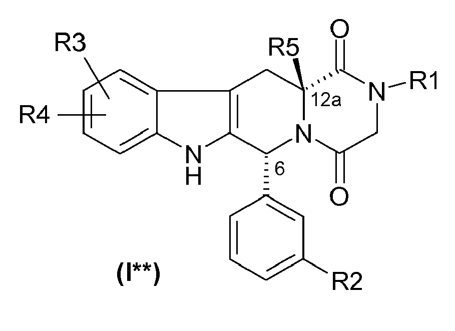

In compounds according to formula I**, in which R5 is hydrogen, methyl or ethyl, the configuration - according to the rules of Cahn, Ingold and Prelog - is R in the 12a position and R in the 6 position.

Further on, compounds of the formula I also to be mentioned are those which have, with respect to the positions 12a and 6, the same configuration as shown in formula I*** or I****:

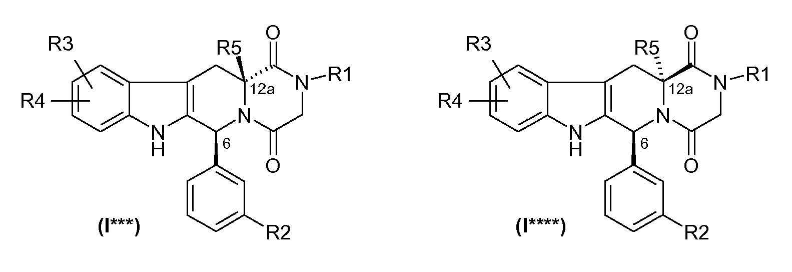

In compounds according to formula I***, in which R5 is hydrogen, methyl or ethyl, the configuration - according to the rules of Cahn, Ingold and Prelog - is R in the 12a position and S in the 6 position.

In compounds according to formula I****, in which R5 is hydrogen, methyl or ethyl, the configuration - according to the rules of Cahn, Ingold and Prelog - is S in the 12a position and S in the 6 position.

A special interest in the compounds according to this invention refers to those compounds of formula I and their salts, stereoisomers and salts of stereoisomers which are included -within the scope of this invention- by one or, when possible, by a combination of more of the following special embodiments:
A special embodiment (embodiment A) of the compounds of formula I according to this invention refers to those compounds of formula I and their salts, stereoisomers and salts of stereoisomers, in which
   - R5: is methyl, and
   - R1, R2, R3, R4: have the meanings as defined herein.
A special embodiment (embodiment B) of the compounds of formula I according to this invention refers to those compounds of formula I and their salts, stereoisomers and salts of stereoisomers, in which
   - R5: is hydrogen, and
   - R1, R2, R3, R4: have the meanings as defined herein.
A special embodiment (embodiment C) of the compounds of formula I according to this invention refers to those compounds of formula I and their salts, stereoisomers and salts of stereoisomers, in which
   - R5: is ethyl, and
   - R1, R2, R3, R4: have the meanings as defined herein.
A special embodiment (embodiment D) of the compounds of formula I according to this invention refers to those compounds of formula land their salts, stereoisomers and salts of stereoisomers, in which
   - R2: is hydroxyl, and
   - R1, R3, R4, R5: have the meanings as defined herein.
A special embodiment (embodiment E) of the compounds of formula I according to this invention refers to those compounds of formula I and their salts, stereoisomers and salts of stereoisomers, in which
   - R2: is hydrogen, and
   - R1, R3, R4, R5: have the meanings as defined herein.
A special embodiment (embodiment 1) of the compounds of formula I according to this invention refers to those compounds of formula I and their salts, stereoisomers and salts of stereoisomers, in which
   - R1: is methyl, and
   - R2, R3, R4, R5: have the meanings as defined herein.
A special embodiment (embodiment 2) of the compounds of formula I according to this invention refers to those compounds of formula I and their salts, stereoisomers and salts of stereoisomers, in which
   - R1: is 2-(R11)-ethyl, and
   - R11, R2, R3, R4, R5: have the meanings as defined herein.
A special embodiment (embodiment 3) of the compounds of formula I according to this invention refers to those compounds of formula I and their salts, stereoisomers and salts of stereoisomers, in which
   - R1: is 2-(N(R111)(R112)-ethyl, and
   - R111, R112, R2, R3, R4, R5: have the meanings as defined herein.
A special embodiment (embodiment 4) of the compounds of formula I according to this invention refers to those compounds of formula I and their salts, stereoisomers and salts of stereoisomers, in which
   - R1: is 2-(N(R111)(R112)-ethyl, and
   - R112: is hydrogen, and
   - R111, R2, R3, R4, R5: have the meanings as defined herein.
A special embodiment (embodiment 5) of the compounds of formula I according to this invention refers to those compounds of formula I and their salts, stereoisomers and salts of stereoisomers, in which
   - R1: is 2-(N(R111)(R112)-ethyl, and
   - R112: is hydrogen, and
   - R111: is 1-4C-alkyl, and
   - R2, R3, R4, R5: have the meanings as defined herein.
A special embodiment (embodiment 6) of the compounds of formula I according to this invention refers to those compounds of formula I and their salts, stereoisomers and salts of stereoisomers, in which
   - R1: is 3-(N(R111)(R112)-n-propyl, and
   - R111, R112, R2, R3, R4, R5: have the meanings as defined herein.
A special embodiment (embodiment 7) of the compounds of formula I according to this invention refers to those compounds of formula I and their salts, stereoisomers and salts of stereoisomers, in which
   - R1: is 3-(N(R111)(R112)-n-propyl, and
   - R112: is hydrogen, and
   - R111, R2, R3, R4, R5: have the meanings as defined herein.
A special embodiment (embodiment 8) of the compounds of formula I according to this invention refers to those compounds of formula I and their salts, stereoisomers and salts of stereoisomers, in which
   - R1: is 3-(N(R111)(R112)-n-propyl, and
   - R112: is hydrogen, and
   - R111: is 1-4C-alkyl, and
   - R2, R3, R4, R5: have the meanings as defined herein.
A special embodiment (embodiment 9) of the compounds of formula I according to this invention refers to those compounds of formula I and their salts, stereoisomers and salts of stereoisomers, in which
   - R1: is 3-(N(R111)(R112)-n-propyl, and
   - R112 and R111: together and with inclusion of the nitrogen atom, to which they are bonded, form a ring Het, and
   - Het, R2, R3, R4, R5: have the meanings as defined herein.
A special embodiment (embodiment 10) of the compounds of formula I according to this invention refers to those compounds of formula I and their salts, stereoisomers and salts of stereoisomers, in which
   - R3: is attached to the 6 position and is methoxy, and
   - R4: is hydrogen, and
   - R1, R2, R5: have the meanings as defined herein.
A special embodiment (embodiment 10a) of the compounds of formula I according to this invention refers to those compounds of formula I and their salts, stereoisomers and salts of stereoisomers, in which
   - R3: is 1-4C-alkoxy, and
   - R4: is hydrogen, and
   - R1, R2, R5: have the meanings as defined herein.
A special embodiment (embodiment 11) of the compounds of formula I according to this invention refers to those compounds of formula I and their salts, stereoisomers and salts of stereoisomers, in which
   - R3: is attached to the 6 position and is ethoxy, and
   - R4: is hydrogen, and
   - R1, R2, R5: have the meanings as defined herein.
A special embodiment (embodiment 12) of the compounds of formula I according to this invention refers to those compounds of formula I and their salts, stereoisomers and salts of stereoisomers, in which
   - R3: is attached to the 6 position and is chloro, and
   - R4: is hydrogen, and
   - R1, R2, R5: have the meanings as defined herein.
A special embodiment (embodiment 13) of the compounds of formula I according to this invention refers to those compounds of formula I and their salts, stereoisomers and salts of stereoisomers, in which
   - R3: is attached to the 6 position and is bromo, and
   - R1, R2, R4, R5: have the meanings as defined herein.
A special embodiment (embodiment 14) of the compounds of formula I according to this invention refers to those compounds of formula I and their salts, stereoisomers and salts of stereoisomers, in which
   - R3: is attached to the 6 position and is difluoromethoxy, and
   - R4: is hydrogen, and
   - R1, R2, R5: have the meanings as defined herein.
A special embodiment (embodiment 15) of the compounds of formula I according to this invention refers to those compounds of formula I and their salts, stereoisomers and salts of stereoisomers, in which
   - R3: is attached to the 6 position and is methoxy, and
   - R4: is fluoro and is attached either to the 5 position or the 7 position, and
   - R1, R2, R5: have the meanings as defined herein.
A special embodiment (embodiment 16) of the compounds of formula I according to this invention refers to those compounds of formula I and their salts, stereoisomers and salts of stereoisomers, in which
   - R3: is attached to the 6 position and is methoxy, and
   - R4: is hydrogen, and
   - R2: is hydroxyl, and
   - R1, R5: have the meanings as defined herein.
A special embodiment (embodiment 17) of the compounds of formula I according to this invention refers to those compounds of formula I and their salts, stereoisomers and salts of stereoisomers, in which
   - R3: is attached to the 6 position and is methoxy, and
   - R4: is hydrogen, and
   - R2: is hydroxyl, and
   - R1, R5: have the meanings as defined herein.
A special embodiment (embodiment 18) of the compounds of formula I according to this invention refers to those compounds of formula I and their salts, stereoisomers and salts of stereoisomers, in which
   - R3: is attached to the 6 position and is methoxy, and
   - R4: is hydrogen, and
   - R2: is hydrogen, and
   - R1, R5: have the meanings as defined herein.
A special embodiment (embodiment 19) of the compounds of formula I according to this invention refers to those compounds of formula I and their salts, stereoisomers and salts of stereoisomers, in which
   - R1: is 1-4C-alkyl, and
   - R3: is attached to the 6 position and is 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy, and
   - R4: is hydrogen, and
   - R2, R5: have the meanings as defined herein.
A special embodiment (embodiment 20) of the compounds of formula I according to this invention refers to those compounds of formula I and their salts, stereoisomers and salts of stereoisomers, in which
   - R1: is 1-4C-alkyl, and
   - R2: is hydroxyl, and
   - R3: is attached to the 6 position and is 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy, and
   - R4: is hydrogen, and
   - R5: has the meanings as defined herein.
A special embodiment (embodiment 20a) of the compounds of formula I according to this invention refers to those compounds of formula I and their salts, stereoisomers and salts of stereoisomers, in which
   - R1: is 1-4C-alkyl, and
   - R2: is hydroxyl, and
   - R3: is attached to the 6 position and is 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy, and
   - R4: is hydroxyl, and
   - R5: has the meanings as defined herein.
A special embodiment (embodiment 21) of the compounds of formula I according to this invention refers to those compounds of formula I and their salts, stereoisomers and salts of stereoisomers, in which
   - R1: is 1-4C-alkyl, and
   - R2: is hydroxyl, and
   - R3: is attached to the 6 position and is 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy, and
   - R4: is hydrogen, and
   - R5: has the meanings as defined herein.
A special embodiment (embodiment 22) of the compounds of formula I according to this invention refers to those compounds of formula I and their salts, stereoisomers and salts of stereoisomers, in which
   - R1: is 2-7C-alkyl substituted by R11, in which R11 has the meanings as defined herein, and
   - R2: is hydroxyl, and
   - R3: is attached to the 6 position and is 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy, and
   - R4: is hydrogen, and
   - R5: has the meanings as defined herein.
A special embodiment (embodiment 23) of the compounds of formula I according to this invention refers to those compounds of formula I and their salts, stereoisomers and salts of stereoisomers, in which
   - R1: is 2-7C-alkyl substituted by R11, in which R11 has the meanings as defined herein, and
   - R2: is hydrogen, and
   - R3: is attached to the 6 position and is 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy, and
   - R4: is hydrogen, and
   - R5: has the meanings as defined herein.
A special embodiment (embodiment 24) of the compounds of formula I according to this invention refers to those compounds of formula I and their salts, stereoisomers and salts of stereoisomers, in which
   - R1: is 2-7C-alkyl substituted by R11, in which R11 has the meanings as defined herein, and
   - R3: is 1-4C-alkoxy, and
   - R2, R4, R5: have the meanings as defined herein.
A special embodiment (embodiment 25) of the compounds of formula I according to this invention refers to those compounds of formula I and their salts, stereoisomers and salts of stereoisomers, in which
   - R3: is 1-4C-alkoxy, and
   - R5: is 1-4C-alkyl, and
   - R1, R2, R4: have the meanings as defined herein.
A special embodiment (embodiment 26) of the compounds of formula I according to this invention refers to those compounds of formula I and their salts, stereoisomers and salts of stereoisomers, in which
   - R3: is 1-4C-alkoxy, and
   - R5: is methyl, and
   - R1, R2, R4: have the meanings as defined herein.
A special embodiment (embodiment 27) of the compounds of formula I according to this invention refers to those compounds of formula I and their salts, stereoisomers and salts of stereoisomers, in which
   - R1: is 2-7C-alkyl substituted by R11, in which R11 has the meanings as defined herein, and
   - R5: is 1-4C-alkyl, and
   - R2, R3, R4: have the meanings as defined herein.
A special embodiment (embodiment 28) of the compounds of formula I according to this invention refers to those compounds of formula I and their salts, stereoisomers and salts of stereoisomers, in which
   - R1: is 2-7C-alkyl substituted by R11, in which R11 has the meanings as defined herein, and
   - R5: is methyl, and
   - R2, R3, R4: have the meanings as defined herein.

Another special embodiment (embodiment 29) of the compounds of formula I according to this invention refers to those compounds which are from formula I* as shown above, and in which R1, R2, R3, R4 have the meanings as defined herein, and their salts.
Another special embodiment (embodiment 30) of the compounds of formula I according to this invention refers to those compounds which are from formula I-AD* or I-AE* as shown below, in which R1 and R3 have the meanings as defined herein, and their salts.

Another special embodiment (embodiment 31) of the compounds of formula I according to this invention refers to those compounds which are from formula I-BD* or I-BE* as shown below, in which R1 and R3 have the meanings as defined herein, and their salts.

Another special embodiment (embodiment 32) of the compounds of formula I according to this invention refers to those compounds which are from formula I* as shown above, in which R1 and R3 have any of the meanings 1.1 to 1.390 indicated in Table 1 given below, and their salts. Another special embodiment (embodiment 33) of the compounds of formula I according to this invention refers to those compounds which are from formula I-AD* as shown below, in which R1 and R3 have any of the meanings 1.1 to 1.390 indicated in Table 1 given below, and their salts.

Another embodiment (embodiment 34) of the compounds of formula I according to this invention refers to those compounds of formula I and their salts, stereoisomers and salts of stereoisomers, in which
- Het: is piperidin-1-yl, morpholin-4-yl, pyrrolidin-1-yl or azetidin-1-yl, and
- R2, R3, R4, R5: have the meanings as defined herein.

Another embodiment (embodiment 35) of the compounds of formula I according to this invention refers to those compounds of formula I and their salts, stereoisomers and salts of stereoisomers, in which
- Het: is 4N-(R113)-piperazin-1-yl, in which
- R113: is hydrogen, methyl, ethyl, isopropyl, cyclopropyl, cyclopropylmethyl, 1-2C-alkylcarbonyl, 2-fluoroethyl, 2,2,2-trifluoroethyl or 2,2-difluoroethyl;
such as e.g. 4-methyl-piperazin-1-yl or 4-acetyl-piperazin-1-yl, and
- R2, R3, R4, R5: have the meanings as defined herein.

A further embodiment (embodiment 36) of the compounds of formula I according to this invention refers to those compounds of formula I and their salts, stereoisomers and salts of stereoisomers, in which
- Het: is optionally substituted by one or two substituents independently selected from methyl and fluorine, and is piperidin-1-yl, pyrrolidin-1-yl, azetidin-1-yl or homopiperidin-1-yl; such as e.g. piperidin-1-yl, pyrrolidin-1-yl or azetidin-1-yl, or 4-methyl-piperidin-1-yl, 4-fluoropiperidin-1-yl, 4,4-difluoro-piperidin-1-yl, (S)-3-fluoro-pyrrolidin-1-yl, (R)-3-fluoro-pyrrolidin-1-yl, 3,3-difluoro-pyrrolidin-1-yl, 3-fluoro-azetidin-1-yl or 3,3-difluoro-azetidin-1-yl, and
- R2, R3, R4, R5: have the meanings as defined herein.

A further embodiment (embodiment 37) of the compounds of formula I according to this invention refers to those compounds of formula I and their salts, stereoisomers and salts of stereoisomers, in which
- Het: is pyrazol-1-yl, imidazol-1-yl or triazol-1-yl, especially imidazol-1-yl, and
- R2, R3, R4, R5: have the meanings as defined herein.

A further embodiment (embodiment 38) of the compounds of formula I according to this invention refers to those compounds of formula I and their salts, stereoisomers and salts of stereoisomers, in which
- Het: is 2,5-dihydro-pyrrol-1-yl or 1,2,3,6-tetrahydropyridin-1-yl, and
- R2, R3, R4, R5: have the meanings as defined herein.

As preferred compounds according to this invention the following compounds of formula I-AD* 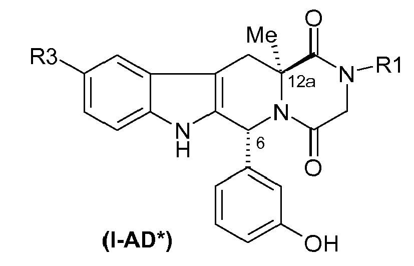 and the salts thereof,
may be mentioned, with the substituents R1 and R3 defined as in the Table 1 given below.

As preferred compounds according to this invention the following compounds of formula I-AE* 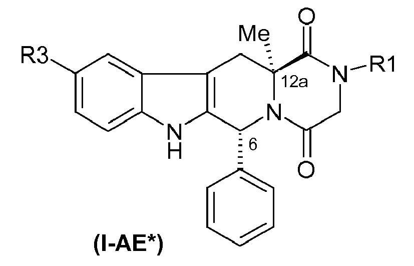 and the salts thereof,
may be mentioned, with the substituents R1 and R3 defined as in the Table 1 given below.

As preferred compounds according to this invention the following compounds of formula I-BD* 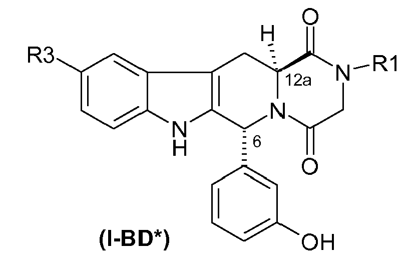 and the salts thereof,
may be mentioned, with the substituents R1 and R3 defined as in the Table 1 given below.

As preferred compounds according to this invention the following compounds of formula I-BE* 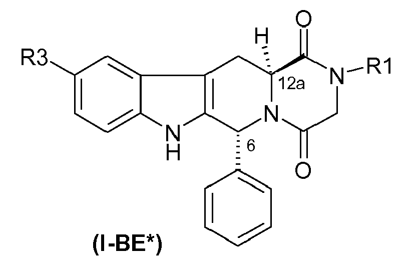 and the salts thereof,
may be mentioned, with the substituents R1 and R3 defined as in the Table 1 given below.

As further preferred compounds according to this invention the following compounds of formula I-AD** 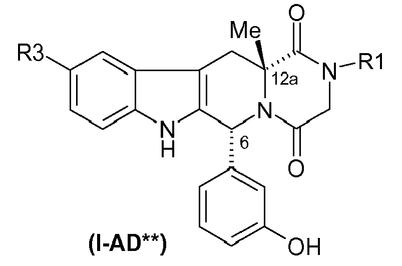 and the salts thereof,
may be mentioned, with the substituents R1 and R3 defined as in the Table 1 given below.

As preferred compounds according to this invention the following compounds of formula I-AE** 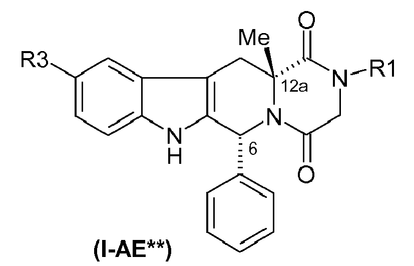 and the salts thereof,
may be mentioned, with the substituents R1 and R3 defined as in the Table 1 given below.

As further preferred compounds according to this invention the following compounds of formula I-BD** 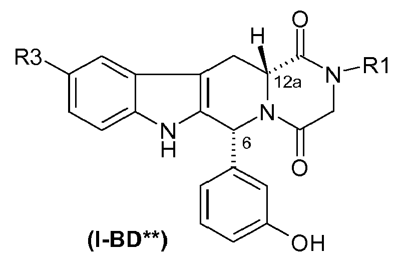 and the salts thereof,
may be mentioned, with the substituents R1 and R3 defined as in the Table 1 given below.

As further preferred compounds according to this invention the following compounds of formula I-BE** 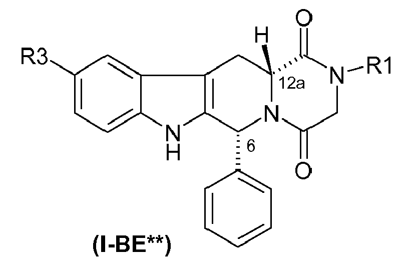 and the salts thereof,
may be mentioned, with the substituents R1 and R3 defined as in the Table 1 given below.

The compounds, or a salt, stereoisomer or a salt of a stereoisomer thereof, according to the invention can be prepared as follows. Preferably, they are prepared in a manner as described by way of example in the following examples. Furthermore, they can be prepared analogously to said preparation procedures or synthesis strategies known to the person skilled in the art.

"Analogously" includes performing a described reaction procedure, while choosing the corresponding reactants to yield the desired product. "Analogous procedures" differ from the described procedure in that reactants are replaced in order to adapt the procedure to represent the synthesis of the desired product.

As outlined in scheme 1, compounds of formula I, in which R1, R2, R3, R4, R5 have the meanings given above, can be obtained starting from compounds of formula II, in which R is 1-4C-alkyl, e.g. methyl or ethyl, and R2, R3, R4 and R5 have the meanings given above. Said compounds of formula II can be reacted in the presence of a base, e.g. triethylamine, and in a suitable solvent, e.g. tetrahydrofurane, with an activated chloro acetic acid Cl-CH2-C(O)Z, in which Z is a suitable leaving group (e.g. commercially available chloro acetyl chloride in which Z is CI), to yield the intermediates VI. Subsequent reaction with primary amines of formula R1-NH₂, in which R1 has the meanings given above, e.g. dimethylamino ethyl amine, leads to the corresponding desired pyrazines of formula I, wherein the reaction is preferably performed at a temperature between 30 and 110°C.

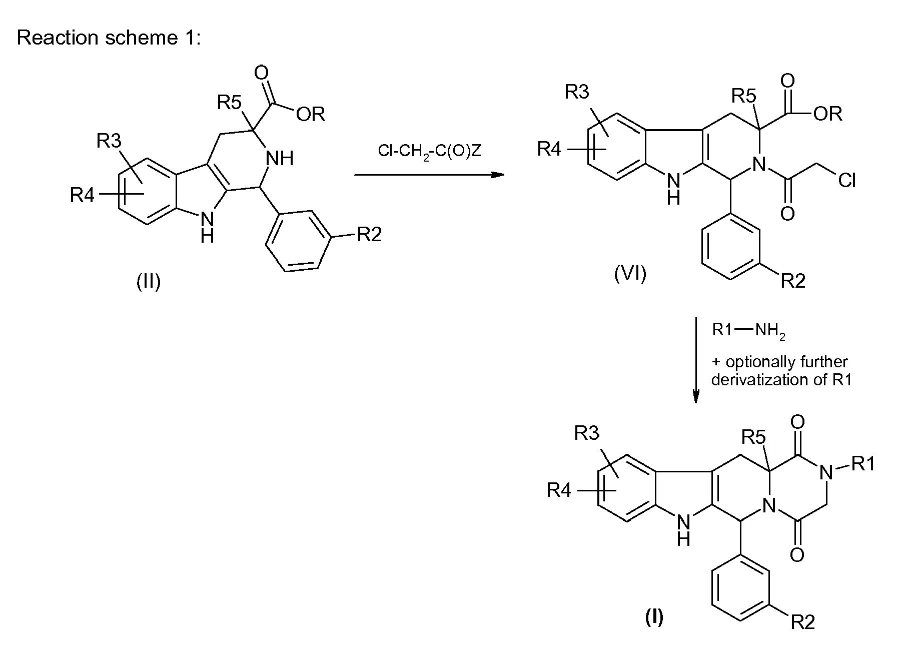

Compounds of formula I, which carry a suitable substituent R1 and of which I* or I*** are preferred, can be converted to further compounds with a different substituent R1 by derivatization. Particularly, compounds of formula I, in which R2, R3 and R4 have the meanings given above and R1 is 2-7C-alkyl (advantageously 2-4C-alkyl) substituted by X, in which X is a suitable leaving group, e.g. chlorine or bromine, can be reacted in a nucleophilic substitution reaction with amines of formula HN(R111)R112, in which R111 and R112 stand for the groups given above, which - if necessary - can be temporarily protected by appropriate protecting groups (such as e.g. free amino functions can be temporarily protected by the tert-butyloxycarbonyl (Boc) protecting group), to prepare corresponding compounds of formula I, in which R1 is 2-7C-alkyl substituted by - N(R111)R112. This nucleophilic substitution reaction can be carried out in a manner habitual per se for the skilled person or as described in the following examples or analogously thereto, e.g. in a suitable solvent (e.g. acetonitrile, methanol or tetrahydrofuran or the like) optionally in the presence of a suitable base or optionally in the presence of microwaves using an excess of the amine of formula HN(R111)R112 at atmospheric or elevated pressure (e.g. in a sealed container) at room temperature, at elevated temperature, at the boiling / reflux temperature or at the microwave super heated boiling temperature of the solvent(s) used.

In a special embodiment, compounds of formula I-1, in which R2, R3, R4 and R5 have the meanings given above and R1 is 1-4C-alkyl substituted by hydroxyl, can be synthesized via the procedure depicted in reaction scheme 1 using the corresponding 1-4C-alkylamine as primary amine (e.g. ethanolamine or propanolamine). Then, as illustrated in reaction scheme 2, the hydroxyl group in these compounds of formula I-1 is converted into a suitable leaving group Y, e.g. mesylate or bromo, and subsequently replaced by nucleophilic substitution with amines of formula HN(R111)R112, in which R111 and R112 have the meanings given above, which - if necessary - can be temporarily protected by appropriate protecting groups.

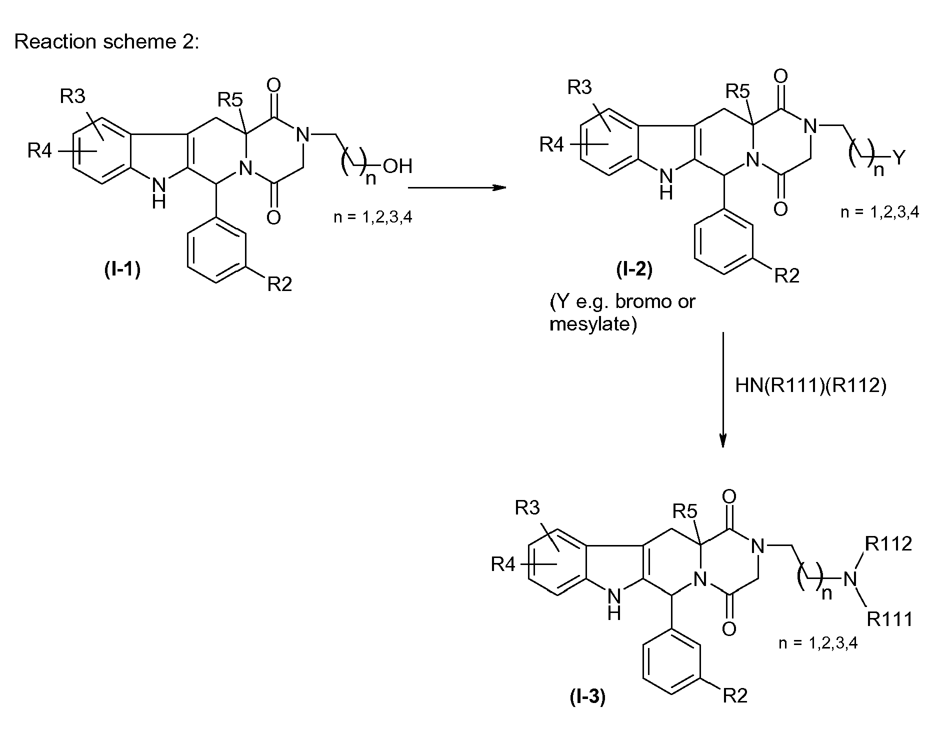

Pure diastereomers and pure enantiomers of the compounds and salts according to the invention may be prepared by processes known to the person skilled in the art. Preferably, they are obtained by asymmetric synthesis, by using chiral starting compounds in synthesis or by splitting up enantiomeric and diastereomeric mixtures obtained in synthesis.
In asymmetric synthesis, chiral synthons or chiral reagents are used as starting material. Diastereomeric and/or enantiomeric compounds may be separated at an appropriate stage in the preparation, e.g. at the stage of an intermediate. Enantiomeric and diastereomeric mixtures can be split up into the pure enantiomers and pure diastereomers by methods known to a person skilled in the art. Preferably, diastereomeric mixtures are separated by crystallization, in particular fractional crystallization, or chromatography. A preferred method for the separation of diastereomeric mixtures is the crystallization. Another preferred method for the separation of diastereomeric mixtures is the separation using chromatography.
Enantiomeric mixtures can preferably be separated e.g. by forming diastereomers with a chiral auxiliary agent, resolving the diastereomers obtained and removing the chiral auxiliary agent. A preferred method of separation is the diastereomeric salt formation of the racemic compounds with optically active auxiliary agents. As chiral auxiliary agents, for example, chiral acids (such as e.g. those mentioned below) can be used to separate enantiomeric bases and chiral bases can be used to separate enantiomeric acids via formation of diastereomeric salts. Preferred chiral acids are mandelic acid, tartaric acid, O,O'-dibenzoyltartaric acid, camphoric acid, quinic acid, glutamic acid, pyroglutamic acid, malic acid, camphorsulfonic acid, 3-bromocamphorsulfonic acid, α-methoxyphenylacetic acid, α-methoxy-α-trifluoromethylphenylacetic acid and 2-phenylpropionic acid. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Another suitable method for the isolation of enantiomers is the enzymatic separation, e.g. using a suitable lipase. Other methods include the kinetic resolution of a racemate, enantioselective (preferential) crystallization (or crystallization by entrainment) from a conglomerate of enantiomorphous crystals under suitable conditions; or by (fractional) crystallization from a suitable solvent in the presence of a chiral auxiliary. A preferred method for the separation of enantiomeric mixtures is the crystallization or the separation using chiral separating columns in chromatography.

If R5 is hydrogen, the mixture of diastereomeric compounds of formula I* and I** is preferably epimerized, e.g. under basic conditions, to give compounds of formula I*. Analogously, starting from a mixture of compounds of formula I*** and I****, compounds of formula I*** (trans-isomer) can be obtained by epimerization.

As shown in reaction scheme 3, enantiomeric compounds of formula I* and I***, in which R1, R2, R3, R4, R5 have the meanings given above, can be obtained by a procedure that is analogous to the procedure depicted in reaction scheme 1. Starting from compounds of formula IIa' respectively IIb', in which R is methyl or ethyl and R2, R3, R4 and R5 have the meanings given above, the procedure leads to the desired products I* and I*** via the intermediates VIa' (from compounds of formula IIa') respectively Vlb' (from compounds of formula IIb').
If the reactant is a compound of formula IIa' in enantiomerically pure form, enantiomerically pure compound I* is obtained. Analogously, a reaction of a compound of formula IIb' leads to an enantiomerically pure compound I***. If a mixture of compounds of the enantiomers described by formula IIa' and IIb', e.g. a racemic mixture thereof, is employed as reactant, a mixture of the compounds of formula I* and I*** is obtained.

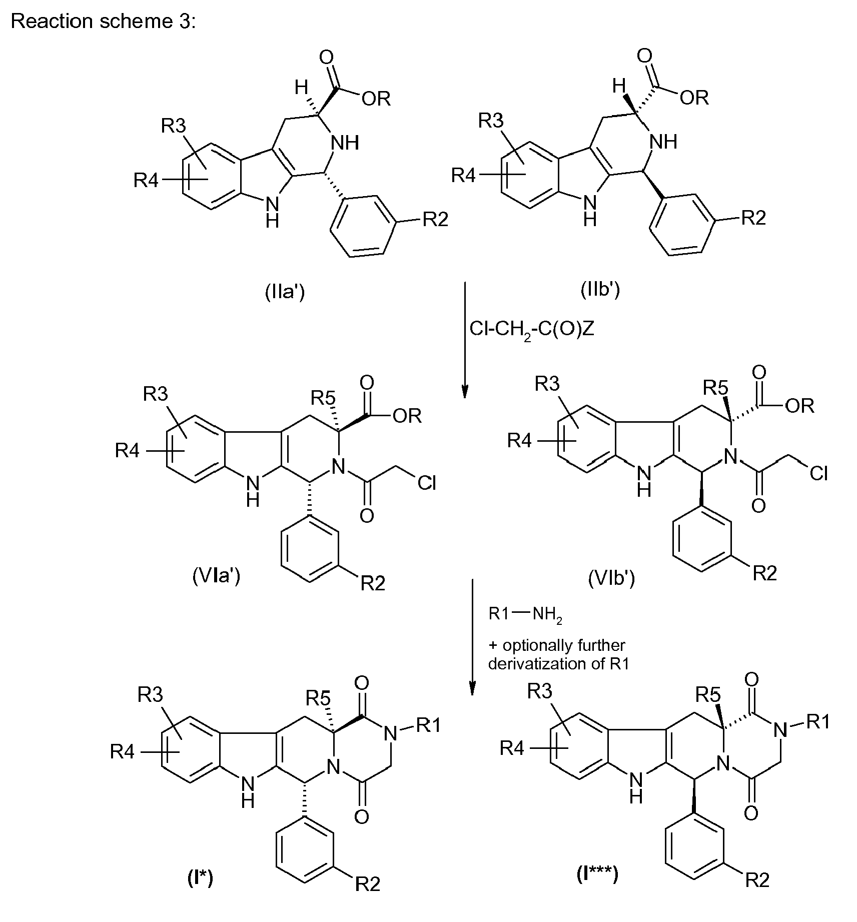

Analogously, compounds of formula I** and I**** can be obtained starting from IIa" respectively IIb". IIa'/IIb' is a pair of enantiomers ("trans") that is diastereomeric to the pair of enantiomers of IIa"/IIb" ("cis").

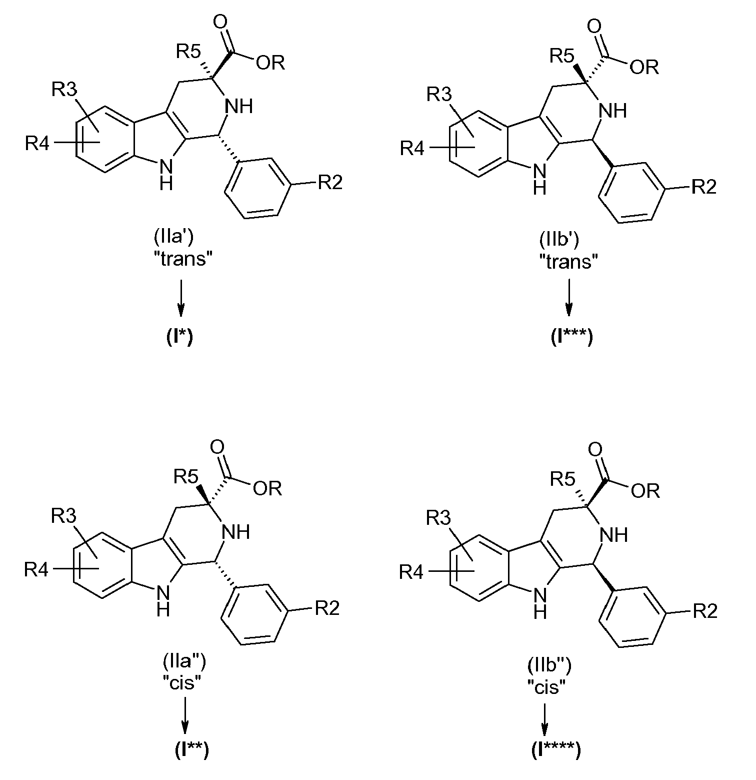

Compounds of formula II, in which R is 1-4C-alkyl, e.g. methyl or ethyl, and R2, R3, R4 and R5 have the meanings given above, can be obtained as follows. As shown in the synthesis route outlined in scheme 4 below, ester compounds of formula IV (particularly, the ethyl esters or, especially, methyl esters of formula IV), in which R3, R4 and R5 have the meanings given above, are condensed and cyclized in a Pictet-Spengler reaction with benzaldehydes of formula formula III, in which R2 has the meanings given above, to give the corresponding compounds of formulae IIa and/or IIb, in which R2, R3, R4 and R5 have the meanings given above, mostly as a mixture. Said Pictet-Spengler reaction can be carried out as it is known to the skilled person or as described in the following examples, advantageously in the presence of a suitable acid as a catalyst or promotor (e.g. trifluoroacetic acid) in a suitable solvent, for example toluene or, particularly dichloromethane, at elevated temperature, preferably between 30 and 110°C, or room temperature.

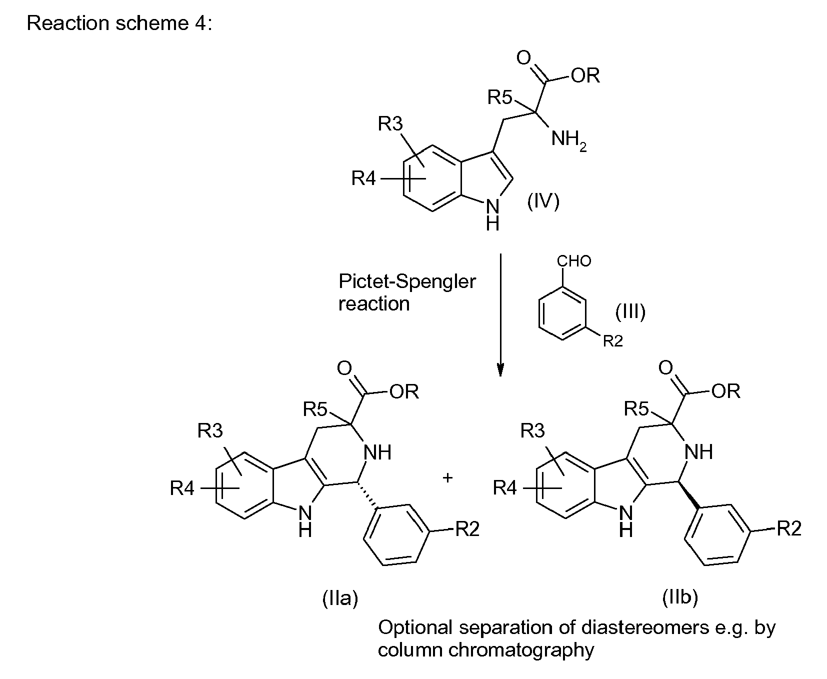

Compounds of formula IV, in which R is methyl or ethyl, and R3, R4 and R5 have the meanings given above, are known or can be prepared according to or analogously to known procedures or are accessible as described later.

Compounds of formula III, in which R2 has the meanings given above, are known or can be obtained in a known manner, for example by formylation of appropriate aromatic compounds, e.g. via hydroxymethylation and subsequent oxidation to the aldehyde, or by reduction of appropriate benzoic acid derivatives to the aldehyde.

The compounds of formula IV, in which R is methyl or ethyl, and R3, R4 and R5 have the meanings given above, can be employed in the abovementioned Pictet-Spengler reaction as racemate or enantiomerically pure compounds. Depending on the reactants, the mixture obtained can contain the compounds of formulae IIa and IIb as diastereomers or as diastereomeric racemates.
Said mixture can be optionally separated as described above or as known to the skilled person. For example, diastereomeric compounds of formulae IIa and IIb can be separated by column chromatography.
If appropriate, said mixture can be also used in the next step without further separation of the diastereoisomers. Then, separation of diastereomers can be carried out subsequently to one of the following steps. If R5 is hydrogen, the diastereomers are preferably epimerized, depending on the molecule, to give the thermodynamically more stable diastereomer as described above, e.g. at the final stage of compounds of formula I.

When the compounds of formula IV are employed as racemic mixture in the abovementioned Pictet-Spengler reaction, the racemate comprising the enantiomeric compounds of formulae IIa' and IIb' can be obtained preferentially or in excess from said reaction.

Starting from the appropriate pure enantiomers of the compounds of formula IV, corresponding compounds of either formula IIa' or formula IIb' (depending from the configuration of the starting compound of formula IV) can be obtained preferentially. Thus, e.g. when (S)-tryptophan methyl ester derivatives or (S)-α-methyltryptophan methyl ester derivatives [i.e. (S)-2-amino-3-(1H-indol-3-yl)-propionic acid methyl ester derivatives respectively (S)-2-amino-3-(1H-indol-3-yl)-2-methyl-propionic acid methyl ester derivatives] are employed in the abovementioned Pictet-Spengler reaction, corresponding compounds of formula IIa' are obtained preferentially.

Compounds of formulae IIa' and IIb' can be separated from diastereomeric compounds as described above for the pure enantiomers and diastereomers of the compounds according to the invention, such as, for example, by column chromatography or crystallization, preferably crystallization of diastereomeric salts with optically active acids, most preferably as described in the examples or analogously thereto.

Compounds of formula IV, in which R is methyl or ethyl, R3 and R4 have the meanings given above and R5 is hydrogen, which are reactants in the reaction depicted in scheme 4, are commercially available or are accessible as shown in reaction scheme 5, e.g. as described in the following examples or analogously thereto, or analogously as described in WO0194345, page 32/33ff.
Compounds of formula IV, in which R is methyl or ethyl, R3 and R4 have the meanings given above and R5 is1-4Calkyl, are commercially available (e.g. α-methyl-tryptophan as racemate or pure enantiomers) or accessible as shown in reaction scheme 5, e.g. as described in the following examples or analogously thereto.

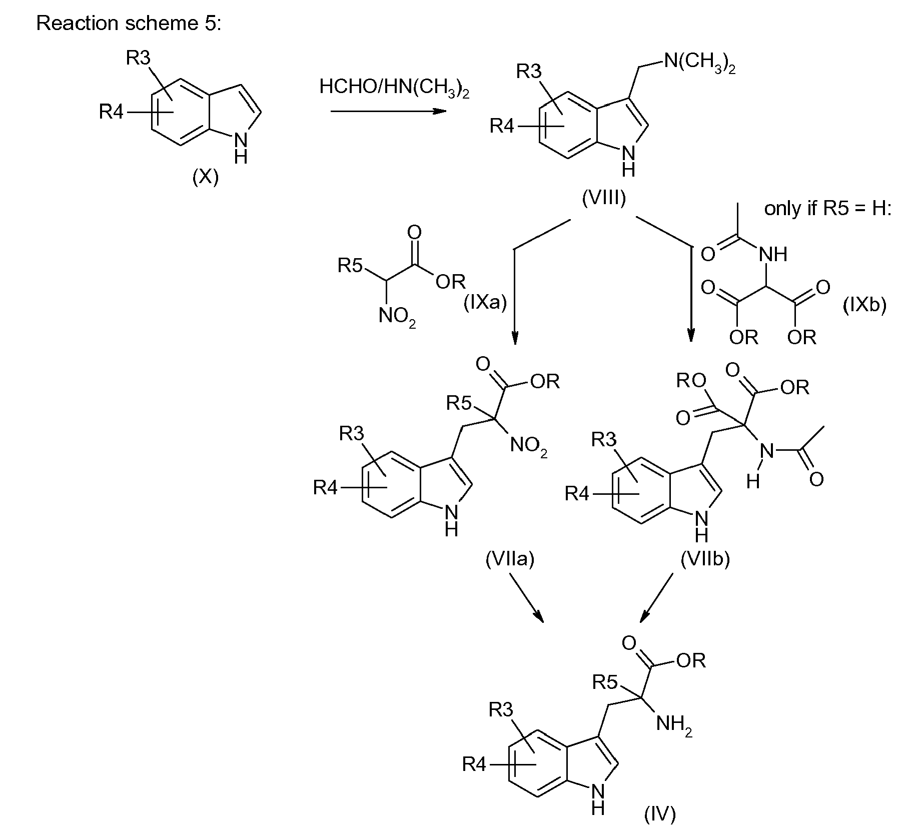

Starting from compounds of formula X, in which R3 and R4 have the meanings mentioned above, the corresponding compounds of formula VIII can be obtained by aminomethylation reaction (Mannich reaction) customary per se to the person skilled in the art.

Compounds of formula VIII are reacted with compounds of formula IXa, in which R is 1-4C-alkyl, e.g. methyl or ethyl, and R5 has the meanings given above, in a nucleophilic substitution reaction to give corresponding compounds of formula VIIa. Said substitution reaction can be carried out as it is known for the skilled person or as described in the following examples, or analogously thereto.

Compounds of formula VIIa, in which R3, R4 and R5 have the meanings given above, are subjected to a reduction reaction of the nitro group to obtain corresponding amine compounds of formula IV. Said reduction reaction can be carried out as known per se to the skilled person, such as, for example, by catalytic hydrogenation, e.g. in the presence of a noble metal catalyst such as palladium on active carbon or, particularly, Raney nickel. Optionally, a catalytic amount of an acid, such as, for example, hydrochloric acid, can be added to the solvent. Alternatively, the reduction may be carried out using a hydrogen-producing mixture, for example, metals such as zinc, zinc-copper couple or iron with organic acids such as acetic acid or mineral acids such as hydrochloric acid.

If R5 is hydrogen, compounds of formula VIII can be alternatively reacted with compounds of formula IXb, in which R is methyl or ethyl, in a nucleophilic substitution reaction to give corresponding compounds of formula Vllb. Said substitution reaction can be carried out as described in the following examples or as described in Bioorg. Med. Chem. Lett. 2005 (15), p. 5039-5044, or analogously thereto. Compounds of formula VIIb are subjected to basic saponification of the ester and the N-acetyl and subsequent decarboxylation to obtain corresponding amine compounds of formula IV, in which R is methyl, ethyl or hydrogen.

Optionally, ester compounds of formula IV can be converted into the corresponding free acids by known saponification reactions. Optionally, the free acids of compounds of formula IV can be also re-converted into the corresponding esters, particularly methyl esters, by known esterification reactions, e.g. using thionylchloride/methanol.

If R5 is hydrogen, the compound IXa (2-nitro-acetic acid) is commercially available, as well as compound IXb in which R is ethyl (diethylacetamido-malonate).

If R5 is 1-4C-alkyl, compounds of formula IXa, in which R is methyl or ethyl and R5 has the meanings given above, can be obtained as outlined in reaction scheme 6. If R5 is methyl, methyl 2-nitro-propionate is known e.g. from H.L. Finkbeiner, G.W. Wagner J. Org. Chem. 1963, 28, 215-217.

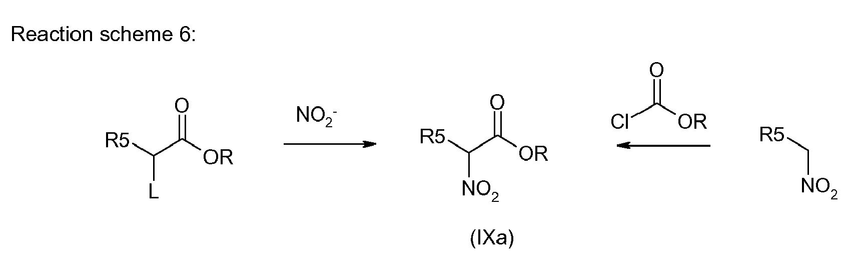

Compounds of formula IXa can be prepared by reaction of compounds of formula R5-CH₂-NO₂, in which R5 has the meanings given above, e.g. cyclopropyl, with a chloroformic acid ester, such as e.g. described in Ram et al. Synthesis 1986, 133-135, or analogously thereto.

Alternatively, compounds of formula IXa can be prepared by reaction of compounds of formula R5-C(H)L-CO₂R, in which L is a suitable leaving group, e.g. iodine, and R5 has the meanings given above, e.g. isopropyl, with a suitable nitrite reagent, e.g. sodium nitrite or silver nitrite, such as e.g. described in J. Am. Chem. Soc. 77, 6654 (1955), or analogously or similarly thereto.

Compounds of formula R5-CH₂-NO₂and R5-C(H)L-CO₂R are known or can be obtained ananlogously to known procedures (e.g. compounds of formula R5-C(H)L-CO₂R can be obtained via Finkelstein reaction); such as e.g. nitromethyl-cyclopropane can be obtained as described in Helv. Chim. Acta 1982, 65, 137-161 and 2-iodo-3-methyl-butyric acid ethyl ester can be obtained from 2-bromo-3-methyl-butyric acid ethyl ester as described in Org. Lett. 1999, 1, 1419-1422, or analogously or similarly thereto.

Compounds of formula X are known or can be obtained according to known procedures or as described in the following examples or analogously thereto. Thus, e.g. 5-methoxy-1H-indole, 5-chloro-1 H-indole, 5-bromo-1H-indole, 5-fluoro-1 H-indole and 5-trifluoromethyl-1 H-indole are commercially available.

Compounds of formula X, which are ether compounds, are obtained from the corresponding alcohol compounds by art-known etherification reaction. Thus, e.g. compounds of formula X, in which R3 is hydroxyl, can be converted into corresponding ether compounds in a manner as described in the following examples, or analogously thereto.

Thus, e.g. compounds of formula X, in which R3 is hydroxyl, can be converted into the corresponding compounds of formula X, in which R3 is 1-4C-alkoxy, e.g. ethoxy, n-propoxy, isopropoxy, cyclopropylmethoxy, difluoromethoxy or trifluoromethoxy, by alkylating reaction using an appropriate alkylating reagent.

Enantiomerically pure starting compounds according to the invention may be obtained as described above for the synthesis or separation of enantiomers and diastereomers of the compounds of formula I. Alternatively, enantiomerically pure tryptophans or tryptophan derivatives (e.g. ester derivatives IV, IIa, IIb, IIa', IIb', IIa", IIb", VIa', Vlb') may be obtained, for example, as described in WO0194345, page 32/33ff, or analogously thereto or following stereoselective amino acid synthesis, e.g. using an appropriate chiral auxiliary. Thus, enantiomerically pure tryptophans may be obtained, for example, as described in Tetrahedron Letters 39 (1998), 9589-9592, or analogously thereto. For example, enantiomerically pure α-methyl-tryptophans, α-ethyl-tryptophans or α-isopropyl-tryptophans may be obtained as described therein starting from N-Boc-(3-bromomethyl)-indole and enantiomerically pure alanine, 2-amino-butyric acid or valine, respectively.

In more detailed example, enantiomerically pure 5-methoxy-α-methyl-tryptophane methyl ester can be obtained by chromatographic separation of the corresponding racemate on chiral separating columns, such as e.g. Daicel CHIRALPAK AD-RH or Daicel CHIRALPAK AD-H; or by means of salt formation of the corresponding racemate with optically active acids, such as e.g. mandelic acid, pyroglutamic acid or, particularly, (S,S)-di-p-anisoyl-tartaric acid, subsequent resolution of the salt [e.g. by (fractional) crystallization from a suitable solvent, such as e.g. ethyl acetate, acetone or, particularly, methanol/water] and release of the desired compound from the salt.

When one of the final steps or purification is carried out under the presence of an inorganic or organic acid (e.g. hydrochloric, trifluoroacetic, acetic or formic acid or the like), the compounds of formula I may be obtained - depending on their individual chemical nature and the individual nature of the acid used - as free base or containing said acid in an stoechiometric or non-stoechiometric quantity. The amount of the acid contained can be determined according to art-known procedures, e.g. by titration or NMR.

It is known to the person skilled in the art that, if there are a number of reactive centers on a starting or intermediate compound, it may be necessary to block one or more reactive centers temporarily by protective groups in order to allow a reaction to proceed specifically at the desired reaction center. A detailed description for the use of a large number of proven protective groups is found, for example, in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000).

The substances according to the invention are isolated and purified in a manner known per se, for example by distilling off the solvent under reduced pressure and recrystallizing the residue obtained from a suitable solvent or subjecting it to one of the customary purification methods, such as, for example, column chromatography on a suitable support material.

Optionally, compounds of the formula I can be converted into their salts, or, optionally, salts of the compounds of the formula I can be converted into the free compounds.

Salts of the compounds of formula I according to the invention can be obtained by dissolving the free compound in a suitable solvent (for example a ketone such as acetone, methylethylketone or methylisobutylketone, an ether such as diethyl ether, diisopropyl ether, tetrahydrofuran or dioxane, a chlorinated hydrocarbon such as methylene chloride or chloroform, a low molecular weight aliphatic alcohol such as methanol, ethanol or isopropanol, or an ester, such as ethyl acetate) which contains the desired acid or base, or to which the desired acid or base is then added, if necessary upon heating. The acid or base can be employed in salt preparation, depending on whether a mono- or polybasic acid or base is concerned and depending on which salt is desired, in an equimolar quantitative ratio or one differing therefrom. The salts are obtained for example by evaporating the solvent, by re-precipitating or by precipitating upon cooling or by precipitating with a non-solvent for the salt and separation, for example by filtration, of the salt after precipitation.

Salts obtained can be converted into the free compounds which, in turn, can be converted into salts. In this manner, pharmaceutically unacceptable salts, which can be obtained, for example, as process products in the production of the compounds, or a salt, stereoisomer or a salt of a stereoisomer thereof, according to the invention on an industrial scale or in the isolation or purification of compounds of formula I, can be converted into pharmaceutically acceptable salts by processes known to the person skilled in the art.
In the salt preparation, the acids or bases are employed in an equimolar ratio or in a ratio differing therefrom, depending on the acid or base concerned, e.g. whether the acid is a mono- or polybasic acid, and on which salt is desired.

Thus, the present invention also relates to processes disclosed herein for preparing compounds according to this invention, which processes comprise one or more steps of converting and/or reacting the mentioned intermediates with the appropriate reaction partners under conditions as disclosed herein. The present invention also relates to intermediates (including their salts, stereoisomers and salts of these stereoisomers), methods and processes, which are disclosed herein and which are useful in synthesizing compounds according to this invention.

The following examples illustrate the invention in greater detail, without restricting it. Further compounds according to this invention, of which the preparation is not explicitly described, can be prepared in an analogous way.

The compounds, which are mentioned in the examples, and the salts thereof, their stereoisomers and salts thereof, represent preferred embodiments of the invention.

In the examples, m.p. stands for melting point, h for hour(s), min for minutes, v:v or v:v:v or v:v:v:v for ratio of volumes, conc. for concentrated, M for molar concentration, THF for tetrahydrofurane, calc. for calculated, fnd. for found, EF for elemental formula, MS for mass spectrometry, LCMS for liquid chromatography mass spectrometry, HPLC for high pressure liquid chromatography, M⁺ for molecular ion in mass spectrometry, MH⁺ for the proton adduct ion of the molecule with the mass M, m/z for observed mass peak (mass per charge), NMR for nuclear magnetic resonance, and other abbreviations have their meanings customary per se to the skilled person.
Room temperature is a temperature between 20 and 25°C.

The names of the compounds have been generated using Autonom in ISIS, Autonom Engine Version 4.0, Copyright (1998) Beilstein Institut Frankfurt am Main.

Further on, according to common practice in stereochemistry, the symbols RS and SR are used to denote the specific configuration of each of the indicated chiral centers of a racemate. If R5 is hydrogen, methyl or ethyl, the symbols are assigned as follows:
In more detail, for example, the term "(6RS,12aSR)" stands for a racemate comprising the one enantiomer having the configuration (6R,12aS) and the other enantiomer having the configuration (6S,12aR); yet in more detail, for example, the term "(6RS,12aRS)" stands for a racemic mixture comprising the one enantiomer having the configuration (6R,12aR) and the other enantiomer having the configuration (6S,12aS); each of these enantiomers and their salts in pure form as well as their mixtures including the racemic mixtures is part of this invention. In compounds of formula I in which R5 is methyl or ethyl, the enantiomer having the configuration (6R,12aS) is a preferred part of this invention. Correspondingly, in compounds of formula I in which R5 is isopropyl or cyclopropyl, this enantiomer having the configuration (6R,12aR) is a preferred part of this invention.
Thus, the trans-configured racemate is described as (6RS,12aSR) or, in an equivalent manner, as (6SR,12aRS) and contains the compound with the configuration (6R,12aS) as depicted in formula I* above and its enantiomer with the configuration (6S,12aR) as depicted in formula I***. Accordingly, the cis-configured racemate is described as (6RS,12aRS) or, in an equivalent manner, as (6SR,12aSR) and contains the compound with the configuration (6R,12aR) as depicted in formula I** above and its enantiomer with the configuration (6S,12aS) as depicted in formula I****.

### Examples

### Final compounds

### 1. (6RS,12aRS)-2-(2-Dimethylamino-ethyl)-6-(3-hydroxy-phenyl)-2,3,6,7,12,12a-hexahydro-pyrazino[1',2':1,6]pyrido[3,4-b]indole-1,4-dione (1a) and (6RS,12aSR)-2-(2-Dimethylamino-ethyl)-6-(3-hydroxy-phenyl)-2,3,6,7,12,12a-hexahydro-pyrazino[1',2':1,6]pyrido[3,4-b]indole-1,4-dione (1b)

A solution of 1.3 g of a mixture of the racemic diastereomers (1RS,3RS)-1-(3-Hydroxy-phenyl)-2,3,4,9-tetrahydro-1H-β-carboline-3-carboxylic acid methyl ester and (1SR,3RS)-1-(3-Hydroxyphenyl)-2,3,4,9-tetrahydro-1H-β-carboline-3-carboxylic acid methyl ester in 40 ml THF is cooled to 0 °C. 1.22 g (1.67 ml) triethyl amine are added. A solution of 1.09 g (770 µl) chloro acteylchloride in 10 ml THF are added dropwise. The ice bath is removed and the mixture is stirred at room temperature for 1 h. 2.4 ml 1 M hydrochloric acid are added and the solvents are removed at reduced pressure. Dichloromethane is added and the mixture is washed with a solution of sodium bicarbonate and brine. The organic layer is dried with magnesium sulfate and the solvents are removed at reduced pressure.
503 mg of the crude intermediate are dissolved in 20 ml ethanol. 591 mg (736 µl) N,N-dimethylethylenediamine are added and the solution is heated to 45 °C for 7 h. The solvents are removed under reduced pressure. Dichloromethane is added and the solution is washed with a solution of sodium bicarbonate. The organic layer is dried with magnesium sulfate and the solvents are removed.
After purification by column chromatography (silica gel; ethyl acetate, methanol, ammonia 10:1:0.5 v:v:v), 105 mg of the cis-diastereomer 1a are obtained. (m/z (MH⁺) = 419.0).
¹H-NMR (DMSO-d6): δ = 11.2 (s, 1 H, NH), 9.3 (s, 1 H, OH), 7.5 (d, 1 H, Hₐᵣₒₘ, J = 7.7 Hz), 7.3 (d, 1 H, Hₐᵣₒₘ, J = 8.0 Hz), 7.2 - 7.0 (m, 3H, Hₐᵣₒₘ), 6.8 (d, 1 H, Hₐᵣₒₘ, J = 7.8 Hz), 6.7 (s, 1 H, Hₐᵣₒₘ), 6.6 (dd, 1 H, Hₐᵣₒₘ, J = 8.0 Hz, J = 1.8 Hz), 6.2 (s, 1 H, CHPh), 4.4 (dd, 1 H, H_{alkyl}, J = 11.6 Hz, J = 4.6 Hz), 4.2 (d, 1 H, CH₂^{a}, J = 17.0 Hz), 4.0 (d, 1 H, CH₂^{b}, J = 16.9 Hz), 3.7 - 3.3 (m, 3H, H_{alkyl}), 2.9 (dd, 1 H, H_{alkyl}, J = 15.5 Hz, J = 11.7 Hz), 2.4 (t, 2H, CH₂, J = 6.4 Hz), 2.3 (s, 6H, CH₃).

66 mg of 1a are dissolved in acetonitrile and potassium carbonate is added. The suspension was heated to 110 °C (sealed tube, microwave reactor) until full conversion was obtained according to LCMS. The solvent was removed at reduced pressure and the residue was dissolved in ethyl acetate. The solution was washed with water and the organic layer was dried with magnesium sulfate. After evaporation of the solvent at reduced pressure, 57 mg of the trans-diastereomer 1b are obtained as a colorless solid. (m/z (MH⁺) = 419.0).

The following compounds are prepared starting from the appropriate starting materials A8a/b, A9a/b or A-I-1 and following the protocol described for the synthesis of example 1 (1a and 1 b).

| No | Name | m/z (MH⁺) |
|---|---|---|
| 2 | (6RS,12aRS)-6-(3-Hydroxy-phenyl)-2-methyl-2,3,6,7,12,12a-hexahydro-pyrazino[1',2':1,6]pyrido[3,4-b]indole-1,4-dione | 362.0 |
| 3 | (6RS,12aSR)-6-(3-Hydroxy-phenyl)-2-methyl-2,3,6,7,12,12a-hexahydro-pyrazino[1',2':1,6]pyrido[3,4-b]indole-1,4-dione | 362.0 |
| 4 | (6RS,12aRS)-6-(3-Hydroxy-phenyl)-2-(2-piperidin-1-yl-ethyl)-2,3,6,7,12,12a-hexahydro-pyrazino[1',2':1,6]pyrido[3,4-b]indole-1,4-dione | 459.1 |
| 5 | (6RS,12aRS)-6-(3-Hydroxy-phenyl)-2-(2-pyrrolidin-1-yl-ethyl)-2,3,6,7,12,12a-hexahydro-pyrazino[1',2':1,6]pyrido[3,4-b]indole-1,4-dione | 445.1 |
| 6 | (6RS,12aSR)-6-(3-Hydroxy-phenyl)-2-(2-pyrrolidin-1-yl-ethyl)-2,3,6,7,12,12a-hexahydro-pyrazino[1',2':1,6]pyrido[3,4-b]indole-1,4-dione | 445.0 |
| 7 | (6RS,12aSR)-6-(3-Hydroxy-phenyl)-2-(2-piperidin-1-yl-ethyl)-2,3,6,7,12,12a-hexahydro-pyrazino[1',2':1,6]pyrido[3,4-b]indole-1,4-dione | 459.1 |
| 8 | (6RS,12aSR)-2-(2-Dimethylamino-ethyl)-6-(3-hydroxy-phenyl)-10-methoxy-12a-methyl-2,3,6,7,12,12a-hexahydro-pyrazino[1',2':1,6]pyrido[3,4-b]indole-1,4-dione | 463.0 |
| 9 | (6RS,12aSR)-6-(3-Hyd roxy-phenyl)-10-methoxy-12a-methyl-2-(2-pyrrolidin-1-yl-ethyl)-2,3,6,7,12,12a-hexahydro-pyrazino[1',2':1,6]pyrido[3,4-b]indole-1,4-dione | 489.0 |
| 10 | (6RS,12aSR)-6-(3-Hydroxy-phenyl)-10-methoxy-2,12a-dimethyl-2,3,6,7,12,12a-hexahydro-pyrazino[1',2':1,6]pyrido[3,4-b]indole-1,4-dione | 406.0 |
| 11 | (6RS,12aSR)-6-(3-Hyd roxy-phenyl)-10-methoxy-12a-methyl-2-(2-piperidin-1-yl-ethyl)-2,3,6,7,12,12a-hexahydro-pyrazino[1',2':1,6]pyrido[3,4-b]indole-1,4-dione | 503.2 |
| 12 | (6RS,12aSR)-2-(3-Dimethylamino-n-propyl)-6-(3-hydroxy-phenyl)-10-methoxy-12a-methyl-2,3,6,7,12,12a-hexahydro-pyrazino[1',2':1,6]pyrido[3,4-b]indole-1,4-dione | 477.1 |
| 13 | (6RS,12aSR)-6-(3-Hydroxy-phenyl)-10-methoxy-12a-methyl-2-(2-morpholin-4-yl-ethyl)-2,3,6,7,12,12a-hexahydro-pyrazino[1',2':1,6]pyrido[3,4-b]indole-1,4-dione | 505.1 |
| 14 | (6RS,12aSR)-6-(3-Hydroxy-phenyl)-10-methoxy-12a-methyl-2-(3-morpholin-4-yl-n-propyl)-2,3,6,7,12,12a-hexahydro-pyrazino[1',2':1,6]pyrido[3,4-b]indole-1,4-dione | 519.2 |
| 15 | (6RS,12aSR)-2-(2-Diethylamino-ethyl)-6-(3-hydroxy-phenyl)-10-methoxy-12a-methyl-2,3,6,7,12,12a-hexahydro-pyrazino[1',2':1,6]pyrido[3,4-b]indole-1,4-dione | 491.1 |
| 16 | (6RS,12aSR)-2-(3-Diethylamino-n-propyl)-6-(3-hydroxy-phenyl)-10-methoxy-12a-methyl-2,3,6,7,12,12a-hexahydro-pyrazino[1',2':1,6]pyrido[3,4-b]indole-1,4-dione | 505.2 |
| 17 | (6RS,12aSR)-10-Chloro-6-(3-hydroxy-phenyl)-2-(2-morpholin-4-yl-ethyl)-2,3,6,7,12,12a-hexahydro-pyrazino[1',2':1,6]pyrido[3,4-b]indole-1,4-dione | 495.1 |
| 18 | (6RS,12aSR)-10-Chloro-6-(3-hydroxy-phenyl)-2-(3-morpholin-4-yl-n-propyl)-2,3,6,7,12,12a-hexahydro-pyrazino[1',2':1,6]pyrido[3,4-b]indole-1,4-dione | 509.2 |
| 19 | (6RS,12aSR)-2-(2-Diisopropylamino-ethyl)-6-(3-hydroxy-phenyl)-10-methoxy-12a-methyl-2,3,6,7,12,12a-hexahydro-pyrazino[1',2':1,6]pyrido[3,4-b]indole-1,4-dione | 519.2 |
| 20 | (6RS,12aSR)-10-Chloro-2-(3-diethylamino-n-propyl)-6-(3-hydroxy-phenyl)-2,3,6,7,12,12a-hexahydro-pyrazino[1',2':1,6]pyrido[3,4-b]indole-1,4-dione | 495.1 |
| 21 | (6RS,12aSR)-10-Chloro-6-(3-hydroxy-phenyl)-2-(2-pyrrolidin-1-yl-ethyl)-2,3,6,7,12,12a-hexahydro-pyrazino[1',2':1,6]pyrido[3,4-b]indole-1,4-dione | 479.1 |
| 22 | (6RS,12aSR)-10-Chloro-2-(2-dimethylamino-ethyl)-6-(3-hydroxy-phenyl)-2,3,6,7,12,12a-hexahydro-pyrazino[1',2':1,6]pyrido[3,4-b]indole-1,4-dione | 453.0 |
| 23 | (6RS,12aSR)-10-Chloro-2-(2-diethylamino-ethyl)-6-(3-hydroxy-phenyl)-2,3,6,7,12,12a-hexahydro-pyrazino[1',2':1,6]pyrido[3,4-b]indole-1,4-dione | 481.1 |
| 24 | (6RS,12aSR)-10-Ch loro-6-(3-hyd roxy-phenyl)-2-(3-i m idazol-1-yl-n-propyl)-2,3,6,7,12,12a-hexahydro-pyrazino[1',2':1,6]pyrido[3,4-b]indole-1,4-dione | 490.0 |
| 25 | (6RS,12aSR)-6-(3-Hydroxy-phenyl)-2-(2-isopropylamino-ethyl)-10-methoxy-12a-methyl-2,3,6,7,12,12a-hexahydro-pyrazino[1',2':1,6]pyrido[3,4-b]indole-1,4-dione | 477.2 |
| 26 | (6RS,12aSR)-10-Chloro-2-(2-diisopropylamino-ethyl)-6-(3-hydroxy-phenyl)-2,3,6,7,12,12a-hexahydro-pyrazino[1',2':1,6]pyrido[3,4-b]indole-1,4-dione | 509.2 |
| 27 | (6RS,12aSR)-6-(3-Hyd roxy-phenyl)-10-methoxy-12a-methyl-2-(3-piperidin-1-yl-n-propyl)-2,3,6,7,12,12a-hexahydro-pyrazino[1',2':1,6]pyrido[3,4-b]indole-1,4-dione | 517.2 |
| 28 | (6RS,12aSR)-6-(3-Hydroxy-phenyl)-10-methoxy-12a-methyl-2-(3-pyrrolidin-1-yl-n-propyl)-2,3,6,7,12,12a-hexahydro-pyrazino[1',2':1,6]pyrido[3,4-b]indole-1,4-dione | 503.2 |
| 29 | (6RS,12aSR)-6-(3-Hydroxy-phenyl)-10-methoxy-12a-methyl-2-(2-methylamino-ethyl)-2,3,6,7,12,12a-hexahydro-pyrazino[1',2':1,6]pyrido[3,4-b]indole-1,4-dione | 449.1 |
| 30 | (6RS,12aSR)-2-(3-Dimethylamino-n-propyl)-6-(3-hydroxy-phenyl)-10-methoxy-2,3,6,7,12,12a-hexahydro-pyrazino[1',2':1,6]pyrido[3,4-b]indole-1,4-dione | 463.2 |
| 31 | (6RS,12aSR)-2-(3-Diethylamino-n-propyl)-6-(3-hydroxy-phenyl)-10-methoxy-2,3,6,7,12,12a-hexahydro-pyrazino[1',2':1,6]pyrido[3,4-b]indole-1,4-dione | 491.2 |

### 1c. (6R,12aR)-2-(2-Dimethylamino-ethyl)-6-(3-hydroxy-phenyl)-2,3,6,7,12,12a-hexahydro-pyrazino[1',2':1,6]pyrido[3,4-b]indole-1,4-dione

A solution of 100 mg (1R,3R)-1-(3-Hydroxy-phenyl)-2,3,4,9-tetrahydro-1H-β-carboline-3-carboxylic acid methyl ester in 3 ml THF is cooled to 0 °C. 126 mg (172 µl) triethylamine and a solution of 91 mg (64.0 µl) chloroacetyl chloride in 1 ml THF are added. The mixture is allowed to warm up to room temperature. After 1 h at room temperature, 770 µl 1M hydrochloric acid are added and the solvents are removed under reduced pressure.
The crude intermediate is dissolved in 3 ml THF and 171 mg N,N-dimethylethylene diamine are added. The mixture is heated to 40 °C for 4 h. Dichloromethane is added and the solution is washed with water and 0.1 M saodium hydroxide. The organic layer is dried with magnesium sulfate and the solvent is removed under reduced pressure. After purification by column chromatography (silica gel; ethyl acetate, methanol, ammonia 10:1:0.5 v:v:v), 41 mg of the title compound are obtained as a yellowish solid. (m/z (MH⁺) = 419.0).

### Salts may be prepared according to the following general procedure:

A mixture of 100 mg of the appropriate free base, 1.00 equivalents of the corresponding acid and 1 ml of an appropriate solvent (preferably chosen from methanol, 2-propanol, diisopropyl ether, ethanol, ethyl acetate, acetone) is heated to reflux. The solution is allowed to cool down to room temperature. The precipitated salt is filtered, washed with solvent and dried at 40 °C under reduced pressure.

Compounds of formula I according to the formulae AD*, I-AE*, I-BD*, I-BE*, I-AD**, I-AE**, I-BD** or I-BE** mentioned below and according to table 1 may be prepared analogously as described for the examples 1a, 1b or 1c, starting from the corresponding reactant chosen from A1 to A10a/b respectively A-I-1 to A-I-19 or A-II-1 to A-II-10.

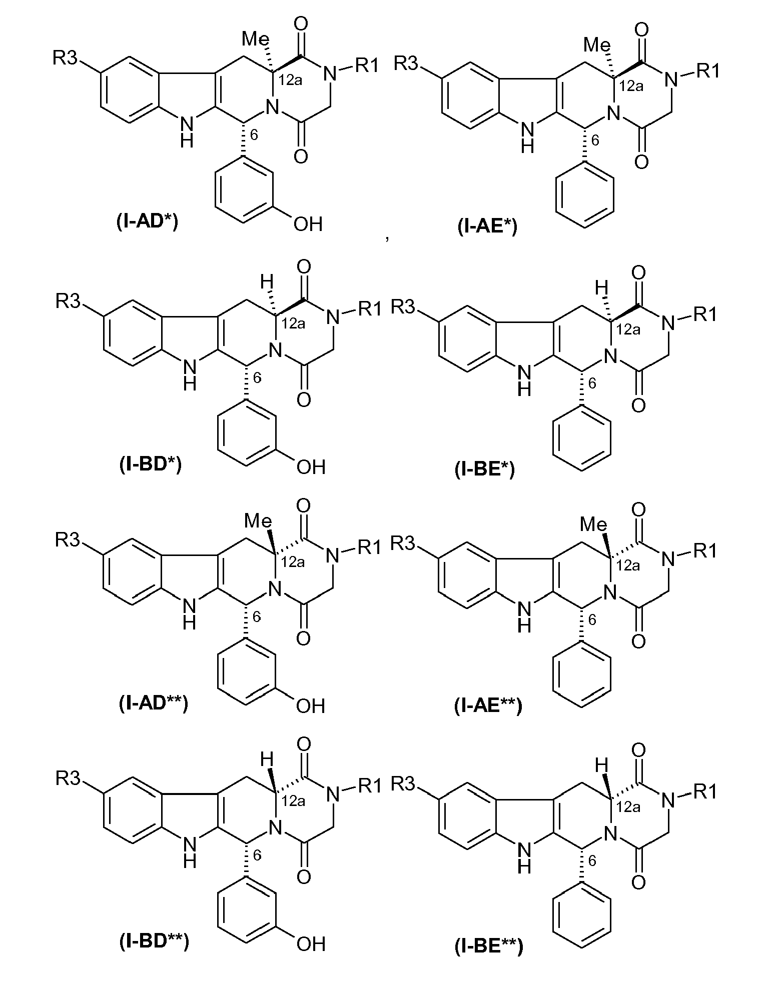

**Table 1:**

| **No.** | **R1** | **R3** |
|---|---|---|
| 1.1 | 2-bromo-ethyl | methoxy |
| 1.2 | 2-amino-ethyl | methoxy |
| 1.3 | 2-methylamino-ethyl | methoxy |
| 1.4 | 2-ethylamino-ethyl | methoxy |
| 1.5 | 2-(2,2-difluoroethyl)-amino-ethyl | methoxy |
| 1.6 | 2-isopropylamino-ethyl | methoxy |
| 1.7 | 2-isobutylamino-ethyl | methoxy |
| 1.8 | 2-cyclopropylmethylamino-ethyl | methoxy |
| 1.9 | 2-(2-hydroxy-ethylamino)-ethyl | methoxy |
| 1.10 | 2-(2-methoxy-ethylamino)-ethyl | methoxy |
| 1.11 | 2-cyclopropylamino-ethyl | methoxy |
| 1.12 | 2-cyclobutylamino-ethyl | methoxy |
| 1.13 | 2-*tert*-butylamino-ethyl | methoxy |
| 1.14 | 2-allylam ino-ethyl | methoxy |
| 1.15 | 2-prop-2-ynylamino-ethyl | methoxy |
| 1.16 | 2-(1-methyl-1H-pyrazol-2-ylamino)-ethyl | methoxy |
| 1.17 | 2-(2-methyl-2H-pyrazol-3-ylamino)-ethyl | methoxy |
| 1.18 | 2-dimethylamino-ethyl | methoxy |
| 1.19 | 2-(ethyl-methyl-am ino)-ethyl | methoxy |
| 1.20 | 2-[(2-hydroxy-ethyl)-methyl-amino]-ethyl | methoxy |
| 1.21 | 2-diethylamino-ethyl | methoxy |
| 1.22 | 2-[(2-hydroxy-ethyl)-ethyl-amino]-ethyl | methoxy |
| 1.23 | 2-[(2-methoxy-ethyl)-ethyl-amino]-ethyl | methoxy |
| 1.24 | 2-(allyl-methyl-am ino)-ethyl | methoxy |
| 1.25 | 2-(methyl-prop-2-ynyl-am ino)-ethyl | methoxy |
| 1.26 | 2-(isopropyl-methyl-am ino)-ethyl | methoxy |
| 1.27 | 2-azetidin-1-yl-ethyl | methoxy |
| 1.28 | 2-morpholin-4-yl-ethyl | methoxy |
| 1.29 | 2-pyrrolidin-1-yl-ethyl | methoxy |
| 1.30 | 2-(3.3-difluoro-pyrrolidin-1-yl)-ethyl | methoxy |
| 1.31 | 2-(2,5-dihydro-pyrrol-1-yl)-ethyl | methoxy |
| 1.32 | 2-piperidin-1-yl-ethyl | methoxy |
| 1.33 | 2-(4-methyl-piperidin-1-yl)-ethyl | methoxy |
| 1.34 | 2-(3,6-dihydro-2H-pyridin-1-yl)-ethyl | methoxy |
| 1.35 | 2-(4-methyl-piperazin-1-yl)-ethyl | methoxy |
| 1.36 | 2-(4-acetyl-piperazin-1-yl)-ethyl | methoxy |
| 1.37 | 2-azepan-1-yl-ethyl | methoxy |
| 1.38 | 2-imidazol-1-yl-ethyl | methoxy |
| 1.39 | 3-chloro-n-propyl | methoxy |
| 1.40 | 3-amino-n-propyl | methoxy |
| 1.41 | 3-methylamino-n-propyl | methoxy |
| 1.42 | 3-ethylamino-n-propyl | methoxy |
| 1.43 | 3-(2,2-difluoroethyl)-amino-n-propyl | methoxy |
| 1.44 | 3-isopropylamino-n-propyl | methoxy |
| 1.45 | 3-isobutylamino-n-propyl | methoxy |
| 1.46 | 3-cyclopropylmethylamino-n-propyl | methoxy |
| 1.47 | 3-(2-hydroxy-ethylamino)-n-propyl | methoxy |
| 1.48 | 3-(2-hydroxy-ethylamino)-n-propyl | methoxy |
| 1.49 | 3-cyclopropylamino-n-propyl | methoxy |
| 1.50 | 3-cyclobutylamino-n-propyl | methoxy |
| 1.51 | 3-*tert*-butylamino-n-propyl | methoxy |
| 1.52 | 3-allylam ino-n-propyl | methoxy |
| 1.53 | 3-prop-2-ynylamino-n-propyl | methoxy |
| 1.54 | 3-(1-methyl-1H-pyrazol-2-ylamino)-n-propyl | methoxy |
| 1.55 | 3-(2-methyl-2H-pyrazol-3-ylamino)-n-propyl | methoxy |
| 1.56 | 3-dimethylamino-n-propyl | methoxy |
| 1.57 | 3-(ethyl-methyl-am ino)-n-propyl | methoxy |
| 1.58 | 3-[(2-hydroxy-ethyl)-methyl-amino]-n-propyl | methoxy |
| 1.59 | 3-diethylamino-n-propyl | methoxy |
| 1.60 | 3-[(2-hydroxy-ethyl)-ethyl-amino]-n-propyl | methoxy |
| 1.61 | 3-[(2-methoxy-ethyl)-ethyl-amino]-n-propyl | methoxy |
| 1.62 | 3-(allyl-methyl-am ino)-n-propyl | methoxy |
| 1.63 | 3-(methyl-prop-2-ynyl-am ino)-n-propyl | methoxy |
| 1.64 | 3-(isopropyl-methyl-am ino)-n-propyl | methoxy |
| 1.65 | 3-azetidin-1-yl-n-propyl | methoxy |
| 1.66 | 3-morpholin-4-yl-n-propyl | methoxy |
| 1.67 | 3-pyrrolidin-1-yl-n-propyl | methoxy |
| 1.68 | 3-(3.3-difluoro-pyrrolidin-1-yl)-n-propyl | methoxy |
| 1.69 | 3-(2,5-dihydro-pyrrol-1-yl)-n-propyl | methoxy |
| 1.70 | 3-piperidin-1-yl-n-propyl | methoxy |
| 1.71 | 3-(4-methyl-piperidin-1-yl)-n-propyl | methoxy |
| 1.72 | 3-(3,6-dihydro-2H-pyridin-1-yl)-n-propyl | methoxy |
| 1.73 | 3-(4-methyl-piperazin-1-yl)-n-propyl | methoxy |
| 1.74 | 3-(4-acetyl-piperazin-1-yl)-n-propyl | methoxy |
| 1.75 | 3-azepan-1-yl-n-propyl | methoxy |
| 1.76 | 3-imidazol-1-yl-n-propyl | methoxy |
| 1.77 | 2-bromo-ethyl | ethoxy |
| 1.78 | 2-amino-ethyl | ethoxy |
| 1.79 | 2-methylamino-ethyl | ethoxy |
| 1.80 | 2-ethylamino-ethyl | ethoxy |
| 1.81 | 2-(2,2-difluoroethyl)-amino-ethyl | ethoxy |
| 1.82 | 2-isopropylamino-ethyl | ethoxy |
| 1.83 | 2-isobutylamino-ethyl | ethoxy |
| 1.84 | 2-cyclopropylmethylamino-ethyl | ethoxy |
| 1.85 | 2-(2-hyd roxy-ethylam ino)-ethyl | ethoxy |
| 1.86 | 2-(2-methoxy-ethylamino)-ethyl | ethoxy |
| 1.87 | 2-cyclopropylamino-ethyl | ethoxy |
| 1.88 | 2-cyclobutylamino-ethyl | ethoxy |
| 1.89 | 2-*tert*-butylamino-ethyl | ethoxy |
| 1.90 | 2-allylam ino-ethyl | ethoxy |
| 1.91 | 2-prop-2-ynylamino-ethyl | ethoxy |
| 1.92 | 2-(1-methyl-1H-pyrazol-2-ylamino)-ethyl | ethoxy |
| 1.93 | 2-(2-methyl-2H-pyrazol-3-ylamino)-ethyl | ethoxy |
| 1.94 | 2-dimethylamino-ethyl | ethoxy |
| 1.95 | 2-(ethyl-methyl-am ino)-ethyl | ethoxy |
| 1.96 | 2-[(2-hydroxy-ethyl)-methyl-amino]-ethyl | ethoxy |
| 1.97 | 2-diethylamino-ethyl | ethoxy |
| 1.98 | 2-[(2-hydroxy-ethyl)-ethyl-amino]-ethyl | ethoxy |
| 1.99 | 2-[(2-methoxy-ethyl)-ethyl-amino]-ethyl | ethoxy |
| 1.100 | 2-(allyl-methyl-am ino)-ethyl | ethoxy |
| 1.101 | 2-(methyl-prop-2-ynyl-am ino)-ethyl | ethoxy |
| 1.102 | 2-(isopropyl-m ethyl-am ino)-ethyl | ethoxy |
| 1.103 | 2-azetidin-1-yl-ethyl | ethoxy |
| 1.104 | 2-morpholin-4-yl-ethyl | ethoxy |
| 1.105 | 2-pyrrolidin-1-yl-ethyl | ethoxy |
| 1.106 | 2-(3.3-difluoro-pyrrolidin-1-yl)-ethyl | ethoxy |
| 1.107 | 2-(2,5-dihydro-pyrrol-1-yl)-ethyl | ethoxy |
| 1.108 | 2-piperidin-1-yl-ethyl | ethoxy |
| 1.109 | 2-(4-methyl-piperidin-1-yl)-ethyl | ethoxy |
| 1.110 | 2-(3,6-dihydro-2H-pyridin-1-yl)-ethyl | ethoxy |
| 1.111 | 2-(4-methyl-piperazin-1-yl)-ethyl | ethoxy |
| 1.112 | 2-(4-acetyl-piperazin-1-yl)-ethyl | ethoxy |
| 1.113 | 2-azepan-1-yl-ethyl | ethoxy |
| 1.114 | 2-imidazol-1-yl-ethyl | ethoxy |
| 1.115 | 3-chloro-n-propyl | ethoxy |
| 1.116 | 3-amino-n-propyl | ethoxy |
| 1.117 | 3-methylamino-n-propyl | ethoxy |
| 1.118 | 3-ethylamino-n-propyl | ethoxy |
| 1.119 | 3-(2,2-difluoroethyl)-amino-n-propyl | ethoxy |
| 1.120 | 3-isopropylamino-n-propyl | ethoxy |
| 1.121 | 3-isobutylamino-n-propyl | ethoxy |
| 1.122 | 3-cyclopropylmethylamino-n-propyl | ethoxy |
| 1.123 | 3-(2-hydroxy-ethylamino)-n-propyl | ethoxy |
| 1.124 | 3-(2-hydroxy-ethylamino)-n-propyl | ethoxy |
| 1.125 | 3-cyclopropylamino-n-propyl | ethoxy |
| 1.126 | 3-cyclobutylamino-n-propyl | ethoxy |
| 1.127 | 3-*tert*-butylamino-n-propyl | ethoxy |
| 1.128 | 3-allylamino-n-propyl | ethoxy |
| 1.129 | 3-prop-2-ynylamino-n-propyl | ethoxy |
| 1.130 | 3-(1-methyl-1H-pyrazol-2-ylamino)-n-propyl | ethoxy |
| 1.131 | 3-(2-methyl-2H-pyrazol-3-ylamino)-n-propyl | ethoxy |
| 1.132 | 3-dimethylamino-n-propyl | ethoxy |
| 1.133 | 3-(ethyl-methyl-amino)-n-propyl | ethoxy |
| 1.134 | 3-[(2-hydroxy-ethyl)-methyl-amino]-n-propyl | ethoxy |
| 1.135 | 3-diethylamino-n-propyl | ethoxy |
| 1.136 | 3-[(2-hydroxy-ethyl)-ethyl-amino]-n-propyl | ethoxy |
| 1.137 | 3-[(2-methoxy-ethyl)-ethyl-amino]-n-propyl | ethoxy |
| 1.138 | 3-(allyl-methyl-amino)-n-propyl | ethoxy |
| 1.139 | 3-(methyl-prop-2-ynyl-amino)-n-propyl | ethoxy |
| 1.140 | 3-(isopropyl-methyl-amino)-n-propyl | ethoxy |
| 1.141 | 3-azetidin-1-yl-n-propyl | ethoxy |
| 1.142 | 3-morpholin-4-yl-n-propyl | ethoxy |
| 1.143 | 3-pyrrolidin-1-yl-n-propyl | ethoxy |
| 1.144 | 3-(3.3-difluoro-pyrrolidin-1-yl)-n-propyl | ethoxy |
| 1.145 | 3-(2,5-dihydro-pyrrol-1-yl)-n-propyl | ethoxy |
| 1.146 | 3-piperidin-1-yl-n-propyl | ethoxy |
| 1.147 | 3-(4-methyl-piperidin-1-yl)-n-propyl | ethoxy |
| 1.148 | 3-(3,6-dihydro-2H-pyridin-1-yl)-n-propyl | ethoxy |
| 1.149 | 3-(4-methyl-piperazin-1-yl)-n-propyl | ethoxy |
| 1.150 | 3-(4-acetyl-piperazin-1-yl)-n-propyl | ethoxy |
| 1.151 | 3-azepan-1-yl-n-propyl | ethoxy |
| 1.152 | 3-imidazol-1-yl-n-propyl | ethoxy |
| 1.153 | 2-bromo-ethyl | difluoromethoxy |
| 1.154 | 2-amino-ethyl | difluoromethoxy |
| 1.155 | 2-methylamino-ethyl | difluoromethoxy |
| 1.156 | 2-ethylamino-ethyl | difluoromethoxy |
| 1.157 | 2-(2,2-difluoroethyl)-amino-ethyl | difluoromethoxy |
| 1.158 | 2-isopropylamino-ethyl | difluoromethoxy |
| 1.159 | 2-isobutylamino-ethyl | difluoromethoxy |
| 1.160 | 2-cyclopropylmethylamino-ethyl | difluoromethoxy |
| 1.161 | 2-(2-hyd roxy-ethylam ino)-ethyl | d ifluoromethoxy |
| 1.162 | 2-(2-methoxy-ethylamino)-ethyl | difluoromethoxy |
| 1.163 | 2-cyclopropylamino-ethyl | difluoromethoxy |
| 1.164 | 2-cyclobutylamino-ethyl | difluoromethoxy |
| 1.165 | 2-*tert*-butylamino-ethyl | difluoromethoxy |
| 1.166 | 2-allylamino-ethyl | difluoromethoxy |
| 1.167 | 2-prop-2-ynylamino-ethyl | difluoromethoxy |
| 1.168 | 2-(1-methyl-1H-pyrazol-2-ylamino)-ethyl | difluoromethoxy |
| 1.169 | 2-(2-methyl-2H-pyrazol-3-ylamino)-ethyl | difluoromethoxy |
| 1.170 | 2-dimethylamino-ethyl | difluoromethoxy |
| 1.171 | 2-(ethyl-methyl-am ino)-ethyl | d ifluoromethoxy |
| 1.172 | 2-[(2-hydroxy-ethyl)-methyl-amino]-ethyl | difluoromethoxy |
| 1.173 | 2-diethylamino-ethyl | difluoromethoxy |
| 1.174 | 2-[(2-hydroxy-ethyl)-ethyl-amino]-ethyl | difluoromethoxy |
| 1.175 | 2-[(2-methoxy-ethyl)-ethyl-amino]-ethyl | difluoromethoxy |
| 1.176 | 2-(allyl-methyl-am ino)-ethyl | difluoromethoxy |
| 1.177 | 2-(methyl-prop-2-ynyl-am ino)-ethyl | difluoromethoxy |
| 1.178 | 2-(isopropyl-methyl-am ino)-ethyl | difluoromethoxy |
| 1.179 | 2-azetidin-1-yl-ethyl | difluoromethoxy |
| 1.180 | 2-morpholin-4-yl-ethyl | difluoromethoxy |
| 1.181 | 2-pyrrolidin-1-yl-ethyl | difluoromethoxy |
| 1.182 | 2-(3.3-difluoro-pyrrolidin-1-yl)-ethyl | difluoromethoxy |
| 1.183 | 2-(2,5-dihydro-pyrrol-1-yl)-ethyl | difluoromethoxy |
| 1.184 | 2-piperidin-1-yl-ethyl | difluoromethoxy |
| 1.185 | 2-(4-methyl-piperidin-1-yl)-ethyl | difluoromethoxy |
| 1.186 | 2-(3,6-dihydro-2H-pyridin-1-yl)-ethyl | difluoromethoxy |
| 1.187 | 2-(4-methyl-piperazin-1-yl)-ethyl | difluoromethoxy |
| 1.188 | 2-(4-acetyl-piperazin-1-yl)-ethyl | difluoromethoxy |
| 1.189 | 2-azepan-1-yl-ethyl | difluoromethoxy |
| 1.190 | 2-imidazol-1-yl-ethyl | difluoromethoxy |
| 1.191 | 3-chloro-n-propyl | d ifluoromethoxy |
| 1.192 | 3-amino-n-propyl | difluoromethoxy |
| 1.193 | 3-methylamino-n-propyl | difluoromethoxy |
| 1.194 | 3-ethylamino-n-propyl | difluoromethoxy |
| 1.195 | 3-(2,2-difluoroethyl)-amino-n-propyl | difluoromethoxy |
| 1.196 | 3-isopropylamino-n-propyl | difluoromethoxy |
| 1.197 | 3-isobutylamino-n-propyl | difluoromethoxy |
| 1.198 | 3-cyclopropylmethylamino-n-propyl | difluoromethoxy |
| 1.199 | 3-(2-hydroxy-ethylamino)-n-propyl | difluoromethoxy |
| 1.200 | 3-(2-hydroxy-ethylamino)-n-propyl | difluoromethoxy |
| 1.201 | 3-cyclopropylamino-n-propyl | difluoromethoxy |
| 1.202 | 3-cyclobutylamino-n-propyl | difluoromethoxy |
| 1.203 | 3-*tert*-butylamino-n-propyl | difluoromethoxy |
| 1.204 | 3-allylamino-n-propyl | difluoromethoxy |
| 1.205 | 3-prop-2-ynylamino-n-propyl | difluoromethoxy |
| 1.206 | 3-(1-methyl-1H-pyrazol-2-ylamino)-n-propyl | difluoromethoxy |
| 1.207 | 3-(2-methyl-2H-pyrazol-3-ylamino)-n-propyl | difluoromethoxy |
| 1.208 | 3-dimethylamino-n-propyl | difluoromethoxy |
| 1.209 | 3-(ethyl-methyl-am ino)-n-propyl | difluoromethoxy |
| 1.210 | 3-[(2-hydroxy-ethyl)-methyl-amino]-n-propyl | difluoromethoxy |
| 1.211 | 3-d iethylam ino-n-propyl | d ifluoromethoxy |
| 1.212 | 3-[(2-hydroxy-ethyl)-ethyl-amino]-n-propyl | difluoromethoxy |
| 1.213 | 3-[(2-methoxy-ethyl)-ethyl-am ino]-n-propyl | difluoromethoxy |
| 1.214 | 3-(allyl-methyl-am ino)-n-propyl | difluoromethoxy |
| 1.215 | 3-(methyl-prop-2-ynyl-am ino)-n-propyl | difluoromethoxy |
| 1.216 | 3-(isopropyl-methyl-am ino)-n-propyl | difluoromethoxy |
| 1.217 | 3-azetidin-1-yl-n-propyl | difluoromethoxy |
| 1.218 | 3-morpholin-4-yl-n-propyl | difluoromethoxy |
| 1.219 | 3-pyrrolidin-1-yl-n-propyl | difluoromethoxy |
| 1.220 | 3-(3.3-difluoro-pyrrolidin-1-yl)-n-propyl | difluoromethoxy |
| 1.221 | 3-(2,5-dihydro-pyrrol-1-yl)-n-propyl | d ifluoromethoxy |
| 1.222 | 3-piperidin-1-yl-n-propyl | difluoromethoxy |
| 1.223 | 3-(4-methyl-piperidin-1-yl)-n-propyl | difluoromethoxy |
| 1.224 | 3-(3,6-dihydro-2H-pyridin-1-yl)-n-propyl | difluoromethoxy |
| 1.225 | 3-(4-methyl-piperazin-1-yl)-n-propyl | difluoromethoxy |
| 1.226 | 3-(4-acetyl-piperazin-1-yl)-n-propyl | difluoromethoxy |
| 1.227 | 3-azepan-1-yl-n-propyl | difluoromethoxy |
| 1.228 | 3-imidazol-1-yl-n-propyl | difluoromethoxy |
| 1.229 | 2-bromo-ethyl | chloro |
| 1.230 | 2-amino-ethyl | chloro |
| 1.231 | 2-methylamino-ethyl | chloro |
| 1.232 | 2-ethylamino-ethyl | chloro |
| 1.233 | 2-(2,2-difluoroethyl)-amino-ethyl | chloro |
| 1.234 | 2-isopropylamino-ethyl | chloro |
| 1.235 | 2-isobutylamino-ethyl | chloro |
| 1.236 | 2-cyclopropylmethylamino-ethyl | chloro |
| 1.237 | 2-(2-hydroxy-ethylamino)-ethyl | chloro |
| 1.238 | 2-(2-methoxy-ethylamino)-ethyl | chloro |
| 1.239 | 2-cyclopropylamino-ethyl | chloro |
| 1.240 | 2-cyclobutylamino-ethyl | chloro |
| 1.241 | 2-*tert*-butylamino-ethyl | chloro |
| 1.242 | 2-allylamino-ethyl | chloro |
| 1.243 | 2-prop-2-ynylamino-ethyl | chloro |
| 1.244 | 2-(1-methyl-1H-pyrazol-2-ylamino)-ethyl | chloro |
| 1.245 | 2-(2-methyl-2H-pyrazol-3-ylamino)-ethyl | chloro |
| 1.246 | 2-dimethylamino-ethyl | chloro |
| 1.247 | 2-(ethyl-methyl-am ino)-ethyl | chloro |
| 1.248 | 2-[(2-hydroxy-ethyl)-methyl-amino]-ethyl | chloro |
| 1.249 | 2-diethylamino-ethyl | chloro |
| 1.250 | 2-[(2-hydroxy-ethyl)-ethyl-amino]-ethyl | chloro |
| 1.251 | 2-[(2-methoxy-ethyl)-ethyl-amino]-ethyl | chloro |
| 1.252 | 2-(allyl-methyl-am ino)-ethyl | chloro |
| 1.253 | 2-(methyl-prop-2-ynyl-am ino)-ethyl | chloro |
| 1.254 | 2-(isopropyl-methyl-am ino)-ethyl | chloro |
| 1.255 | 2-azetidin-1-yl-ethyl | chloro |
| 1.256 | 2-morpholin-4-yl-ethyl | chloro |
| 1.257 | 2-pyrrolidin-1-yl-ethyl | chloro |
| 1.258 | 2-(3.3-difluoro-pyrrolidin-1-yl)-ethyl | chloro |
| 1.259 | 2-(2,5-dihydro-pyrrol-1-yl)-ethyl | chloro |
| 1.260 | 2-piperidin-1-yl-ethyl | chloro |
| 1.261 | 2-(4-methyl-piperidin-1-yl)-ethyl | chloro |
| 1.262 | 2-(3,6-dihydro-2H-pyridin-1-yl)-ethyl | chloro |
| 1.263 | 2-(4-methyl-piperazin-1-yl)-ethyl | chloro |
| 1.264 | 2-(4-acetyl-piperazin-1-yl)-ethyl | chloro |
| 1.265 | 2-azepan-1-yl-ethyl | chloro |
| 1.266 | 2-imidazol-1-yl-ethyl | chloro |
| 1.267 | 3-chloro-n-propyl | chloro |
| 1.268 | 3-amino-n-propyl | chloro |
| 1.269 | 3-methylamino-n-propyl | chloro |
| 1.270 | 3-ethylamino-n-propyl | chloro |
| 1.271 | 3-(2,2-difluoroethyl)-amino-n-propyl | chloro |
| 1.272 | 3-isopropylamino-n-propyl | chloro |
| 1.273 | 3-isobutylamino-n-propyl | chloro |
| 1.274 | 3-cyclopropylmethylamino-n-propyl | chloro |
| 1.275 | 3-(2-hydroxy-ethylamino)-n-propyl | chloro |
| 1.276 | 3-(2-hydroxy-ethylamino)-n-propyl | chloro |
| 1.277 | 3-cyclopropylamino-n-propyl | chloro |
| 1.278 | 3-cyclobutylamino-n-propyl | chloro |
| 1.279 | 3-*tert*-butylamino-n-propyl | chloro |
| 1.280 | 3-allylamino-n-propyl | chloro |
| 1.281 | 3-prop-2-ynylamino-n-propyl | chloro |
| 1.282 | 3-(1-methyl-1H-pyrazol-2-ylamino)-n-propyl | chloro |
| 1.283 | 3-(2-methyl-2H-pyrazol-3-ylamino)-n-propyl | chloro |
| 1.284 | 3-dimethylamino-n-propyl | chloro |
| 1.285 | 3-(ethyl-methyl-amino)-n-propyl | chloro |
| 1.286 | 3-[(2-hydroxy-ethyl)-methyl-amino]-n-propyl | chloro |
| 1.287 | 3-diethylamino-n-propyl | chloro |
| 1.288 | 3-[(2-hydroxy-ethyl)-ethyl-amino]-n-propyl | chloro |
| 1.289 | 3-[(2-methoxy-ethyl)-ethyl-amino]-n-propyl | chloro |
| 1.290 | 3-(allyl-methyl-amino)-n-propyl | chloro |
| 1.291 | 3-(methyl-prop-2-ynyl-am ino)-n-propyl | chloro |
| 1.292 | 3-(isopropyl-methyl-amino)-n-propyl | chloro |
| 1.293 | 3-azetidin-1-yl-n-propyl | chloro |
| 1.294 | 3-morpholin-4-yl-n-propyl | chloro |
| 1.295 | 3-pyrrolidin-1-yl-n-propyl | chloro |
| 1.296 | 3-(3.3-difluoro-pyrrolidin-1-yl)-n-propyl | chloro |
| 1.297 | 3-(2,5-dihydro-pyrrol-1-yl)-n-propyl | chloro |
| 1.298 | 3-piperidin-1-yl-n-propyl | chloro |
| 1.299 | 3-(4-methyl-piperidin-1-yl)-n-propyl | chloro |
| 1.300 | 3-(3,6-dihydro-2H-pyridin-1-yl)-n-propyl | chloro |
| 1.301 | 3-(4-methyl-piperazin-1-yl)-n-propyl | chloro |
| 1.302 | 3-(4-acetyl-piperazin-1-yl)-n-propyl | chloro |
| 1.303 | 3-azepan-1-yl-n-propyl | chloro |
| 1.304 | 3-imidazol-1-yl-n-propyl | chloro |
| 1.305 | 2-bromo-ethyl | bromo |
| 1.306 | 2-amino-ethyl | bromo |
| 1.307 | 2-methylamino-ethyl | bromo |
| 1.308 | 2-ethylamino-ethyl | bromo |
| 1.309 | 2-(2,2-difluoroethyl)-amino-ethyl | bromo |
| 1.310 | 2-isopropylamino-ethyl | bromo |
| 1.311 | 2-isobutylamino-ethyl | bromo |
| 1.312 | 2-cyclopropylmethylamino-ethyl | bromo |
| 1.313 | 2-(2-hydroxy-ethylamino)-ethyl | bromo |
| 1.314 | 2-(2-methoxy-ethylamino)-ethyl | bromo |
| 1.315 | 2-cyclopropylamino-ethyl | bromo |
| 1.316 | 2-cyclobutylamino-ethyl | bromo |
| 1.317 | 2-*tert*-butylamino-ethyl | bromo |
| 1.318 | 2-allylamino-ethyl | bromo |
| 1.319 | 2-prop-2-ynylamino-ethyl | bromo |
| 1.320 | 2-(1-methyl-1H-pyrazol-2-ylamino)-ethyl | bromo |
| 1.321 | 2-(2-methyl-2H-pyrazol-3-ylamino)-ethyl | bromo |
| 1.322 | 2-dimethylamino-ethyl | bromo |
| 1.323 | 2-(ethyl-methyl-am ino)-ethyl | bromo |
| 1.324 | 2-[(2-hydroxy-ethyl)-methyl-amino]-ethyl | bromo |
| 1.325 | 2-diethylamino-ethyl | bromo |
| 1.326 | 2-[(2-hydroxy-ethyl)-ethyl-amino]-ethyl | bromo |
| 1.327 | 2-[(2-methoxy-ethyl)-ethyl-amino]-ethyl | bromo |
| 1.328 | 2-(allyl-methyl-am ino)-ethyl | bromo |
| 1.329 | 2-(methyl-prop-2-ynyl-am ino)-ethyl | bromo |
| 1.330 | 2-(isopropyl-methyl-am ino)-ethyl | bromo |
| 1.331 | 2-azetidin-1-yl-ethyl | bromo |
| 1.332 | 2-morpholin-4-yl-ethyl | bromo |
| 1.333 | 2-pyrrolidin-1-yl-ethyl | bromo |
| 1.334 | 2-(3.3-difluoro-pyrrolidin-1-yl)-ethyl | bromo |
| 1.335 | 2-(2,5-dihydro-pyrrol-1-yl)-ethyl | bromo |
| 1.336 | 2-piperidin-1-yl-ethyl | bromo |
| 1.337 | 2-(4-methyl-piperidin-1-yl)-ethyl | bromo |
| 1.338 | 2-(3,6-dihydro-2H-pyridin-1-yl)-ethyl | bromo |
| 1.339 | 2-(4-methyl-piperazin-1-yl)-ethyl | bromo |
| 1.340 | 2-(4-acetyl-piperazin-1-yl)-ethyl | bromo |
| 1.341 | 2-azepan-1-yl-ethyl | bromo |
| 1.342 | 2-imidazol-1-yl-ethyl | bromo |
| 1.343 | 3-chloro-n-propyl | bromo |
| 1.344 | 3-amino-n-propyl | bromo |
| 1.345 | 3-methylamino-n-propyl | bromo |
| 1.346 | 3-ethylamino-n-propyl | bromo |
| 1.347 | 3-(2,2-difluoroethyl)-amino-n-propyl | bromo |
| 1.348 | 3-isopropylamino-n-propyl | bromo |
| 1.349 | 3-isobutylamino-n-propyl | bromo |
| 1.350 | 3-cyclopropylmethylamino-n-propyl | bromo |
| 1.351 | 3-(2-hyd roxy-ethylam ino)-n-propyl | bromo |
| 1.352 | 3-(2-hydroxy-ethylamino)-n-propyl | bromo |
| 1.353 | 3-cyclopropylamino-n-propyl | bromo |
| 1.354 | 3-cyclobutylamino-n-propyl | bromo |
| 1.355 | 3-*tert*-butylamino-n-propyl | bromo |
| 1.356 | 3-allylamino-n-propyl | bromo |
| 1.357 | 3-prop-2-ynylamino-n-propyl | bromo |
| 1.358 | 3-(1-methyl-1H-pyrazol-2-ylamino)-n-propyl | bromo |
| 1.359 | 3-(2-methyl-2H-pyrazol-3-ylamino)-n-propyl | bromo |
| 1.360 | 3-dimethylamino-n-propyl | bromo |
| 1.361 | 3-(ethyl-methyl-am ino)-n-propyl | bromo |
| 1.362 | 3-[(2-hydroxy-ethyl)-methyl-amino]-n-propyl | bromo |
| 1.363 | 3-diethylamino-n-propyl | bromo |
| 1.364 | 3-[(2-hydroxy-ethyl)-ethyl-amino]-n-propyl | bromo |
| 1.365 | 3-[(2-methoxy-ethyl)-ethyl-amino]-n-propyl | bromo |
| 1.366 | 3-(allyl-methyl-amino)-n-propyl | bromo |
| 1.367 | 3-(methyl-prop-2-ynyl-amino)-n-propyl | bromo |
| 1.368 | 3-(isopropyl-methyl-amino)-n-propyl | bromo |
| 1.369 | 3-azetidin-1-yl-n-propyl | bromo |
| 1.370 | 3-morpholin-4-yl-n-propyl | bromo |
| 1.371 | 3-pyrrolidin-1-yl-n-propyl | bromo |
| 1.372 | 3-(3.3-difluoro-pyrrolidin-1-yl)-n-propyl | bromo |
| 1.373 | 3-(2,5-dihydro-pyrrol-1-yl)-n-propyl | bromo |
| 1.374 | 3-piperidin-1-yl-n-propyl | bromo |
| 1.375 | 3-(4-methyl-piperidin-1-yl)-n-propyl | bromo |
| 1.376 | 3-(3,6-dihydro-2H-pyridin-1-yl)-n-propyl | bromo |
| 1.377 | 3-(4-methyl-piperazin-1-yl)-n-propyl | bromo |
| 1.378 | 3-(4-acetyl-piperazin-1-yl)-n-propyl | bromo |
| 1.379 | 3-azepan-1-yl-n-propyl | bromo |
| 1.380 | 3-imidazol-1-yl-n-propyl | bromo |
| 1.381 | methyl | methoxy |
| 1.382 | methyl | ethoxy |
| 1.383 | methyl | difluoromethoxy |
| 1.384 | methyl | chloro |
| 1.385 | methyl | bromo |
| 1.386 | ethyl | methoxy |
| 1.387 | ethyl | ethoxy |
| 1.388 | ethyl | difluoromethoxy |
| 1.389 | ethyl | chloro |
| 1.390 | ethyl | bromo |

### Starting compounds

The numbering of the heterocyclic system of the starting compounds may differ from the numbering used for the final compounds, due to the systematic names generated using Autonom in ISIS, Autonom Engine Version 4.0, Copyright (1998) Beilstein Institut Frankfurt am Main.
Thus, eg. starting compounds of type A are numbered as follows:

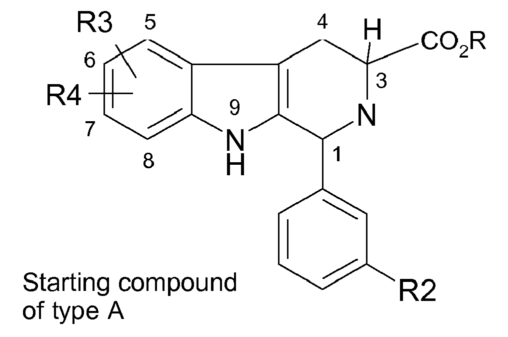

### A1. (1RS,3RS)-6-Fluoro-1-(3-hydroxy-phenyl)-2,3,4,9-tetrahydro-1H-β-carboline-3-carboxylic acid methyl ester and (1RS,3SR)-6-Fluoro-1-(3-hydroxy-phenyl)-2,3,4,9-tetrahydro-1H-β-carboline-3-carboxylic acid methyl ester

To a suspension of 460 mg (1.95 mmol) 2-amino-3-(5-fluoro-1 H-indol-3-yl)-propionic acid methyl ester (compound B1) and 3-hydroxybenzaldehyde (286 mg, 2.34 mmol) in 10 ml toluene are added 940 µl (2.67 mmol) trifluoroacetic acid. The resulting solution is heated to 45 °C for 14 h. The solution is cooled to room temperature and water is added. A saturated aqueous solution of sodium carbonate is added until the pH is basic. The mixture is extracted with ethyl acetate. The organic layer is washed with brine and dried with magnesium sulfate. The solvent is removed at reduced pressure. The residue (890 mg) is used for the next step without further purification.

Starting from the corresponding indolyl-propionic acid methyl ester B2 to B6, the following compounds can be obtained analogously as described exemplarily for the compounds A1.

### A2. (1RS,3RS)-7-Fluoro-1-(3-hydroxy-phenyl)-2,3,4,9-tetrahydro-1H-β-carboline-3-carboxylic acid methyl ester and (1RS,3SR)-7-Fluoro-1-(3-hydroxy-phenyl)-2,3,4,9-tetrahydro-1H-β-carboline-3-carboxylic acid methyl ester

### A3. (1RS,3RS)-6-Bromo-1-(3-hydroxy-phenyl)-2,3,4,9-tetrahydro-1H-β-carboline-3-carboxylic acid methyl ester and (1RS,3SR)-6-Bromo-1-(3-hydroxy-phenyl)-2,3,4,9-tetrahydro-1H-β-carboline-3-carboxylic acid methyl ester

### A4. (1RS,3RS)-6-Methyl-1-(3-hydroxy-phenyl)-2,3,4,9-tetrahydro-1H-β-carboline-3-carboxylic acid methyl ester and (1RS,3SR)-6-Methyl-1-(3-hydroxy-phenyl)-2,3,4,9-tetrahydro-1H-β-carboline-3-carboxylic acid methyl ester

### A5. (1RS,3RS)-5-Methyl-1-(3-hydroxy-phenyl)-2,3,4,9-tetrahydro-1H-β-carboline-3-carboxylic acid methyl ester and (1RS,3SR)-5-Methyl-1-(3-hydroxy-phenyl)-2,3,4,9-tetrahydro-1H-β-carboline-3-carboxylic acid methyl ester

### A6. (1RS,3RS)-7-Methyl-1-(3-hydroxy-phenyl)-2,3,4,9-tetrahydro-1H-β-carboline-3-carboxylic acid methyl ester and (1RS,3SR)-7-Methyl-1-(3-hydroxy-phenyl)-2,3,4,9-tetrahydro-1H-β-carboline-3-carboxylic acid methyl ester

### A7. (1RS,3RS)-1-(3-Hydroxy-phenyl)-6-methoxy-2,3,4,9-tetrahydro-1H-β-carboline-3-carboxylic acid methyl ester and (1RS,3SR)-1-(3-Hydroxy-phenyl)-6-methoxy-2,3,4,9-tetrahydro-1H-β-carboline-3-carboxylic acid methyl ester

To a suspension of 510 mg (2.10 mmol, 1.00 eq) 2-amino-3-(5-methoxy-1 H-indol-3-yl)-propionic acid methyl ester (compound B7) and 3-hydroxy benzaldehyde (308 mg, 2.52 mmol) in 10 ml toluene are added 1.00 ml (2.67 mmol, 1.67 eq) trifluoroacetic acid. The resulting solution is heated to 45 °C for 14 h. The solution is cooled to room temperature and water is added. A saturated aqueous solution of sodium carbonate is added until the pH was basic. The mixture is extracted with ethyl acetate. The organic layer is washed with brine and dried with magnesium sulfate. The solvent is removed at reduced pressure. After column chromatography (silica gel, toluene/ethyl acetate 2:1) 80 mg of the mixture of the title diastereomers is obtained, which can be used without separation in the next step.

### A8a1. (1R,3R)-1-(3-Hydroxy-phenyl)-2,3,4,9-tetrahydro-1H-β-carboline-3-carboxylic acid methyl ester (cis) and

### A8b1.(1S,3R)-1-(3-Hydroxy-phenyl)-2,3,4,9-tetrahydro-1H-β-carboline-3-carboxylic acid methyl ester (trans)

To a solution of 2.73 g commercially available D-tryptophane methyl ester in 30 ml dichloromethane are added 1.68 g 3-hydroxy benzaldehyde and 5.44 g trimethyl orthoformiate. The mixture is stirred at room temperature for 48 h. The solvents are removed under reduced pressure and the residue is dissolved in 50 ml dichloromethane. The solution is cooled to 0 °C and 5.28 g (1.54 ml) trifluoro acetic acid are added. The solution is allowed to warm up to room temperature and is stirred for 17 h. The mixture is washed with a solution of sodium bicarbonate and the organic layer is dried with sodium sulfate. The solvents are removed at reduced pressure. The cis-diastereomer (1R,3R)-1-(3-Hydroxy-phenyl)-2,3,4,9-tetrahydro-1H-β-carboline-3-carboxylic acid methyl ester is obtained as a colorless solid by crystallization from toluene/ethyl acetate. (m/z (MH⁺) = 323.1) The trans-diastereomer (1S,3R)-1-(3-Hydroxy-phenyl)-2,3,4,9-tetrahydro-1H-β-carboline-3-carboxylic acid methyl ester is obtained as a solorless solid by column chromatography of the mother liquid (silica gel; hexanes, ethyl acetate 1:1). (m/z (MH⁺) = 323.0)

### A8a. (1RS,3RS)-1-(3-Hydroxy-phenyl)-2,3,4,9-tetrahydro-1H-β-carboline-3-carboxylic acid methyl ester and

### A8b. (1SR,3RS)-1-(3-Hydroxy-phenyl)-2,3,4,9-tetrahydro-1H-β-carboline-3-carboxylic acid methyl ester

The title compounds are obtained starting from commercially available racemic DL-tryptophane methyl ester following the protocol described for the synthesis of (1 R,3R)-1-(3-Hydroxy-phenyl)-2,3,4,9-tetrahydro-1H-β-carboline-3-carboxylic acid methyl ester (compound A8a1) and (1S,3R)-1-(3-Hydroxy-phenyl)-2,3,4,9-tetrahydro-1H-β-carboline-3-carboxylic acid methyl ester (compound A8b1).

### A9a. (1RS,3RS)-6-Chloro-1-(3-hydroxy-phenyl)-2,3,4,9-tetrahydro-1H-β-carboline-3-carboxylic acid methyl ester and

### A9b. (1SR,3RS)-6-Chloro-1-(3-hydroxy-phenyl)-2,3,4,9-tetrahydro-1H-β-carboline-3-carboxylic acid methyl ester

The title compounds are obtained starting from (RS)-2-amino-3-(5-chloro-1 H-indol-1-yl)-propionic acid methyl ester (compound B8) following the protocol described for the synthesis of (1R,3R)-1-(3-Hydroxy-phenyl)-2,3,4,9-tetrahydro-1H-β-carboline-3-carboxylic acid methyl ester (compound A8a1) and (1S,3R)-1-(3-Hydroxy-phenyl)-2,3,4,9-tetrahydro-1H-β-carboline-3-carboxylic acid methyl ester (compound A8b1).

### A10a. (1RS,3RS)-6-Methoxy-1-(3-hydroxy-phenyl)-2,3,4,9-tetrahydro-1H-β-carboline-3-carboxylic acid methyl ester and

### A10b. (1SR,3RS)-6-Methoxy-1-(3-hydroxy-phenyl)-2,3,4,9-tetrahydro-1H-β-carboline-3-carboxylic acid methyl ester

The title compounds are obtained starting from (RS)-2-amino-3-(5-methoxy-1H-indol-1-yl)-propionic acid methyl ester (compound B7) following the protocol described for the synthesis of (1R,3R)-1-(3-Hydroxy-phenyl)-2,3,4,9-tetrahydro-1H-β-carboline-3-carboxylic acid methyl ester (compound A8a1) and (1S,3R)-1-(3-Hydroxy-phenyl)-2,3,4,9-tetrahydro-1H-β-carboline-3-carboxylic acid methyl ester (compound A8b1).

### A-I-1. (1RS,3SR)-1-(3-Hydroxy-phenyl)-6-methoxy-3-methyl-2,3,4,9-tetrahydro-1H-beta-carboline-3-carboxylic acid methyl ester and (1RS,3RS)-1-(3-hydroxy-phenyl)-6-methoxy-3-methyl-2,3,4,9-tetrahydro-1H-beta-carboline-3-carboxylic acid methyl ester

To a solution of (+/-)-2-amino-3-(5-methoxy-1H-indol-3-yl)-2-methyl-propionic acid methyl ester (5.178 g, 19.5 mmol) and 3-hydroxybenzaldehyde (2.85 g, 23.4 mmol, 1.2 equiv.) in dry dichloromethane (80 ml) trifluoroacetic acid (1.5 ml, 19.5 mol, 1 equiv.) is added and the mixture is stirred under argon at room temperature. When TLC indicates the disappearance of (+/-)-2-amino-3-(5-methoxy-1H-indol-3-yl)-2-methyl-propionic acid methyl ester (1 day), a little methanol is added to dissolve the product precipitated during the reaction. The mixture is then diluted with dichloromethane (750 ml) and is washed with 2 M aqueous HCl, aqueous NaHCO₃ and water, is dried and concentrated. Column chromatography (dichloromethane-ethyl acetate 9:1 → 7:1) of the residue gives 5.22 g (73 %) of (1RS,3SR)-1-(3-hydroxy-phenyl)-6-methoxy-3-methyl-2,3,4,9-tetrahydro-1H-beta-carboline-3-carboxylic acid methyl ester (m.p. 183-184 °C from ethyl acetate-light petroleum) and 1.07 g (15 %) of (1RS,3RS)-1-(3-hydroxy-phenyl)-6-methoxy-3-methyl-2,3,4,9-tetrahydro-1H-beta-carboline-3-carboxylic acid methyl ester (m.p. 175-177 °C from ethyl acetate-light petroleum).

According to NMR experiments (such as the nuclear Overhauser effect), the main product of the Pictet-Spengler reaction is the diasteromer with the configuration (1RS,3SR). This diastereomer has a higher retention factor (silica gel, ethyl acetate-light petroleum ether) than the minor product having the configuration (1RS,3RS).
(1RS,3SR)-1-(3-Hydroxy-phenyl)-6-methoxy-3-methyl-2,3,4,9-tetrahydro-1H-beta-carboline-3-carboxylic acid methyl ester may be separated into its enantiomers by preparative HPLC:
(-)-1-(3-Hydroxy-phenyl)-6-methoxy-3-methyl-2,3,4,9-tetrahydro-1H-β-carboline-3-carboxylic acid methyl ester
(+)-1-(3-Hydroxy-phenyl)-6-methoxy-3-methyl-2,3,4,9-tetrahydro-1H-β-carboline-3-carboxylic acid methyl ester
The absolute stereochemistry of (+)-1-(3-Hydroxy-phenyl)-6-methoxy-3-methyl-2,3,4,9-tetrahydro-1H-β-carboline-3-carboxylic acid methyl ester may be tentatively assigned to (1 R,3S)-1-(3-Hydroxy-phenyl)-6-methoxy-3-methyl-2,3,4,9-tetrahydro-1H-β-carboline-3-carboxylic acid methyl ester.

### A-I-2. (1RS,3SR)-6-Ethoxy-1-(3-hydroxy-phenyl)-3-methyl-2,3,4,9-tetrahydro-1H-beta-carboline-3-carboxylic acid methyl ester

A mixture of (+/-)-2-amino-3-(5-ethoxy-1H-indol-3-yl)-2-methyl-propionic acid methyl ester (829 mg, 3 mmol), 3-hydroxybenzaldehyde (439 mg, 3.6 mmol) and trifluoroacetic acid (223 µl, 3 mmol) in dry dichloromethane (40 ml) is stirred for 6 h under argon. Methanol (2 ml) is added to dissolve the precipitated product and the mixture is diluted with dichloromethane (150 ml). It is washed with saturated aqueous NaHCO₃ (2 x 50 ml) and water (2 x 50 ml), is dried, and the solvent is removed under reduced pressure. The residue is purified by column chromatography (dichloromethane-ethyl acetate, 7:1 → 4:1) to provide (1RS,3SR)-1-(3-hydroxy-phenyl)-6-ethoxy-3-methyl-2,3,4,9-tetrahydro-1H-beta-carboline-3-carboxylic acid methyl ester (1.05 g, 93%). M.p. 173-175 °C (from ethyl acetate-hexane).

### A-I-3. (1RS,3SR)-1-(3-Hydroxy-phenyl)-6-(2-methoxy-ethoxy)-3-methyl-2,3,4,9-tetrahydro-1H-beta-carboline-3-carboxylic acid methyl ester and (1RS,3RS)-1-(3-hydroxy-phenyl)-6-(2-methoxy-ethoxy)-3-methyl-2,3,4,9-tetrahydro-1H-beta-carboline-3-carboxylic acid methyl ester

To a solution of (+/-)-2-amino-3-[5-(2-methoxy-ethoxy)-1H-indol-3-yl]-2-methyl-propionic acid methyl ester (800 mg, 2.60 mmol) and 3-hydroxybenzaldehyde (383 mg, 3.14 mmol, 1.2 equiv) in dry dichloromethane (20 ml) trifluoroacetic acid (200 µl, 2.6 mmol, 1 equiv) is added and the mixture is stirred under argon at room temperature. When TLC indicated the disappearance of the starting material (1 day), a little methanol is added to dissolve the precipitated product. Then it is diluted with CH₂Cl₂ (250 ml) and washed with 2 M aqueous HCl, aqueous NaHCO₃ and water, dried, and concentrated. Column chromatography (dichloromethane-ethyl acetate, 7:1 →5:1) of the residue gives (1RS,3SR)-1-(3-hydroxy-phenyl)-6-(2-methoxy-ethoxy)-3-methyl-2,3,4,9-tetrahydro-1H-beta-carboline-3-carboxylic acid methyl ester (839 mg, 78 %) and (1RS,3RS)-1-(3-hydroxy-phenyl)-6-(2-methoxy-ethoxy)-3-methyl-2,3,4,9-tetrahydro-1 H-beta-carboline-3-carboxylic acid methyl ester (152 mg, 14 %).

### A-I-4. (1RS,3SR)-6-Chloro-1-(3-hydroxy-phenyl)-3-methyl-2,3,4,9-tetrahydro-1H-beta-carboline-3-carboxylic acid methyl ester

Starting from the appropriate starting compounds, the title compound is prepared analogously to the procedure described for compound A-I-1. M.p.: 240-241 °C

### A-I-5. (1RS,3SR)-6-Bromo-1-(3-hydroxy-phenyl)-3-methyl-2,3,4,9-tetrahydro-1H-beta-carboline-3-carboxylic acid methyl ester

Starting from the appropriate starting compounds, the title compound is prepared analogously to the procedure described for compound A-I-1. m/z (MH⁺) = 415.0/417.1, m.p.: 240-241 °C

Starting from the appropriate compounds B-I-6 to B-I-10, the following compounds A-I-6 to A-I-10 may be prepared using analogous procedures to those to attain to compound A-I-1.

### A-I-6. (1RS,3SR)-3-Ethyl-1-(3-hydroxy-phenyl)-6-methoxy-2,3,4,9-tetrahydro-1H-beta-carboline-3-carboxylic acid ethyl ester

### A-I-7. (1RS,3SR)-6-Ethoxy-3-ethyl-1-(3-hydroxy-phenyl)-2,3,4,9-tetrahydro-1H-beta-carboline-3-carboxylic acid ethyl ester

### A-I-8. (1RS,3SR)-3-Ethyl-1-(3-hydroxy-phenyl)-6-(2-methoxy-ethoxy)-2,3,4,9-tetrahydro-1H-beta-carboline-3-carboxylic acid ethyl ester

### A-I-9. (1RS,3SR)-6-Chloro-3-ethyl-1-(3-hydroxy-phenyl)-2,3,4,9-tetrahydro-1H-beta-carboline-3-carboxylic acid ethyl ester

### A-I-10. (1RS,3SR)-6-Bromo-3-ethyl-1-(3-hydroxy-phenyl)-2,3,4,9-tetrahydro-1H-beta-carboline-3-carboxylic acid ethyl ester

### A-I-11. (1RS,3SR)-6-Cyclopropylmethoxy-1-(3-hydroxy-phenyl)-3-methyl-2,3,4,9-tetrahydro-1H-β-carboline-3-carboxylic acid methyl ester

Starting from the appropriate starting compound (B-I-11), the title compound is prepared analogously to the procedure described for compound A-I-1. MS: m/z (MH⁺) = 406.9

### A-I-12. (1RS,3SR)-6-(1,1-Difluoro-methoxy)-1-(3-hydroxy-phenyl)-3-methyl-2,3,4,9-tetrahydro-1H-β-carboline-3-carboxylic acid methyl ester

Starting from the appropriate starting compound (B-I-12), the title compound is prepared analogously to the procedure described for compound A-I-1. MS: m/z (MH⁺) = 402.9

### A-I-13. (1RS,3SR)-6-Trifluoromethoxy-1-(3-hydroxy-phenyl)-3-methyl-2,3,4,9-tetrahydro-1H-β-carboline-3-carboxylic acid methyl ester

Starting from the appropriate starting compound (B-I-13), the title compound may be prepared analogously to the procedure described for compound A-I-1.

Starting from the appropriate compounds B-I-14 to B-I-16, the following compounds A-I-14 to A-I-16 may be prepared using analogous procedures to those to attain to compound A-I-1.

### A-I-14. (1RS,3SR)-6-Cyclopropylmethoxy-3-ethyl-1-(3-hydroxy-phenyl)-2,3,4,9-tetrahydro-1H-β-carboline-3-carboxylic acid ethyl ester

### A-I-15. (1RS,3SR)-6-(1,1-Difluoro-methoxy)-3-ethyl-1-(3-hydroxy-phenyl)-2,3,4,9-tetrahydro-1H-β-carboline-3-carboxylic acid ethyl ester

### A-I-16. (1RS,3SR)-3-Ethyl-1-(3-hydroxy-phenyl)-6-trifluoromethoxy-2,3,4,9-tetrahydro-1H-β-carboline-3-carboxylic acid ethyl ester

### A-I-17. (1RS,3SR)-5-Fluoro-1-(3-hydroxy-phenyl)-6-methoxy-3-methyl-2,3,4,9-tetrahydro-1H-beta-carboline-3-carboxylic acid methyl ester

Starting from the appropriate starting compounds, the title compound is prepared analogously to the procedure described for compound A-I-1. m/z (MH⁺) = 385

### A-I-18. (1RS,3SR)-7-Fluoro-1-(3-hydroxy-phenyl)-6-methoxy-3-methyl-2,3,4,9-tetrahydro-1H-beta-carboline-3-carboxylic acid methyl ester

Starting from the appropriate starting compounds, the title compound is prepared analogously to the procedure described for compound A-I-1. m/z (MH+) = 385.0

### A-I-19. (1RS,3SR)-6-Chloro-7-fluoro-1-(3-hydroxy-phenyl)-3-methyl-2,3,4,9-tetrahydro-1H-beta-carboline-3-carboxylic acid methyl ester

Starting from the appropriate starting compounds, the title compound is prepared analogously to the procedure described for compound A-I-1. m/z (MH+) = 389

### A-II-1. (1RS,3SR)-6-Methoxy-3-methyl-1-phenyl-2,3,4,9-tetrahydro-1H-β-carboline-3-carboxylic acid methyl ester and (1RS,3RS)-6-methoxy-3-methyl-1-phenyl-2,3,4,9-tetrahydro-1H-β-carboline-3-carboxylic acid methyl ester

To a solution of 1.0 g (3.81 mmol) (RS)-2-amino-3-(5-methoxy-1 H-indol-3-yl)-2-methyl-propionic acid methyl ester in 15 ml dichloromethane are added 470 µl (4.57 mmol) benzaldehyde. 300 µl (3.81 mmol) trifluoro acetic acid are added. The mixture is stirred at room temperature over night. Water and a saturated aqueous solution of sodium hydrogencarbonate are added and the aqueous layer is extracted with dichloromethane. The combined organic layers are washed with brine and dried with magnesium sulfate. The solvent is removed at reduced pressure. After column chromatography (silica gel, toluene/ethyl acetate 9:1) 625 mg (1 RS,3SR)-6-methoxy-3-methyl-1-phenyl-2,3,4,9-tetrahydro-1H-β-carboline-3-carboxylic acid methyl ester (m.p. 194-197°C, m/z (MH⁺) = 350.9) and 92 mg (1RS,3RS)-6-methoxy-3-methyl-1-phenyl-2,3,4,9-tetrahydro-1H-β-carboline-3-carboxylic acid methyl ester (m.p. 172-175°C, m/z (MH⁺) = 350.9) are obtained as colorless solids.

Starting from the appropriate compounds B-I-1 to B-I-5, the following compounds A-II-2 to A-II-5 may be prepared using analogous procedures to those to attain to compound A-II-1.

### A-II-2. (1RS,3SR)-6-Ethoxy-3-methyl-1-phenyl-2,3,4,9-tetrahydro-1H-beta-carboline-3-carboxylic acid methyl ester

### A-II-3.(1RS,3SR)-6-(2-Methoxy-ethoxy)-3-methyl-1-phenyl-2,3,4,9-tetrahydro-1H-beta-carboline-3-carboxylic acid methyl ester

### A-II-4. (1RS,3SR)-6-Chloro-3-methyl-1-phenyl-2,3,4,9-tetrahydro-1H-beta-carboline-3-carboxylic acid methyl ester

### A-II-5. (1 RS,3SR)-6-Bromo-3-methyl-1-phenyl-2,3,4,9-tetrahydro-1 H-beta-carboline-3-carboxylic acid methyl ester

Starting from the appropriate compounds B-I-6 to B-I-10, the following compounds A-II-6 to A-II-10 may be prepared using analogous procedures to those to attain to compound A-II-1.

### A-II-6. (1RS,3SR)-3-Ethyl-6-methoxy-1-phenyl-2,3,4,9-tetrahydro-1H-beta-carboline-3-carboxylic acid ethyl ester; MS: m/z (MH+) = 379.0

### A-II-7. (1RS,3SR)-6-Ethoxy-3-ethyl-3-methyl-1-phenyl-2,3,4,9-tetrahydro-1H-beta-carboline-3-carboxylic acid ethyl ester

### A-II-8. (1RS,3SR)-3-Ethyl-6-(2-methoxy-ethoxy)-1-phenyl-2,3,4,9-tetrahydro-1H-beta-carboline-3-carboxylic acid ethyl ester

### A-II-9. (1RS,3SR)-6-Chloro-3-Ethyl-1-phenyl-2,3,4,9-tetrahydro-1H-beta-carboline-3-carboxylic acid ethyl ester

### A-II-10. (1RS,3SR)-6-Bromo-3-ethyl-1-phenyl-2,3,4,9-tetrahydro-1H-beta-carboline-3-carboxylic acid ethyl ester

### B1. 2-Amino-3-(5-fluoro-1H-indol-3-yl)-propionic acid methyl ester

To a suspension of 1.02 g (4.60 mmol) commercially available 5-fluoro-DL-tryptophan in 15 ml methanol at 0 °C are added dropwise 1.67 ml (23 mmol) thionyl chloride. The mixture is stirred at 0 °C for 1 h and for 14 h at room temperature. The solvent is removed at reduced pressure and the residue is dissolved in ethyl acetate. The solution is washed with a saturated aqueous solution of sodium carbonate and with brine. The organic layer is dried with magnesium sulfate and the solvent is removed at reduced pressure. 1.0 g of the title compound are obtained as pale crystals.

Starting from the appropriate art-known tryptophane derivatives, the following ester compounds can be obtained as described exemplarily for compound B1.

### B2. 2-Amino-3-(6-fluoro-1H-indol-3-yl)-propionic acid methyl ester

### B3. 2-Amino-3-(5-bromo-1H-indol-3-yl)-propionic acid methyl ester

### B4. 2-Amino-3-(5-methyl-1H-indol-3-yl)-propionic acid methyl ester

### B5. 2-Amino-3-(4-methyl-1H-indol-3-yl)-propionic acid methyl ester

### B6. 2-Amino-3-(6-methyl-1H-indol-3-yl)-propionic acid methyl ester

### B7. 2-Amino-3-(5-methoxy-1H-indol-3-yl)-propionic acid methyl ester

To a suspension of 1.06 g (4.30 mmol, 1.00 eq) commercially available 5-methoxy-DL-tryptophan in 15 ml methanol at 0 °C are added dropwise 1.56 ml (21.5 mmol, 5.00 eq) thionyl chloride. The mixture is stirred at 0 °C for 1 h and for 14 h at room temperature. The solvent is removed at reduced pressure and the residue was dissolved in ethyl acetate. The solution is washed with a saturated aqueous solution of sodium carbonate and with brine. The organic layer is dried with magnesium sulfate and the solvent is removed at reduced pressure. 1.21 g of the desired product are obtained as pale crystals.

### B8. 2-amino-3-(5-chloro-1H-indol-1-yl)-propionic acid methyl ester

A solution of 5 g (RS)-2-amino-3-(5-chloro-1 H-indol-1-yl)-propionic acid in 200 ml methanol is cooled to 0 °C and 12.5 ml thionyl chloride are added. The mixture is stirred for 30 °C at 0 °C and at room temperature over night. The solvent is removed at reduced pressure and the residue is co-evaporated with toluene for three times. The title compound is obtained as a colorless solid. (

### B-I-1. (+/-)-2-Amino-3-(5-methoxy-1H-indol-3-yl)-2-methyl-propionic acid methyl ester

To a solution of (+/-)-3-(5-methoxy-1 H-indol-3-yl)-2-methyl-2-nitro-propionic acid methyl ester (4.26 g) in methanol (80 mL) wet Raney nickel (ca 12 g) is added, and the mixture is stirred under hydrogen at atmospheric pressure at room temperature overnight. The solid is filtered through Celite, is washed with methanol, and the filtrate is concentrated. Column chromatography of the residue (dichloromethane-methanol, 98:2 → 95:5) gives the title compound (3.45 g, 90%). M.p. 131-132 °C (from ethyl acetate-light petroleum).

### B-I-2. (+/-)-2-Amino-3-(5-ethoxy-1H-indol-3-yl)-2-methyl-propionic acid methyl ester

To a stirred solution of (+/-)-3-(5-ethoxy-1H-indol-3-yl)-2-methyl-2-nitro-propionic acid methyl ester (5.3 g, 17.3 mmol) in dry methanol (50 ml) Raney nickel is added and the mixture is stirred at room temperature under H₂ at atmospheric pressure overnight. The reaction mixture is filtered through a pad of Celite and the solid is washed with methanol. The filtrate is concentrated and the residue is purified by column chromatography (dichloromethane-methanol, 95:5) to give (+/-)-2-amino-3-(5-ethoxy-1 H-indol-3-yl)-2-methyl-propionic acid methyl ester (4.2 g, 90 %) as a white crystals. M.p. 165-166 °C (from ethyl acetate-hexane).

### B-I-3. (+/-)-2-amino-3-[5-(2-methoxy-ethoxy)-1H-indol-3-yl]-2-methyl-propionic acid methyl ester

To a stirred solution of (+/-)-3-[5-(2-methoxy-ethoxy)-1H-indol-3-yl]-2-methyl-2-nitro-propionic acid methyl ester (12.7 g, 37.8 mmol) in dry methanol (200 ml) Raney nickel (ca 20 g) is added and the mixture is stirred at room temperature under H₂ at atmospheric pressure overnight. The reaction mixture is filtered through a pad of Celite and the solid is washed with methanol. The filtrate is concentrated and the residue is purified by column chromatography (dichloromethane-methanol, 9:1) to give (+/-)-2-amino-3-[5-(2-methoxy-ethoxy) -1 H-indol-3-yl]-2-methyl-propionic acid methyl ester (5.98 g, 52 %). M.p. 117-118 (from ethyl acetate - light petroleum).

### B-I-4. (+/-)-2-Amino-3-(5-chloro-1H-indol-3-yl)-2-methyl-propionic acid methyl ester

Starting from the appropriate starting compounds, the title compound is prepared analogously to the procedure described for compound B-I-1. M.p.: 170°C

### B-I-5. (+/-)-2-Amino-3-(5-bromo-1H-indol-3-yl)-2-methyl-propionic acid methyl ester

Starting from the appropriate starting compounds, the title compound is prepared analogously to the procedure described for compound B-I-1. m/z (MH⁺) = 311.0/313.0, m.p.: 181°C

Starting from the appropriate compounds C6 to C10, the following compounds B-I-6 to B-I-10 may be prepared using analogous procedures to those to attain to compound B-I-1.

### B-I-6. (+/-)-2-Amino-2-ethyl-3-(5-methoxy-1H-indol-3-yl)-propionic acid ethyl ester

In more detail, the title compound, i.e. (RS)-2-Amino-2-(5-methoxy-1H-indol-3-ylmethyl)-butyric acid ethyl ester, is obtained as follows:
Raney nickel is added to a solution of 13.1 g (RS)-2-(5-methoxy-1H-indol-3-ylmethyl)-2-nitro-butyric acid ethyl ester in 150 ml methanol. The mixture is stirred for 15 h under a hydrogen atmosphere (atmospheric pressure) and filtered through celite. The solvent is removed under reduced pressure. 8.36 g of the title compound are obtained as a colourless oil. MS: m/z (MH⁺) = 291.0

### B-I-7. (+/-)-2-Amino-3-(5-ethoxy-1H-indol-3-yl)-2-ethyl-propionic acid ethyl ester

### B-I-8. (+/-)-2-Amino-2-ethyl-3-[5-(2-methoxy-ethoxy)-1H-indol-3-yl]-propionic acid ethyl ester

### B-I-9. (+/-)-2-Amino-3-(5-chloro-1H-indol-3-yl)-2-ethyl-propionic acid ethyl ester

### B-I-10. (+/-)-2-Amino-3-(5-bromo-1H-indol-3-yl)-2-ethyl-propionic acid ethyl ester

### B-I-11. (RS)-2-Amino-3-(5-cyclopropylmethoxy-1H-indol-3-yl)-2-methyl-propionic acid methyl ester

Starting from the appropriate starting compounds, the title compound is prepared analogously to the procedure described for compound B-I-1. M.p. 172 °C (from dichloromethane - light petroleum). ¹H-NMR (CDCl₃): 0.36 (m, 2H, cyclopropyl CH₂), 0.64 (m, 2H, cyclopropyl CH₂), 1.26 (m, 1 H, cyclopropyl CH), 1.44 (s, 3H, CMe), 2.95 and 3.23 (2d, 2H, CCH₂), 3.61 (s, 3H, OMe), 3.84 (d, 2H, CH₂O), 6.85-7.3 (m, 4H, aromatic), 7.95 (bs, 1H, NH).

### B-I-12. (RS)-2-Amino-3-[5-(1,1-difluoro-methoxy)-1H-indol-3-yl]-2-methyl-propionic acid methyl ester

Starting from the appropriate starting compounds, the title compound is prepared analogously to the procedure described for compound B-I-1. M.p. 140-142 °C (from ethyl acetate - light petroleum). ¹H-NMR (CDCl₃): 1.46 (s, 3H, C*Me*), 2.93 and 3.30 (2d, 2H, *J* = 14.3 Hz, CH₂), 3.60 (bs, 2H, NH₂), 3.66 (s, 3H, OMe), 6.53 (t, 1H, *J*_{H,F} = 75 Hz, CHF₂), 6.95 (dd, 1 H, aromatic), 7.08 (bs, 1H, NH), 7.30 (m, 3H, aromatic). ¹³C-NMR (CDCl₃): 26.2 (CCH₃), 36.1 (CH₂), 52.2 (OMe), 58.6 (*C*NH₂), 109.6, 112.1, 115.0, 125.5 (aromatic CHs), 109.9, 128.2, 133.9, 144.9 (quaternary aromatic carbons), 168.1 (COOMe).

### B-I-13. (RS)-2-Amino-3-(5-trifluoromethoxy-1H-indol-3-yl)-2-methyl-propionic acid methyl ester

Starting from the appropriate starting compounds, the title compound may be prepared analogously to the procedure described for compound B-I-1.

Starting from the appropriate compounds C14 to C16, the following compounds B-I-14 to B-I-16 may be prepared using analogous procedures to those to attain to compound B-I-1.

### B-I-14. (+/-)-2-Amino-3-(5-cyclopropylmethoxy-1H-indol-3-yl)-2-ethyl-propionic acid ethyl ester

### B-I-15. (+/-)-2-Amino-3-[5-(1,1-difluoro-methoxy)-1H-indol-3-yl]-2-ethyl-propionic acid ethyl ester

### B-I-16. (+/-)-2-Amino-2-ethyl-3-(5-trifluoromethoxy-1H-indol-3-yl)-propionic acid ethyl ester

### B-I-17. (RS)-2-Amino-2-(5-methoxy-1H-indol-3-ylmethyl)-3-methyl-butyric acid ethyl ester

Starting from the appropriate starting compounds, the title compound is prepared analogously to the procedure described for compound B-I-1. MS: m/z (MH⁺) = 305.0

### B-I-18. (RS)-2-Amino-3-(4-fluoro-5-methoxy-1H-indol-3-yl)-2-methyl-propionic acid methyl ester

Starting from the appropriate starting compounds, the title compound is prepared analogously to the procedure described for compound B-I-1. m/z (MH+) = 264

### B-I-19. (RS)-2-Amino-3-(6-fluoro-5-methoxy-1H-indol-3-yl)-2-methyl-propionic acid methyl ester

Starting from the appropriate starting compounds, the title compound is prepared analogously to the procedure described for compound B-I-1. m/z (MH⁺) = 264

### B-I-20. (RS)-2-Amino-3-(5-chloro-6-fluoro-1H-indol-3-yl)-2-methyl-propionic acid methyl ester

Starting from the appropriate starting compounds, the title compound is prepared analogously to the procedure described for compound B-I-1. m/z (MH⁺) = 284.8

### C1. (+/-)-3-(5-Methoxy-1H-indol-3-yl)-2-methyl-2-nitro-propionic acid methyl ester

A solution of commercially available 5-methoxy gramine (6.24 g) and commercially available methyl 2-nitro-propionate (4.07 g) in a mixture of toluene (50 ml) and N,N-dimethylformamide (2 ml) is refluxed for one day while bubbling argon through the reaction mixture. The solvent is evaporated, the residue is taken up in dichloromethane (300 ml), is washed subsequently with 2 M aqueous HCl, 2 M aqueous NaOH, and water, is dried and concentrated. Column chromatography of the residue (toluene-acetone, 98:2 → 95:5) gives the title compound (3.42 g, 38%). M.p. 109-110 °C (from ethyl acetate - light petroleum).

### C2. (+/-)-3-(5-Ethoxy-1H-indol-3-yl)-2-methyl-2-nitro-propionic acid methyl ester

A mixture of (5-ethoxy-1H-indol-ylmethyl)-dimethyl-amine (2.18 g, 10 mmol) and commercially available methyl 2-nitro-propionate (1.60 g, 12 mmol, 1.2 equiv) in dry toluene (17 ml) is refluxed. When TLC (toluene-acetone, 9:1) indicates the absence of starting material the mixture is cooled and is diluted with chloroform (35 ml). It is subsequently washed with 10 % aqueous HCl (2 x 10 ml), water (10 ml), 5 % aqueous NaOH (2 x 10 ml), water (10 ml), and 20 % aqueous Na₂SO₄ (10 ml), is dried, and the solvents are removed under reduced pressure. The residue is purified by column chromatography (light petroleum-ethyl acetate, 4:1 → 7:3) to give (+/-)-3-(5-ethoxy-1H-indol-3-yl)-2-methyl-2-nitro-propionic acid methyl ester (2.07 g, 68 %) as a white solid. M.p. 80-82 °C (from ethyl acetate-hexane).

### C3. (+/-)-3-[5-(2-Methoxy-ethoxy)-1H-indol-3-yl]-2-methyl-2-nitro-propionic acid methyl ester

To a solution of (5-(2-methoxy-ethoxy)-1H-indol-ylmethyl)-dimethyl-amine (15.2 g, 61.4 mmol) in a mixture of toluene (100 ml) and N,N-dimethylformamide (50 ml) methyl 2-nitropropionate (8.5 g, 63.9 mmol) is added. The mixture is refluxed for 2 days with stirring while a rapid stream of argon is passed through the solution. The solvent is evaporated, the residue is taken up in dichloromethane (600 ml), is washed subsequently with 2 M hydrochloric acid, 2 M aqueous NaOH, and water, is dried and evaporated. Column chromatography of the residue (toluene-acetone, 9: 1) provides (+/-)-3-[5-(2-methoxy-ethoxy)-1 H-indol-3-yl]-2-methyl-2-nitro-propionic acid methyl ester (9.34 g, 45%).

### C4. (+/-)-3-(5-Chloro-1H-indol-3-yl)-2-methyl-2-nitro-propionic acid methyl ester

Starting from the appropriate starting compounds, the title compound is prepared analogously to the procedure described for compound C1.

### C5. (+/-)-3-(5-Bromo-1H-indol-3-yl)-2-methyl-2-nitro-propionic acid methyl ester

Starting from the appropriate starting compounds, the title compound is prepared analogously to the procedure described for compound C1.

Starting from ethyl 2-nitrobutyrate and with choice of the appropriate amine compound D1 to D5 as reaction partner, the following compounds C6 to C10 may be prepared using similar procedures to those to attain to compound C1.

### C6. (+/-)-2-Ethyl-3-(5-methoxy-1H-indol-3-yl)-2-nitro-propionic acid ethyl ester

### C7. (+/-)-3-(5-Ethoxy-1H-indol-3-yl)-2-ethyl-2-nitro-propionic acid ethyl ester

### C8. (+/-)-2-Ethyl-3-[5-(2-methoxy-ethoxy)-1H-indol-3-yl]-2-nitro-propionic acid ethyl ester

### C9. (+/-)-3-(5-Chloro-1H-indol-3-yl)-2-ethyl-2-nitro-propionic acid ethyl ester

### C10. (+/-)-3-(5-Bromo-1H-indol-3-yl)-2-ethyl-2-nitro-propionic acid ethyl ester

### C11. (RS)-3-(5-Cyclopropylmethoxy-1H-indol-3-yl)-2-methyl-2-nitro-propionic acid methyl ester

Starting from compound D6, the title compound is prepared analogously to the procedure described for compound C1. ¹H-NMR (CDCl₃): 0.39 (m, 2H, cyclopropyl CH₂), 0.68 (m, 2H, cyclopropyl CH₂), 1.32 (m, 1H, cyclopropyl CH), 1.74 (s, 3H, CMe), 3.59 and 3.81 (2d, 2H, CCH₂), 3.82 (s, 3H, OMe), 3.82-3.87 (m, 2H, CH₂O), 6.86-7.3 (m, 4H, aromatic), 8.06 (bs, 1H, NH)

### C12. (RS)-3-[5-(1,1-Difluoro-methoxy)-1H-indol-3-yl]-2-methyl-2-nitro-propionic acid methyl ester

Starting from compound D7, the title compound is prepared analogously to the procedure described for compound C1. ¹H-NMR (CDCl₃): 1.73 (s, 3H, CMe), 3.57 and 3.75 (2d, 2H, J = 15 Hz, CH₂), 3.76 (s, 3H, OMe), 6.49 (t, 1H, *J*_{H,F} = 75 Hz, CHF₂), 6.92-7.36 (m, 3H, aromatic), 8.42 (bs, 1H, NH). ¹³C-NMR (CDCl₃): 21.3 (CCH₃), 32.2 (CH₂₂), 53.5 (OMe), 93.6 (CNO₂), 109.4, 112.3, 115.6, 126.2 (aromatic CHs), 107.4, 128.3, 133.6, 145.2 (quaternary aromatic carbons), 168.1 (COOMe)

### C13. (RS)-3-(5-Trifluoromethoxy-1H-indol-3-yl)-2-methyl-2-nitro-propionic acid methyl ester

Starting from compound D8, the title compound may be prepared analogously to the procedure described for compound C1.

Starting from ethyl 2-nitrobutyrate and with choice of the appropriate amine compound D6 to D8 as reaction partner, the following compounds C14 to C16 may be prepared using similar procedures to those to attain to compound C1.

### C14. (+/-)-3-(5-Cyclopropylmethoxy-1 H-indol-3-yl)-2-ethyl-2-nitro-propionic acid ethyl ester

### C15. (+/-)-3-[5-(1,1-Difluoro-methoxy)-1H-indol-3-yl]-2-ethyl-2-nitro-propionic acid ethyl ester

### C16. (+/-)-2-Ethyl-2-nitro-3-(5-trifluoromethoxy-1 H-indol-3-yl)-propionic acid ethyl ester

### C17. (RS)-2-(5-Methoxy-1H-indol-3-ylmethyl)-3-methyl-2-nitro-butyric acid ethyl ester

Starting from 3-methyl-2-nitro-butyric acid ethyl ester and compound D1, the title compound is prepared analogously to the procedure described for compound C1. In this case 1 equivalent potassium hydrogen carbonate is added to the reaction mixture. MS: m/z (MH⁺) = 334.9

### C18. (RS)-3-(4-Fluoro-5-methoxy-1H-indol-3-yl)-2-methyl-2-nitro-propionic acid methyl ester

Starting from the appropriate starting compounds, the title compound is prepared analogously to the procedure described for compound C1. m/z (MH⁺) = 310.7

### C19. (RS)-3-(6-Fluoro-5-methoxy-1H-indol-3-yl)-2-methyl-2-nitro-propionic acid methyl ester

Starting from the appropriate starting compounds, the title compound is prepared analogously to the procedure described for compound C1. m/z (MH⁺) = 310.6

### C20. (RS)-3-(6-Chloro-5-methoxy-1H-indol-3-yl)-2-methyl-2-nitro-propionic acid methyl ester

Starting from the appropriate starting compounds, the title compound is prepared analogously to the procedure described for compound C1. m/z (M-H⁺)⁻ = 313.2

The following compounds D1-D11, E1-E10, F1, G1, H1, I1, J1, K1 are commercially available or are prepared as follows:

### D1. (5-Methoxy-1H-indol-3-ylmethyl)-dimethyl-amine

The title compound (5-methoxy-gramine) is commercially available.

### D2. (5-Ethoxy-1H-indol-3-ylmethyl)-dimethyl-amine

A mixture of 5-ethoxy-indole (7.84 g, 48.7 mmol), 40% aqueous dimethylamine (9.25 ml, 73 mmol, 1.5 equiv), and 96% acetic acid (30 ml) is stirred at 0 °C, then 36% aqueous formaldehyde solution (6.33 ml, 82.7 mmol, 1.7 equiv) is added drop wise. The mixture is allowed to come to room temperature, and after stirring overnight TLC (dichloromethane-methanol, 4:1) indicates the absence of starting material. 10% Aqueous NaOH (150 ml) is added and the mixture is stirred at room temperature for 2 h. It is then extracted with dichloromethane (4 x 200 ml), the organic layer is dried and concentrated. The residue is purified by column chromatography (dichloromethane-methanol, 4:1 v:v → methanol-aqueous ammonia 50:1 v:v) to give crude product (10.18 g, 96 %), which is crystallized from acetone to provide pure (5-ethoxy-1H-indol-ylmethyl)-dimethyl-amine (10.2 g, 96 %) as white crystals. M.p. 95-97 °C.

### D3. [5-(2-Methoxy-ethoxy)-1H-indol-3-ylmethyl]-dimethyl-amine

A solution of 5-(2-methoxy-ethoxy)-indole (2.06 g, 11.0 mmol) in acetic acid (7 ml) and 40 % aqueous dimethylamine (2.1 ml) is cooled to 0 °C, and 36 % aqueous formaldehyde (1.38 ml) (precooled to 0 °C) is added drop wise. The mixture is stirred at room temperature overnight, 2 M hydrochloric acid is added, and the mixture is washed with dichloromethane. The aqueous layer is made alkaline with 10 % NaOH, and is extracted with dichloromethane. The combined organic layer is washed with water, is dried and concentrated. The residue is purified by column chromatography (dichloromethane-methanol, 4:1 v:v → dichloromethane-methanol-water-aqueous ammonia, 10:20:1:1 v:v:v:v) to afford [5-(2-methoxy-ethoxy)-1H-indol-ylmethyl]-dimethyl-amine (2.42 g, 90 %). M.p. 163-164 °C (from toluene-N,N-dimethylformamide).

### D4. (5-Chloro-1H-indol-3-ylmethyl)-dimethyl-amine

Starting from the appropriate starting compounds, the title compound is prepared analogously to the procedure described for compound D2 or D3. M.p.: 127-130°C

### D5. (5-Bromo-1H-indol-3-ylmethyl)-dimethyl-amine

Starting from the appropriate starting compounds, the title compound is prepared analogously to the procedure described for compound D2 or D3. M.p.: 139°C

### D6. (5-Cyclopropylmethoxy-1H-indol-3-ylmethyl)-dimethyl amine

Starting from the appropriate starting compounds, the title compound is prepared analogously to the procedure described for compound D2 or D3. ¹H-NMR (CDCl₃): 0.36 (m, 2H, cyclopropyl CH₂), 0.64 (m, 2H, cyclopropyl CH₂), 1.26 (m, 1 H, cyclopropyl CH), 2.34 (s, 6H, 2 NMe₂), 3.8 (m, 2H, CH₂O), 6.8-7.4 (m, 4H, aromatic), 8.84 (bs, 1H, NH)

### D7. [5-(1,1-Difluoro-methoxy)-1H-indol-3-ylmethyl]-dimethyl amine

Starting from the appropriate starting compounds, the title compound is prepared analogously to the procedure described for compound D2 or D3. ¹H-NMR (CDCl₃ + CD₃OD): 2.30 (s, 6H, NMe₂), 3.66 (s, 2H, CH₂), 6.53 (t, 1 H, *J*_{H,F} = 75 Hz, CHF₂), 6.95 (dd, 1 H, aromatic), 7.2-7.4 (m, 3H, aromatic). ¹³C-NMR (CDCl₃): 44.4 (NMe₂), 53.6 (CH₂), 109.2, 109.7, 112.1, 114.8, 126.6, 128.1, 133.9, 145.0 (aromatic)

### D8. [5-Trifluoromethoxy-1H-indol-3-ylmethyl]-dimethyl amine

Starting from the appropriate starting compounds, the title compound may be prepared analogously to the procedure described for compound D2 or D3.

### D9. (4-Fluoro-5-methoxy-1H-indol-3-ylmethyl)-dimethyl amine

Starting from the appropriate starting compounds, the title compound is prepared analogously to the procedure described for compound D2 or D3. m/z (MH⁺) = 222.8

### D10. (6-Fluoro-5-methoxy-1H-indol-3-ylmethyl)-dimethyl amine

Starting from the appropriate starting compounds, the title compound is prepared analogously to the procedure described for compound D2 or D3. m/z (MH+) = 222.6

### D11. (5-Chloro-5-fluoro-1H-indol-3-ylmethyl)-dimethyl amine

Starting from the appropriate starting compounds, the title compound is prepared analogously to the procedure described for compound D2 or D3. m/z (MH+) = 226.8

### E1. 5-Ethoxy-indole

A mixture of commercially available 5-hydroxy-indole (18 g, 13.5 mmol), anhydrous K₂CO₃ (93.5 g, 5 equiv) and iodoethane (40.5 ml, 3.75 equiv) in acetone (180 mL) is stirred at 50 °C under argon. When TLC (dichloromethane-methanol, 95:5 v:v) indicates the disappearance of 5-hydroxy-indole (4 days), the mixture is filtered, the solid is washed with acetone, then the filtrate is concentrated to give 17.67 g (90 %) of the title compound, which is sufficiently pure to be used in the next step. M.p. 144-146 °C (from ethanol).

### E2. 5-(2-Methoxy-ethoxy)-1H-indole

To a solution of 5-hydroxy-indole (15.2 g, 114 mmol) in 250 ml of dry acetone 2-methoxyethyl iodide (15 ml, 141 mmol, 1.25 equiv) and anhydrous K₂CO₃ (46.7 g, 338 mmol, 3 equiv) are added and the mixture is refluxed. Additional amounts of 0.5 equiv of 2-methoxyethyl iodide and K₂CO₃ are added each day. After 6 days TLC (toluene-acetone, 9:1 v:v) indicates the absence of starting material. The solid is removed by filtration, and the solvent is evaporated. The residue is taken up in dichloromethane (800 ml) and the solution is washed with 2 M aqueous HCl, 10 % aqueous NaHCO₃, and water. The organic layer is dried and concentrated. Column chromatography (toluene-acetone, 9:1 v:v) provides 5-(2-methoxy-ethoxy)-1H-indole (18.8 g, 86%). M.p. 58-60 °C (from ethyl acetate-light petroleum).

### E3. 5-Chloro-1H-indole

The title compound is commercially available.

### E4. 5-Bromo-1H-indole

The title compound is commercially available.

### E5. 5-Cyclopropylmethoxy-1H-indol

To a solution of 7.3 g 5-hydroxy-indole in 130 ml of dry acetone are added 10.5 ml bromomethyl cyclopropane and 22.7 g anhydrous potassium carbonate. The mixture is heated to reflux for 24 h and an additional amount of 5 ml bromomethyl cyclopropane are added. The mixture is heated to reflux for additional 4 days. The mixture is filtered and the solvent is removed under reduced pressure. The residue is dissolved in dichloro methane and washed with an aqueous solution of hydrochloric acid (2 M), 10 % aq. NaHCO₃ and water. The organic layer is dried and the solvent is removed under reduced pressure. After purification by column chromatography (silica gel; toluene, acetone 95:5 v:v), 9.62 g, 94 %) of the title compound are obtained as an oil. ¹H-NMR (CDCl₃): 0.36 (m, 2H, cyclopropyl CH₂), 0.64 (m, 2H, cyclopropyl CH₂), 1.30 (m, 1 H, cyclopropyl CH), 3.83 (d, 2H, J = 7.0 Hz, CH₂O), 6.45 (s, 1 H, aromatic), 6.90 (dd, 1 H, aromatic),.7.09-7.27 (m, 3H, aromatic), 8.05 (bs, 1H, NH). ¹³C-NMR (CDCl₃): 3.1 (2 cyclopropyl CH₂), 10.4 (cyclopropyl CH), 74.2 (CH₂O), 101.2, 101.6, 104.0, 104.6, 149.8 (aromatic)

### E6. 5-(1,1-Difluoro-methoxy)-1H-indol

Chlorodifluoromethane is bubbled trough an ice-cooled solution of 6.65 g 5-hydroxy-indole and 3.69 g tetrabutylammonium iodide in a mixture of 70 ml dioxane and 20 ml of an aqueous solution of sodium hydroxide (50 %). After TLC indicating the absence of starting material, 500 ml dichloromethane are added. The mixture is washed with water. The organic layer is dried and the solvent is removed under reduced pressure. After column chromatography (silica gel; toluene, acetone 99:1 v:v), 2.19 g (24 %) of the title compound are obtained as a colorless liquid. MS: [M+H]: 184.1, [M-H]: 182.0. ¹H-NMR (CDCl₃): 6.48 (t, 1 H, *J*_{H,F} = 75 Hz, CHF₂), 6.52 (m, 1 H, aromatic), 6.98 (dd, 1 H, aromatic), 7.2-7.4 (m, 3H, aromatic). ¹³C-NMR (CDCl₃): 103.0, 111.5, 111.9, 115.4, 117.1, 122.2, 126.0, 128.4, 133.6 (aromatic carbons)

### E7. 5-Trifluoromethoxy-1H-indol

The title compound may be obtained from 5-hydroxy-1H-indol by trifluoromethylation reaction.

### E8. 6-Fluoro-5-methoxy-1H-indole and

### E9. 4-Fluoro-5-methoxy-1H-indole

Both title compounds are prepared analogously to a procedure described in WO2003/064413 (p. 91f) for the preparation of 4-fluoro-5-methoxyindole and 6-fluoro-5-methoxyindole as a mixture. In this case, the regioisomeric intermediates (4-fluoro-5-methoxy-2-nitro-phenyl)-acetonitrile and (2-fluoro-3-methoxy-6-nitro-phenyl)-acetonitrile are separated by a sequence of crystallization of (4-fluoro-5-methoxy-2-nitro-phenyl)-acetonitrile (m/z (MH⁺) = 166.1) from 2-propanol followed by crystallization of (2-fluoro-3-methoxy-6-nitro-phenyl)-acetonitrile (m/z (MH⁺) = 166.1) from toluene using the mother liquid of the previous crystallization.

### E10. 5-Chloro-6-fluoro-1H-indole

To a suspension of 12.4 g sodium 1-acetyl-6-fluoro-1H-indole-2-sulfonate in 30 ml acetonitrile are added 7.1 g N-chlorsuccinimid. The mixture is stirred at room temperature for 2 hours and heated to 110 °C. 450 ml of an aqueous solution of sodium hydroxide (1 M) are added. The solution is stirred at 110 °C for 1 hour and cooled to 0 °C. The organic layer is separated and the solvent is removed. After purification of the residue by column chromatography (heptane/methyl tert.-butyl ether), 7.82 g (39 %) of the title compound are obtained. m/z (M-H⁺)⁻ = 168.0

### F1. 2-Methoxyethyl iodide

The crude 2-methoxyethyl tosylate is dissolved in 1600 ml of acetone and Nal (300 g, 2 mol, 2 equiv) is added. The mixture is heated to reflux and the progress of the reaction is monitored by TLC (toluene-acetone, 9:1 v:v). After 3 h the mixture is cooled to room temperature and the solid is removed by filtration. The solvent is evaporated, the residue is taken up in dichloromethane (700 ml) and is washed with 10 % aqueous Na₂S₂O₃ and water. The organic layer is dried and the solvent evaporated. The residue is distilled at reduced pressure to yield 108 g (58 %) of 2-methoxyethyl iodide. B.p. 34-36 °C at 30 mbar.

### G1. Toluene-4-sulfonic acid 2-methoxy-ethyl ester

A slurry of p-toluenesulfonyl chloride (205 g, 1.08 mol) and pyridine (150 mL) is stirred under an argon atmosphere. The temperature is maintained below 5 °C (ice-water bath), while ethylene glycol monomethyl ether (80 ml, 1 mol) is added slowly from a dropping funnel. After the addition is complete, the mixture is stirred for 1 h below 5 °C. The mixture is poured into ice-water (1 L) and is extracted with dichloromethane (1.2 I). The organic layer is washed with ice-cold 6 M HCl (3x350 ml), and is reduced to a minimum volume by evaporation in vacuo.

### H1. 3-Methyl-2-nitro-butyric acid ethyl ester

To an ice cooled solution of 5.31 g sodium nitrite and 8 g dried phloroglucinol in 70 ml dimethyl formamide is added a solution of 11.3 g 2-iodo-3-methyl-butyric acid ethyl ester in 30 ml dimethyl formamide. The solution is allowed to warm up to room temperature and is stired over night. The solvent is removed at reduced pressure. The residue is dissolved in ethyl acetate and washed with water. The organic layer is dried and the solvent is removed. The title compound is obtained as an oil. MS: m/z (M⁺) = 176.1

### I1. 2-lodo-3-methyl-butyric acid ethyl ester

A mixture of 10 g commercially available ethyl-2-bromo isovalerate and 17.8 g sodium iodide in 150 ml acetone are heated to reflux over night. The solvent is removed under reduced pressure. Dichloromethane is added to the residue and the solution is washed with an aqueous solution (10 %) of sodium thiosulfate and brine. The organic layer is dried and the solvent is removed under reduced pressure. 11.34 g (93 %) of the title compound are obtained as a yellowish oil. MS: m/z (M⁺) = 255.9

### J1. Sodium 1-acetyl-6-fluoro-1H-indole-2-sulfonate

A mixture of 14.0 g 6-fluoro-1H-indole-2-sulfonate and 87 ml acetic anhydride are stirred for 20 min at 70 °C. 35 ml additional acetic anhydride are added and the temperature is kept at 70 °C for 15 min. Additional 46 ml acetic anhydride are added and the temperature is increased to 110 °C. After 1 hour, the temperature is reduced to 90 °C for additional 90 min. After cooling to room temperature, 180 ml diethyl ether are added. The precipitate is filtered and dried under reduced pressure. 12.5 g (76 %) of the title compound are obtained as a colourless solid. m/z (M-H⁺)⁻ = 258

### K1. Sodium 6-Fluoro-1H-indole-2-sulfonate

To a solution of 23.4 g sodium bisulfite in 80 ml water a solution of 13.5 g 6-fluoro indole in ethanol is added drop wise. The obtained suspension is stirred at room temperature over night. The precipitate is filtered and washed with cold water, cold methanol and diethyl ether. 7.0 g (29 %) of the title compound are obtained as a colourless solid.

### Commercial utility

The compounds of formula I, of which I*, I**, I*** and I**** are preferred, and their pharmaceutically acceptable salts (= the compounds, or a salt, stereoisomer or a salt of a stereoisomer thereof, according to the invention ) have valuable pharmaceutical and/or pharmacological properties which make them commercially utilizable. It has been unexpectedly found that these derivatives are potent and highly efficacious inhibitors of cellular hyperproliferation and/or cell-cycle specific inducers of apoptosis in cancer cells. In particular, the compounds, or a salt, stereoisomer or a salt of a stereoisomer thereof, according to the invention act as inhibitors of the mitotic kinesin Eg5. The compounds, or a salt, stereoisomer or a salt of a stereoisomer thereof, according to the invention display cell-cycle dependent, anti-proliferative and/or apoptosis inducing activity. Thus, they are commercially applicable in the therapy of diseases responsive to the inhibition of this kinesin, such as the diseases mentioned below, in particular the treatment of hyperproliferative diseases and disorders responsive to the induction of apoptosis.

In the context of this invention, the term "hyperproliferation" is used to describe aberrant and/or dysregulated cellular growth, a hallmark of diseases like cancer. This hyperproliferation might be caused by single or multiple cellular / molecular alterations in respective cells and can be, in context of a whole organism, of benign or malignant behaviour. The term "inhibition of cell proliferation" is used herein to denote an ability of the compound to retard the growth of and/or kill a cell contacted with that compound as compared to cells not contacted with that compound. Most preferable this inhibition of cell proliferation is 100%, meaning that proliferation of all cells is stopped and/or cells undergo programmed cell death. In some preferred embodiments the contacted cell is a neoplastic cell. A neoplastic cell is defined as a cell with aberrant cell proliferation and/or the potential to metastasize to different tissues or organs. A benign neoplasia is described by hyperproliferation of cells, incapable of forming an aggressive, metastasizing tumor in-vivo. In contrast, a malignant neoplasia is described by cells with different cellular and biochemical abnormalities, e.g. capable of forming tumor metastasis. The aquired functional abnormalities of malignant neoplastic cells (also defined as "hallmarks of cancer") are limitless replicative potential, self-sufficiency in growth signals, insensitivity to anti-growth signals, evasion from apoptosis, sustained angiogenesis and tissue invasion and metastasis.

Hyperproliferative diseases and/or disorders responsive to the induction of apoptosis are diseases and/or disorders resulting from the abovementioned cellular conditions.

The term "inducer of apoptosis" is used herein to identify a compound which induces programmed cell death in cells contacted with that compound. Apoptosis is defined by complex biochemical events within the contacted cell, such as the activation of cystein specific proteinases ("caspases") and the fragmentation of chromatin. Induction of apoptosis in cells contacted with the compound might not necessarily be coupled with inhibition of cell proliferation. Preferably, the inhibition of cell proliferation and/or induction of apoptosis is specific to cells with aberrant cell growth (hyperproliferation). Thus, compared to cells with aberrant cell growth, normal proliferating or arrested cells are less sensitive or even insensitive to the proliferation inhibiting or apoptosis inducing activity of the compound. Finally, cytotoxic is used in a more general sense to identify compounds which kill cells by various mechanisms, including the induction of apoptosis / programmed cell death in a cell cycle dependent or cell-cycle independent manner.

The term "cell cycle specific" is used herein to identify a compound as inducing apoptosis and killing only proliferating cells actively passing a specific phase of the cell cycle, but not exerting this effect in resting, non-dividing cells. Continously proliferating cells are typical for diseases like cancer and characterized by cells passing all phases of the cell division cycle, namely in the G ("gap") 1, S ("DNA synthesis"), G2 and M ("mitosis") phase.

The mitotic kinesin Eg5 is an enzyme essential for the assembly and function of the bipolar mitotic spindle. Eg5 plays essential roles during various phases of mitosis. Drugs that perturb mitosis have proven clinically effective in the treatment of many cancers. Despite the diverse array of essential spindle proteins that could be exploited as targets for the discovery of novel cancer therapies, all spindle-targeted therapeutics in clinical use today act on only one protein, tubulin. Surprisingly, kinesin Eg5 expression is most abundant in proliferating human tissues, whereas it is absent from most postmitotic cells, such as e.g. human central nervous system neurons, pointing to an exclusive or almost confined role for Eg5 in cell proliferation. In contrary to drugs that directly interfere with microtubule dynamic instability, Eg5 kinesin inhibitors are expected not to disrupt microtubule-based cellular processes, e.g. neuronal transport, that are unrelated to proliferation. During mitosis, Eg5 is essentially involved in organizing microtubules into a bipolar structure that forms the mitotic spindle.
Experimental perturbation of Eg5 function causes a characteristic malformation or dysfunction of the mitotic spindle, frequently resulting in cell cycle arrest and cell death.

On account of their Eg5-inhibiting properties, the compounds, or a salt, stereoisomer or a salt of a stereoisomer thereof, according to the invention can be used to modulate mitotic spindle formation, thus causing prolonged cell cycle arrest in mitosis, which is frequently followed by apoptosis. By "modulate" herein is meant altering mitotic spindle formation, including increasing and decreasing spindle formation. By "mitotic spindle formation" herein is meant organization of microtubules into bipolar structures by mitotic kinesins. By "dysfunction of the mitotic spindle" herein is meant mitotic arrest and monopolar spindle formation. "Malformation of the mitotic spindle" encompasses the splaying of mitotic spindle poles, or otherwise causing morphological perturbation of the mitotic spindle.

On account of their Eg5-inhibiting properties, the compounds, or a salt, stereoisomer or a salt of a stereoisomer thereof, according to the invention can further be useful in the treatment of benign or malignant neoplasia.
A "neoplasia" is defined by cells displaying aberrant cell proliferation and/or survival and/or a block in differentiation. A "benign neoplasia" is described by hyperproliferation of cells, incapable of forming an aggressive, metastasizing tumor in-vivo. In contrast, a "malignant neoplasia" is described by cells with multiple cellular and biochemical abnormalities, capable of forming a systemic disease, for example forming tumor metastasis in distant organs.

The compounds, or a salt, stereoisomer or a salt of a stereoisomer thereof, according to the invention are capable of modulating, particularly inhibiting Eg5 activity. They are capable of modulating the mitotic spindle, particularly inhibiting mitosis.
Thus, the compounds, or a salt, stereoisomer or a salt of a stereoisomer thereof, according to the invention have cellular anti-proliferative properties. Thus, they modulate apoptosis and/or aberrant cell growth in the therapy of benign or malign neoplastic diseases, preferably cancer.

Various diseases are caused by aberrant cell proliferation ("hyperproliferation") as well as evasion from apoptosis. These diseases include e.g. benign hyperplasia like that of the prostate ("BPH") or colon epithelium, psoriasias, glomerulonephritis or osteoarthritis. Most importantly these diseases include malignant neoplasia commonly described as cancer and characterized by tumor cells finally metastasizing into distinct organs or tissues. Malignant neoplasia include solid and hematological tumors. Solid tumors are exemplified by tumors of the breast, bladder, bone, brain, central and peripheral nervous system, colon, endocrine glands (eg thyroid and adrenal cortex), esophagus, endometrium, germ cells, head and neck, kidney, liver, lung, larynx and hypopharynx, mesothelioma, sarcoma, ovary, pancreas, prostate, rectum, renal, small intestine, soft tissue, testis, stomach, skin, ureter, vagina and vulva. Malignant neoplasia include inherited cancers exemplified by retinoblastoma and Wilms tumor. In addition, malignant neoplasia include primary tumors in said organs and corresponding secondary tumors in distant organs ("tumor metastases"). Hematological tumors are exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, cancers of unknown primary site as well as AIDS related malignancies.

The invention therefore relates to a use of the compounds, or a salt, stereoisomer or a salt of a stereoisomer thereof, according to the invention in the manufacture of pharmaceutical compositions, a method of treatment or a combination according to the invention, in which the cancer to be treated is selected from the group consisting of cancer of the breast, bladder, bone, brain, central and peripheral nervous system, colon, endocrine glands, esophagus, endometrium, germ cells, head and neck, kidney, liver, lung, larynx and hypopharynx, mesothelioma, sarcoma, ovary, pancreas, prostate, rectum, renal, small intestine, soft tissue, testis, stomach, skin, ureter, vagina and vulva;
inherited cancers, retinomblastoma and Wilms tumor;
leukemia, lymphoma, non-Hodgkins disease, chronic and acute myeloid leukaemia, acute lymphoblastic leukemia, Hodgkins disease, multiple myeloma and T-cell lymphoma; myelodysplastic syndrome, plasma cell neoplasia, paraneoplastic syndromes, cancers of unknown primary site and AIDS related malignancies.

It is to be noted that a cancer disease as well as a malignant neoplasia does not necessarily require the formation of metastases in distant organs. Certain tumors exert devastating effects on the primary organ itself through their aggressive growth properties. These can lead to the destruction of the tissue and organ structure finally resulting in failure of the assigned organ function.

Neoplastic cell proliferation might affect normal cell behaviour and organ function. For example the formation of new blood vessels, a process described as neovascularization, is induced by tumors or tumor metastases. compounds, or a salt, stereoisomer or a salt of a stereoisomer thereof, according to the invention can be commercially applicable for the treatment of pathophysiological relevant processes caused by benign or neoplastic cell proliferation, such as but not limited to neovascularization by unphysiological proliferation of vascular endothelial cells.

Drug resistance is of particular importance for the frequent failure of standard cancer therapeutics. This drug resistance is caused by various cellular and molecular mechanisms like overexpression of drug efflux pumps or mutation within the cellular target protein. The commercial applicability of the compounds, or a salt, stereoisomer or a salt of a stereoisomer thereof, according to the invention is not limited to 1^{st} line treatment of patients. Patients with resistance to defined cancer chemotherapeutics or target specific anti-cancer drugs (2^{nd} or 3^{nd} line treatment) can be also amenable for treatment with the compounds, or a salt, stereoisomer or a salt of a stereoisomer thereof, according to the invention.

Due to their cellular anti-proliferative properties, compounds according to the present invention may be also commercially usable for treatment of diseases associated with cell cycle and cell proliferation, such as, besides cancer discussed above, for example, fibroproliferative and differentiative disorders, psoriasis, rheumatoid arthritis, atherosclerosis, hyperplasia, restenosis, cardiac hypertrophy, (auto)immune disorders, fungal disorders, bone diseases, or acute or chronic inflammation.

Thus, the invention relates to compounds, or a salt, stereoisomer or a salt of a stereoisomer thereof, according to the invention for use in the treatment of diseases.

In particular, the compounds according to the present invention are commercially applicable for the treatment of hyperproliferative diseases and disorders responsive to the induction of apoptosis. In a preferred aspect, they are useful for the treatment, prevention or amelioration of hyperproliferative diseases and disorders responsive to the induction of apoptosis.
In a preferred aspect, the compounds, or a salt, stereoisomer or a salt of a stereoisomer thereof, according to the invention are useful in the treatment, prevention or amelioration of benign neoplasia or malignant neoplasia, particularly cancer, more particularly a cancer that is susceptible to Eg5 inhibition, more particularly any of the cancer diseases described above. Preferred is the treatment of said diseases.

Thus, the present invention further relates to compounds, or a salt, stereoisomer or a salt of a stereoisomer thereof, according to the invention for use in therapy, such as, for example, in the treatment, prevention or amelioration of hyperproliferative diseases and disorders responsive to the induction of apoptosis. In a preferred aspect, the invention relates to compounds, or a salt, stereoisomer or a salt of a stereoisomer thereof, according to the invention for use in the treatment, prevention or amelioration of benign neoplasia or malignant neoplasia, particularly cancer.

In the context of their properties, functions and usabilities mentioned herein, the compounds according to the present invention are expected to be distinguished by valuable and desirable effects related therewith, such as e.g. by low toxicity, superior bioavailability in general (such as e.g. good enteral absorption), superior therapeutic window, absence of significant side effects, and/or further beneficial effects related with their therapeutic and pharmaceutical suitability.

In a preferred aspect, the invention relates to a method for treating, preventing or ameliorating hyperproliferative diseases and disorders responsive to the induction of apoptosis in mammals, comprising administering to said mammals in need thereof a pharmaceutically active and therapeutically effective and tolerable amount of one or more of the compounds or a salt, stereoisomer or a salt of a stereoisomer thereof, according to the invention. Preferred is the method of treatment of said diseases and disorders.

In a particularly preferred aspect, the invention relates to a method of treatment of the diseases, disorders, conditions or illnesses mentioned above, particularly benign neoplasia or malignant neoplasia, comprising administering to said mammals in need thereof a pharmaceutically active and therapeutically effective and tolerable amount of one or more of the compounds, or a salt, stereoisomer or a salt of a stereoisomer thereof, according to the invention. In a particular aspect, the disease is cancer, more particularly a cancer that is susceptible to Eg5 inhibition, more particularly any of the cancer diseases described above.

The invention further includes a method of modulating, particularly inhibiting, Eg5 activity in cells comprising administering a pharmaceutically active and therapeutically effective and tolerable amount of one or more of the compounds, or a salt, stereoisomer or a salt of a stereoisomer thereof, according to the invention to a patient in need of such modulation, particularly inhibition.

The present invention further includes a method of modulating apoptosis or aberrant cell growth in the therapy of benign or malignant neoplastic diseases, e.g. cancer, comprising administering to a subject in need of such therapy a pharmaceutically active and therapeutically effective and tolerable amount of one or more of the compounds, or a salt, stereoisomer or a salt of a stereoisomer thereof, according to the invention.

The present invention further includes a method to modulate the mitotic spindle, i.e., for example, altering mitotic spindle formation, including decreasing spindle formation, or increasing or decreasing spindle pole separation causing malformation of the mitotic spindle poles, comprising administering a pharmaceutically active and therapeutically effective and tolerable amount of one or more of the compounds, or a salt, stereoisomer or a salt of a stereoisomer thereof, according to the invention to a patient in need of such modulation.

The present invention further includes a method to inhibit mitosis in cells comprising administering a pharmaceutically active and therapeutically effective and tolerable amount of one or more of the compounds, or a salt, stereoisomer or a salt of a stereoisomer thereof, according to the invention to a patient in need of such inhibition.

The present invention further includes a method for treating, preventing or ameliorating diseases and/or disorders associated with Eg5 kinesin activity, such as, for example, hyperproliferative diseases and/or disorders responsive to induction of apoptosis, for example, benign or malignant neoplasia, e.g. cancer, in a mammal comprising administering a pharmaceutically active and therapeutically effective and tolerable amount of one or more compounds according to the present invention to said mammal in need thereof.

The present invention further relates to the use of the compounds, or a salt, stereoisomer or a salt of a stereoisomer thereof, according to the invention for the production of pharmaceutical compositions which are employed for the treatment, prophylaxis and/or amelioration of one or more of the illnesses mentioned.

The present invention particularly relates to the use of the compounds, or a salt, stereoisomer or a salt of a stereoisomer thereof, according to the invention in the production of pharmaceutical compositions for the treatment, prevention or amelioration of hyperproliferative diseases and disorders responsive to the induction of apoptosis.
The invention particularly relates to the use of the compounds, or a salt, stereoisomer or a salt of a stereoisomer thereof, according to the invention in the production of pharmaceutical compositions for the treatment, prevention or amelioration of benign neoplasia or malignant neoplasia, particularly cancer, more particularly a cancer that is susceptible to Eg5 inhibition, more particularly any of the cancer diseases described above. Preferred is the use of the compounds, or a salt, stereoisomer or a salt of a stereoisomer thereof, according to the invention for the production of pharmaceutical compositions which are used in the treatment of mammals.

The invention further relates to a compound according to the invention or a pharmaceutically acceptable salt thereof, for the treatment, prevention or amelioration of hyperproliferative diseases and disorders responsive to the induction of apoptosis. Particularly, the invention relates to a compound according to the invention or a pharmaceutically acceptable salt thereof, for the treatment, prevention or amelioration of benign neoplasia or malignant neoplasia, particularly cancer.
The invention further relates to a pharmaceutical composition, comprising a compound according to the invention or a pharmaceutically acceptable salt thereof, for the treatment, prevention or amelioration of hyperproliferative diseases and disorders responsive to the induction of apoptosis. Particularly, the invention relates to a pharmaceutical composition, comprising a compound according to the invention or a pharmaceutically acceptable salt thereof, for the treatment, prevention or amelioration of benign neoplasia or malignant neoplasia, particularly cancer.

The present invention further relates to pharmaceutical compositions comprising one or more of the compounds, or a salt, stereoisomer or a salt of a stereoisomer thereof, according to the invention and a pharmaceutically acceptable carrier or diluent.

The present invention particularly relates to pharmaceutical compositions comprising one or more compounds, or a salt, stereoisomer or a salt of a stereoisomer thereof, according to the invention together with pharmaceutically acceptable auxiliaries and/or excipients.

The present invention further relates to a combination comprising one or more of the compounds, or a salt, stereoisomer or a salt of a stereoisomer thereof, according to the invention and pharmaceutically acceptable auxiliaries, excipients and/or vehicles, e.g. for treating, preventing or ameliorating benign neoplasia and malignant neoplasia, particularly cancer, such as e.g. any of those cancer diseases described above.

The present invention further relates to a composition comprising a therapeutically effective and tolerable amount of one or more compounds, or a salt, stereoisomer or a salt of a stereoisomer thereof, according to the invention together with the usual pharmaceutically acceptable vehicles, diluents and/or excipients for use in therapy, e.g. for treating, preventing or ameliorating hyperproliferative diseases, such as e.g. cancer, and/or disorders responsive to induction of apoptosis.

The present invention further relates to compounds, or a salt, stereoisomer or a salt of a stereoisomer thereof, according to the invention having Eg5 inhibiting properties. The present invention further relates to compounds, or a salt, stereoisomer or a salt of a stereoisomer thereof, according to the invention having anti-proliferative and/or apoptosis inducing activity.

The present invention further relates to pharmaceutical compositions according to the invention having Eg5 inhibiting properties. The present invention further relates to pharmaceutical compositions according to the invention having anti-proliferative activity. The present invention further relates to pharmaceutical compositions according to the invention having apoptosis inducing activity.

The invention further relates to the use of a pharmaceutical composition comprising one or more of the compounds, or a salt, stereoisomer or a salt of a stereoisomer thereof, according to the invention as sole active ingredient(s) and a pharmaceutically acceptable carrier or diluent in the manufacture of pharmaceutical products for the treatment and/or prophylaxis of the illnesses mentioned above.

Additionally, the invention relates to an article of manufacture, which comprises packaging material and a pharmaceutical agent contained within said packaging material, wherein the pharmaceutical agent is therapeutically effective inhibiting Eg5 and/or inhibiting cellular hyperproliferation and/or inducing apoptosis, ameliorating the symptoms of a Eg5 mediated disease and/or a hyperproliferative disease and/or a disorder responsive to the induction of apoptosis, and wherein the packaging material comprises a label or package insert which indicates that the pharmaceutical agent is useful for preventing or treating a Eg5 mediated disease and/or a hyperproliferative disease and/or a disorder responsive to the induction of apoptosis, and wherein said pharmaceutical agent comprises one or more compounds, or a salt, stereoisomer or a salt of a stereoisomer thereof, according to the invention. The packaging material, label and package insert otherwise parallel or resemble what is generally regarded as standard packaging material, labels and package inserts for pharmaceuticals having related utilities.

The pharmaceutical compositions according to the invention are prepared by processes which are known per se and familiar to the person skilled in the art. As pharmaceutical compositions, the compounds, or a salt, stereoisomer or a salt of a stereoisomer thereof, according to the invention are either employed as such, or preferably in combination with suitable pharmaceutical auxiliaries and/or excipients, e.g. in the form of tablets, coated tablets, dragees, pills, cachets, granules, capsules, caplets, suppositories, patches (e.g. as TTS), emulsions (such as e.g. micro-emulsions or lipid emulsions), suspensions (such as e.g. nano suspensions), gels, solubilisates or solutions (e.g. sterile solutions), or encapsuled in liposomes or as beta-cyclodextrine or beta-cyclodextrin derivative inclusion complexes or the like, the active compound content advantageously being between 0.1 and 95% and where, by the appropriate choice of the auxiliaries and/or excipients, a pharmaceutical administration form (e.g. a delayed release form or an enteric form) exactly suited to the active compound and/or to the desired onset of action can be achieved.

The person skilled in the art is familiar with auxiliaries, vehicles, excipients, diluents, carriers or adjuvants which are suitable for the desired pharmaceutical formulations, preparations or compositions on account of his/her expert knowledge. In addition to solvents, gel formers, ointment bases and other active compound excipients, for example antioxidants, dispersants, emulsifiers, preservatives, solubilizers (such as e.g. polyoxyethylenglyceroltriricinoleat 35, PEG 400, Tween 80, Captisol, Solutol HS15 or the like), colorants, complexing agents, permeation promoters, stabilizers, fillers, binders, thickeners, disintegrating agents, buffers, pH regulators (e.g. to obtain neutral, alkaline or acidic formulations), polymers, lubricants, coating agents, propellants, tonicity adjusting agents, surfactants, flavorings, sweeteners or dyes, can be used.

In particular, auxiliaries and/or excipients of a type appropriate to the desired formulation and the desired mode of administration are used.

The administration of the compounds, pharmaceutical compositions or combinations according to the invention may be performed in any of the generally accepted modes of administration available in the art. Illustrative examples of suitable modes of administration include intravenous, oral, nasal, parenteral, topical, transdermal and rectal delivery. Oral and intravenous delivery are preferred.

For the treatment of dermatoses, the compounds of the invention can be in particular administered in the form of those pharmaceutical compositions which are suitable for topical application. For the production of the pharmaceutical compositions, the compounds of the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, lotions, pastes, gels or solutions.

The pharmaceutical compositions according to the invention can be prepared by processes known per se. The dosage of the compounds of the invention (= active compounds) is carried out in the order of magnitude customary for Eg5 inhibitors, inhibitors for cellular hyperproliferation or apoptosis inducers. Topical application forms (such as ointments) for the treatment of dermatoses thus contain the active compounds in a concentration of, for example, 0.1-99%. The customary dose in the case of systemic therapy (p.o.) may be between 0.03 and 60 mg/kg per day, (i. v.) may be between 0.03 and 60 mg/kg/h. In another embodiment, the customary dose in the case of systemic therapy (p.o.) is between 0.3 and 30 mg/kg per day, (i. v.) is between 0.3 and 30 mg/kg/h.

The choice of the optimal dosage regime and duration of medication, particularly the optimal dose and manner of administration of the active compounds necessary in each case can be determined by a person skilled in the art on the basis of his/her expert knowledge.

Depending upon the particular disease to be treated or prevented, additional therapeutic active agents, which are normally administered to treat or prevent that disease, may optionally be coadministered with the compounds, or a salt, stereoisomer or a salt of a stereoisomer thereof, according to the invention. As used herein, additional therapeutic agents that are normally administered to treat or prevent a particular disease are known as appropriate for the disease being treated.

For example, compounds, or a salt, stereoisomer or a salt of a stereoisomer thereof, according to the invention may be combined with one or more standard therapeutic agents used for treatment of the diseases as mentioned before.

In this context, the present invention further relates to a combination comprising
a first active ingredient, which is at least one compound or a salt, stereoisomer or a salt of a stereoisomer thereof, according to the invention, and
a second active ingredient, which is at least one anti-cancer agent.
Preferred is a combination for separate, sequential, simultaneous, concurrent or chronologically staggered use in the treatment, prevention or amelioration of hyperproliferative diseases and disorders responsive to the induction of apoptosis.

Preferred is a combination, in which the second active ingredient is one or more of those art-known anti-cancer agents that is mentioned herein below. Preferred is a combination for use in therapy of any of those diseases mentioned herein, particularly in the treatment, prevention or amelioration of hyperproliferative diseases and disorders responsive to the induction of apoptosis, more particularly in the treatment of cancer.

The term "combination" according to the invention may be present as a fixed combination, a non-fixed combination or a kit-of-parts.

A "fixed combination" is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A "kit-of-parts" is defined as a combination wherein the said first active ingredient and the said second active ingredient are present in more than one unit. One example of a "kit-of-parts" is a combination wherein the said first active ingredient and the said second active ingredient are present separately. The components of the kit-of-parts may be administered separately, sequentially, simultaneously, concurrently or chronologically staggered.

The present invention further relates to a pharmaceutical composition comprising
a first active ingredient, which is at least one compound according to the invention, and
a second active ingredient, which is at least one art-known anti-cancer agent, such as e.g. one or more of those mentioned herein above, and, optionally,
a pharmaceutically acceptable carrier or diluent,
for separate, sequential, simultaneous, concurrent or chronologically staggered use in therapy.

The present invention further relates to a combination product comprising
a.) at least one compound according to the invention formulated with a pharmaceutically acceptable carrier or diluent, and
b.) at least one art-known anti-cancer agent, such as e.g. one or more of those mentioned herein above, formulated with a pharmaceutically acceptable carrier or diluent.

The present invention further relates to a kit-of-parts comprising a preparation of a first active ingredient, which is a compound according to the invention, and a pharmaceutically acceptable carrier or diluent; a preparation of a second active ingredient, which is an art-known anti-cancer agent, such as one of those mentioned above, and a pharmaceutically acceptable carrier or diluent; for simultaneous, concurrent, sequential, separate or chronologically staggered use in therapy. Optionally, said kit comprises instructions for its use in therapy, e.g. to treat hyperproliferative diseases and/or disorders responsive to the induction of apoptosis, such as e.g. cancer, more precisely, any of those cancer diseases described above.

The present invention further relates to a combined preparation comprising at least one compound according to the invention and at least one art-known anti-cancer agent for simultaneous, concurrent, sequential or separate administration.

The present invention further relates to combinations, compositions, formulations, preparations or kits according to the present invention having Eg5 inhibitory activity and/or anti-proliferative and/or apoptosis inducing properties.

In one particular embodiment, compounds, or a salt, stereoisomer or a salt of a stereoisomer thereof, according to the invention may be combined with one or more art-known anti-cancer agents, such as e.g. with one or more chemotherapeutic and/or target specific anti-cancer agents as described below.

Examples of known chemotherapeutic anti-cancer agents frequently used in combination therapy include, but not are limited to (i) alkylating/carbamylating agents such as Cyclophosphamid (Endoxan®), Ifosfamid (Holoxan®), Thiotepa (Thiotepa Lederle®), Melphalan (Alkeran®), or chloroethylnitrosourea (BCNU); (ii) platinum derivatives like cis-platin (Platinex® BMS), oxaliplatin, satraplatin or carboplatin (Cabroplat® BMS); (iii) antimitotic agents / tubulin inhibitors such as vinca alkaloids (vincristine, vinblastine, vinorelbine), taxanes such as Paclitaxel (Taxol®), Docetaxel (Taxotere®) and analogs as well as new formulations and conjugates thereof (like the nanoparticle formulation Abraxane® with paclitaxel bound to albumin), epothilones such as Epothilone B (Patupilone®), Azaepothilone (Ixabepilone®) or ZK-EPO, a fully synthetic epothilone B analog; (iv) topoisomerase inhibitors such as anthracyclines (exemplified by Doxorubicin / Adriblastin®), epipodophyllotoxines (examplified by Etoposide / Etopophos®) and camptothecin and camptothecin analogs (exemplified by Irinotecan / Camptosar® or Topotecan / Hycamtin®); (v) pyrimidine antagonists such as 5-fluorouracil (5-FU), Capecitabine (Xeloda®), Arabinosylcytosine / Cytarabin (Alexan®) or Gemcitabine (Gemzar®); (vi) purin antagonists such as 6-mercaptopurine (Puri-Nethol®), 6-thioguanine or fludarabine (Fludara®) and finally (vii) folic acid antagonists such as methotrexate (Farmitrexat®) or premetrexed (Alimta®).

Examples of anti-cancer agents, preferably target specific anti-cancer-agents, used in experimental or standard cancer therapy include but are not limited to (i) kinase inhibitors such as e.g. Imatinib (Glivec®), ZD-1839 / Gefitinib (Iressa®), Bay43-9006 (Sorafenib, Nexavar®), SU11248 / Sunitinib (Sutent®), OSI-774 / Erlotinib (Tarceva®), Dasatinib (Sprycel®), Lapatinib (Tykerb®), or, see also below, Vatalanib, Vandetanib (Zactima®) or Pazopanib; (ii) proteasome inhibitors such as PS-341 / Bortezumib (Velcade®); (iii) histone deacetylase inhibitors like SAHA (Zolinza®), PXD101, MS275, MGCD0103, Depsipeptide / FK228, NVP-LBH589, NVP-LAQ824, Valproic acid (VPA), CRA / PCI 24781, ITF2357, SB939 and butyrates (iv) heat shock protein 90 inhibitors like 17-allylaminogeldanamycin (17-AAG) or 17-dimethylaminogeldanamycin (17-DMAG); (v) vascular targeting agents (VTAs) like combretastin A4 phosphate or AVE8062 / AC7700 and anti-angiogenic drugs like the VEGF antibodies, such as Bevacizumab (Avastin®), or KDR tyrosine kinase inhibitors such as PTK787 / ZK222584 (Vatalanib) or Vandetanib (Zactima®) or Pazopanib; (vi) monoclonal antibodies such as Trastuzumab (Herceptin®) or Rituximab (MabThera / Rituxan®) or Alemtuzumab (Campath®) or Tositumomab (Bexxar®) or C225/ Cetuximab (Erbitux®) or Avastin (see above) or Bevacizumab or Panitumumab (Vectibix®) as well as mutants and conjugates of monoclonal antibodies, e.g. Gemtuzumab ozogamicin (Mylotarg®) or Ibritumomab tiuxetan (Zevalin®), and antibody fragments; (vii) oligonucleotide based therapeutics like G-3139 / Oblimersen (Genasense®) or the DNMT1 inhibitor MG98; (viii) Toll-like receptor / TLR 9 agonists like Promune®, TLR 7 agonists like Imiquimod (Aldara®) or Isatoribine and analogues thereof, or TLR 7/8 agonists like Resiquimod as well as immunostimulatory RNA as TLR 7/8 agonists; (ix) protease inhibitors (x) hormonal therapeutics such as anti-estrogens (e.g. Tamoxifen or Raloxifen), anti-androgens (e.g. Flutamide or Casodex), LHRH analogs (e.g. Leuprolide, Goserelin or Triptorelin) and aromatase inhibitors.

Other known anti-cancer agents which may be used for combination therapy include bleomycin, retinoids such as all-trans retinoic acid (ATRA), DNA methyltransferase inhibitors such as 5-Aza-2'-deoxycytidine (Decitabine, Dacogen®) and 5-azacytidine, alanosine, cytokines such as interleukin-2, interferons such as interferon α2 or interferon-γ, death receptor agonists, such as TRAIL, DR4/5 agonistic antibodies, FasL and TNF-R agonists (e.g. TRAIL receptor agonists like mapatumumab or lexatumumab).

As exemplary anti-cancer agents, which may be useful in the combination therapy according to the present invention, any of the following drugs may be mentioned, without being restricted thereto, 5 FU, actinomycin D, ABARELIX, ABCIXIMAB, ACLARUBICIN, ADAPALENE, ALEMTUZUMAB, ALTRETAMINE, AMINOGLUTETHIMIDE, AMIPRILOSE, AMRUBICIN, ANASTROZOLE, ANCITABINE, ARTEMISININ, AZATHIOPRINE, BASILIXIMAB, BENDAMUSTINE, BEVACIZUMAB, BEXXAR, BICALUTAMIDE, BLEOMYCIN, BORTEZOMIB, BROXURIDINE, BUSULFAN, CAMPATH, CAPECITABINE, CARBOPLATIN, CARBOQUONE, CARMUSTINE, CETRORELIX, CHLORAMBUCIL, CHLORMETHINE, CISPLATIN, CLADRIBINE, CLOMIFENE, CYCLOPHOSPHAMIDE, DACARBAZINE, DACLIZUMAB, DACTINOMYCIN, DASATINIB, DAUNORUBICIN, DECITABINE, DESLORELIN, DEXRAZOXANE, DOCETAXEL, DOXIFLURIDINE, DOXORUBICIN, DROLOXIFENE, DROSTANOLONE, EDELFOSINE, EFLORNITHINE, EMITEFUR, EPIRUBICIN, EPITIOSTANOL, EPTAPLATIN, ERBITUX, ERLOTINIB, ESTRAMUSTINE, ETOPOSIDE, EXEMESTANE, FADROZOLE, FINASTERIDE, FLOXURIDINE, FLUCYTOSINE, FLUDARABINE, FLUOROURACIL, FLUTAMIDE, FORMESTANE, FOSCARNET, FOSFESTROL, FOTEMUSTINE, FULVESTRANT, GEFITINIB, GENASENSE, GEMCITABINE, GLIVEC, GOSERELIN, GUSPERIMUS, HERCEPTIN, IDARUBICIN, IDOXURIDINE, IFOSFAMIDE, IMATINIB, IMPROSULFAN, INFLIXIMAB, IRINOTECAN, IXABEPILONE, LANREOTIDE, LAPATINIB, LETROZOLE, LEUPRORELIN, LOBAPLATIN, LOMUSTINE, LUPROLIDE, MELPHALAN, MERCAPTOPURINE, METHOTREXATE, METUREDEPA, MIBOPLATIN, MIFEPRISTONE, MILTEFOSINE, MIRIMOSTIM, MITOGUAZONE, MITOLACTOL, MITOMYCIN, MITOXANTRONE, MIZORIBINE, MOTEXAFIN, MYLOTARG, NARTOGRASTIM, NEBAZUMAB, NEDAPLATIN, NILUTAMIDE, NIMUSTINE, OCTREOTIDE, ORMELOXIFENE, OXALIPLATIN, PACLITAXEL, PALIVIZUMAB, PANITUMUMAB, PATUPILONE, PAZOPANIB, PEGASPARGASE, PEGFILGRASTIM, PEMETREXED, PENTETREOTIDE, PENTOSTATIN, PERFOSFAMIDE, PIPOSULFAN, PIRARUBICIN, PLICAMYCIN, PREDNIMUSTINE, PROCARBAZINE, PROPAGERMANIUM, PROSPIDIUM CHLORIDE, RALOXIFEN, RALTITREXED, RANIMUSTINE, RANPIRNASE, RASBURICASE, RAZOXANE, RITUXIMAB, RIFAMPICIN, RITROSULFAN, ROMURTIDE, RUBOXISTAURIN, SARGRAMOSTIM, SATRAPLATIN, SIROLIMUS, SOBUZOXANE, SORAFENIB, SPIROMUSTINE, STREPTOZOCIN, SUNITINIB, TAMOXIFEN, TASONERMIN, TEGAFUR, TEMOPORFIN, TEMOZOLOMIDE, TENIPOSIDE, TESTOLACTONE, THIOTEPA, THYMALFASIN, TIAMIPRINE, TOPOTECAN, TOREMIFENE, TRAIL, TRASTUZUMAB, TREOSULFAN, TRIAZIQUONE, TRIMETREXATE, TRIPTORELIN, TROFOSFAMIDE, UREDEPA, VALRUBICIN, VATALANIB, VANDETANIB, VERTEPORFIN, VINBLASTINE, VINCRISTINE, VINDESINE, VINORELBINE, VOROZOLE and ZEVALIN.

The anti-cancer agents mentioned herein above as combination partners of the compounds, or a salt, stereoisomer or a salt of a stereoisomer thereof, according to the invention are meant to include pharmaceutically acceptable derivatives thereof, such as e.g. their pharmaceutically acceptable salts.

The person skilled in the art is aware on the base of his/her expert knowledge of the kind, total daily dosage(s) and administration form(s) of the additional therapeutic agent(s) coadministered. Said total daily dosage(s) can vary within a wide range.

In practicing the present invention, the compounds, or a salt, stereoisomer or a salt of a stereoisomer thereof, according to the invention may be administered in combination therapy separately, sequentially, simultaneously, concurrently or chronologically staggered (such as e.g. as combined unit dosage forms, as separate unit dosage forms, as adjacent discrete unit dosage forms, as fixed or non-fixed combinations, as kit-of-parts or as admixtures) with one or more standard therapeutics (chemotherapeutic and/or target specific anti-cancer agents), in particular art-known anti-cancer agents, such as any of e.g. those mentioned above.

The combinations, e.g. compositions, preparations, formulations, kits or packages mentioned in the context of the combination therapy according to the invention may also include more than one of the compounds, or a salt, stereoisomer or a salt of a stereoisomer thereof, according to the invention and/or more than one of the art-known anti-cancer agents mentioned.

The first and second active ingredient of a combination or kit-of-parts according to the invention may be provided as separate formulations (i.e. independently of one another), which are subsequently brought together for simultaneous, concurrent, sequential, separate or chronologically staggered use in combination therapy; or packaged and presented together as separate components of a combination pack for simultaneous, concurrent, sequential, separate or chronologically staggered use in combination therapy.

The type of pharmaceutical formulation of the first and second active ingredient of a combination or kit-of-parts according to the invention can be the same, i.e. both ingredients are formulated in separate tablets or capsules, or can be different, i.e. suited for different administration forms, such as e.g. one active ingredient is formulated as tablet or capsule and the other is formulated for e.g. intravenous administration.

The amounts of the first and second active ingredients of the combinations, compositions or kits according to the invention may together comprise a therapeutically effective amount for the treatment, prophylaxis or amelioration of a (hyper)proliferative diseases and/or a disorder responsive to the induction of apoptosis, particularly one of those diseases mentioned herein, such as e.g. malignant or benign neoplasia, especially cancer, like any of those cancer diseases mentioned herein.

In addition, compounds according to the present invention can be used in the pre- or post-surgical treatment of cancer. In further addition, compounds of the present invention can be used in combination with radiation therapy.

A combination according to the invention can refer to a composition comprising both the compound(s) according to the invention and the other active anti-cancer agent(s) in a fixed combination (fixed unit dosage form), or a medicament pack comprising the two or more active ingredients as discrete separate dosage forms (non-fixed combination). In case of a medicament pack comprising the two or more active ingredients, the active ingredients are preferably packed into blister cards which are suited for improving compliance.

Each blister card preferably contains the medicaments to be taken on one day of treatment. If the medicaments are to be taken at different times of day, the medicaments can be disposed in different sections on the blister card according to the different ranges of times of day at which the medicaments are to be taken (for example morning and evening or morning, midday and evening). The blister cavities for the medicaments to be taken together at a particular time of day are accommodated in the respective range of times of day. The various times of day are, of course, also put on the blister in a clearly visible way. It is also possible, of course, for example to indicate a period in which the medicaments are to be taken, for example stating the times.

The daily sections may represent one line of the blister card, and the times of day are then identified in chronological sequence in this column.

Medicaments which must be taken together at a particular time of day are placed together at the appropriate time on the blister card, preferably a narrow distance apart, allowing them to be pushed out of the blister easily, and having the effect that removal of the dosage form from the blister is not forgotten.

In addition, the present invention further relates to:
- the use of said combinations according to the invention for the production of pharmaceutical compositions which are used in the treatment, prevention or amelioration of hyperproliferative diseases and disorders responsive to the induction of apoptosis.
- a combination according to the invention for the treatment, prevention or amelioration of hyperproliferative diseases and disorders responsive to the induction of apoptosis,
- a method for treating, preventing or ameliorating hyperproliferative diseases and disorders responsive to the induction of apoptosis in mammals, comprising administering to said mammals in need thereof a pharmaceutically active and therapeutically effective and tolerable amount of said combination.
- a commercial package comprising a said combination.
In a preferred aspect, hyperproliferative diseases and disorders responsive to the induction of apoptosis include benign neoplasia, malignant neoplasia and cancer.

### Biological Investigations

The anti-proliferative / cytotoxic activity of the compounds described herein can be tested on subclones of RKO human colon adenocarcinoma cells (Schmidt et al., Oncogene 19, 2423-2429; 2000) using the Alamar Blue cell viability assay (described in O'Brien et al. Eur J Biochem 267, 5421-5426, 2000). The compounds are dissolved as 10 mM solutions in DMSO and subsequently diluted in semi-logarithmic steps. DMSO dilutions are further diluted 1:100 v:v into Dulbecco's modified Eagle's medium (DMEM) containing 10% fetal calf serum to a final concentration twice as much as the final concentration in the test. RKO subclones are seeded into 96 well flat bottom plates at a density of 4000 cells per well in a volume of 50 µl per well. 24 hours after seeding the 50 µl each of the compound dilutions in DMEM medium are added into each well of the 96 well plate. Each compound dilution is tested as triplicates. Wells containing untreated control cells are filled with 50 µl DMEM medium containing 1% DMSO. The cells are then incubated with the substances for 72 hours at 37°C in a humidified atmosphere containing 5% carbon dioxide. To determine the viability of the cells, 10 µl of an Alamar Blue solution (Biosource) are added and the fluorescence is measured at an extinction of 544 nm and an emission of 590 nm. For the calculation of the cell viability the emission value from untreated cells is set as 100% viability and the emission rates of treated cells are set in relation to the values of untreated cells. Viabilities are expressed as % values. The Graphpad Prism program is used for the calculation of EC₅₀ values for anti-proliferative / cytotoxic activity out of the obtained dose-response curves.

To determine the cell cycle specific mode of action, subclones of RKO colon adenocarcinoma cells (RKOp21 or RKOp27 as described by Schmidt et al. in Oncogene 19, 2423-2429; 2000) are seeded into 96 well flat bottom plates at a density of 16000 cells per well in a volume of 50 µl per well in DMEM growth medium with 10% FCS containing 10 µM Ponasterone A. 24 hours after seeding the 50 µl each of the compound dilutions in DMEM medium are added into each well of the 96-well plate. Each compound dilution is tested as triplicates. Wells containing untreated control cells are filled with 50 µl DMEM medium containing 1% DMSO. The cells are then incubated with the substances for 72 hours at 37°C in a humidified atmosphere containing 5% carbon dioxide. To determine the viability of the cells, 10 µl of an Alamar Blue solution (Biosource) are added and the fluorescence is measured at an extinction of 544 nm and an emission of 590 nm. For the calculation of the cell viability the emission value from untreated cells is set as 100% viability and the emission rates of treated cells are set in relation to the values of untreated cells. Viabilities are expressed as % values. The Graphpad Prism program (GraphPad Software, Inc) is used for the calculation of EC₅₀ values out of the obtained dose-response curves. Viability is compared of proliferating cells grown in the absence of the inducer Ponasterone A, versus viability of cells arrested by the expression of ectopic p27Kip1 induced by Ponasterone A.

Representative values for anti-proliferation / cytotoxicity [measured as -log EC₅₀ (mol/l)] determined in the aforementioned assays follow from the following table A, in which the numbers of the compounds correspond to the numbers of the examples.

**Table A**

| **Anti-proliferative / cytotoxic activity on RKO colon cancer cells** | |
|---|---|
| | Examples |
| -log EC₅₀ [mol/l] RKO p27 uninduced (proliferating) ≥ 7.0 | 8, 9, 11, 12, 16, 19, 25 |
| -log EC₅₀ [mol/l] RKO p27 uninduced (proliferating) < 7.0 but ≥ 6.0 | 6, 7, 10, 13, 14, 15, 27, 28 |

The value of -log EC₅₀ [mol/l] RKO p27 induced (arrested) was below the minimum determined by the assay specification (≤ 4.0, ≤ 5.0, ≤ 5.5 or ≤ 6.0).

## Claims

1. Compound of formula I 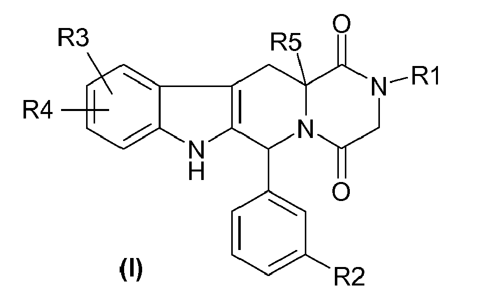 in which
R1 is 1-4C-alkyl, 3-7C-cycloalkyl, 2-4C-alkenyl, 2-4C-alkinyl, 3-7C-cycloalkyl-1-4C-alkyl, or 2-7C-alkyl substituted by R11, in which
R11 is -N(R111)R112, hydroxyl or halogen, in which
R111 is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
R112 is hydrogen, 1-4C-alkyl, 3-7C-cycloalkyl, or 3-7C-cycloalkyl-1-4C-alkyl,
or R111 and R112 together and with inclusion of the nitrogen atom, to which they are bonded, form a ring Het, in which
Het is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
R113 is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
R2 is hydrogen or hydroxyl,
R3 is hydrogen, 1-4C-alkyl, halogen, 1-4C-alkoxy, trifluoromethyl, cyano, hydroxyl, 1-4C-alkoxy-2-4C-alkoxy, hydroxy-2-4C-alkoxy, 3-7C-cycloalkoxy, 3-7C-cycloalkyl-1-4C-alkoxy, or completely or predominantly fluorine-substituted 1-4C-alkoxy,
R4 is hydrogen, 1-4C-alkyl or halogen,
R5 is hydrogen or 1-4C-alkyl,
or a salt, stereoisomer or a salt of a stereoisomer thereof.

2. Compound according to claim 1, in which
R1 is 2-7C-alkyl substituted by R11, in which
R11 is -N(R111)R112, in which
R111 is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
R112 is hydrogen, 1-4C-alkyl, 3-7C-cycloalkyl, or 3-7C-cycloalkyl-1-4C-alkyl,
or R111 and R112 together and with inclusion of the nitrogen atom, to which they are bonded, form a ring Het, in which
Het is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
R113 is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
R2 is hydrogen or hydroxyl,
R3 is 1-4C-alkoxy,
R4 is hydrogen, 1-4C-alkyl or halogen,
R5 is hydrogen or 1-4C-alkyl,
or a salt, stereoisomer or a salt of a stereoisomer thereof.

3. Compound according to claim 1, in which
R1 is 1-4C-alkyl, 3-7C-cycloalkyl, 2-4C-alkenyl, 2-4C-alkinyl, 3-7C-cycloalkyl-1-4C-alkyl, or 2-7C-alkyl substituted by R11, in which
R11 is -N(R111)R112, hydroxyl or halogen, in which
R111 is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
R112 is hydrogen, 1-4C-alkyl, 3-7C-cycloalkyl, or 3-7C-cycloalkyl-1-4C-alkyl,
or R111 and R112 together and with inclusion of the nitrogen atom, to which they are bonded, form a ring Het, in which
Het is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1- yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
R113 is hydrogen, 1-4C-alkyl, 3-7C-cycloalkyl, 3-7C-cycloalkyl-1-4C-alkyl, 1-4C-alkylcarbonyl, amidino, or completely or partially fluorine-substituted 1-4C-alkyl, and
R2 is hydrogen or hydroxyl,
R3 is 1-4C-alkoxy,
R4 is hydrogen, 1-4C-alkyl or halogen,
R5 is 1-4C-alkyl,
or a salt, stereoisomer or a salt of a stereoisomer thereof.

4. Compound according to claim 1, in which
R1 is 2-7C-alkyl substituted by R11, in which
R11 is -N(R111)R112 or halogen, in which
R111 is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
R112 is hydrogen, 1-4C-alkyl,
or R111 and R112 together and with inclusion of the nitrogen atom, to which they are bonded, form a ring Het, in which
Het is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
R113 is hydrogen or 1-4C-alkyl, and
R2 is hydrogen or hydroxyl,
R3 is hydrogen, 1-4C-alkyl, halogen, 1-4C-alkoxy, or completely or predominantly fluorine-substituted 1-4C-alkoxy,
R4 is hydrogen or halogen,
R5 is 1-4C-alkyl,
or a salt, stereoisomer or a salt of a stereoisomer thereof.

5. Compound according to claim 1 or 4, in which
R1 is 2-7C-alkyl substituted by R11, in which
R11 is -N(R111)R112, or halogen, in which
R111 is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
R112 is hydrogen or 1-4C-alkyl
or R111 and R112 together and with inclusion of the nitrogen atom, to which they are bonded, form a ring Het, in which
Het is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
R113 is hydrogen, 1-4C-alkyl or 1-4C-alkylcarbonyl, and
R2 is hydroxyl,
R3 is hydrogen, 1-4C-alkyl, halogen, 1-4C-alkoxy, or completely or predominantly fluorine-substituted 1-4C-alkoxy,
R4 is hydrogen or halogen,
R5 is 1-4C-alkyl,
or a salt, stereoisomer or a salt of a stereoisomer thereof.

6. Compound according to claim 1, in which
R1 is 2-7C-alkyl substituted by R11, in which
R11 is -N(R111)R112 or halogen, in which
R111 is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
R112 is hydrogen, 1-4C-alkyl,
or R111 and R112 together and with inclusion of the nitrogen atom, to which they are bonded, form a ring Het, in which
Het is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
R113 is hydrogen, 1-4C-alkyl, or 1-4C-alkylcarbonyl, and
R2 is hydrogen or hydroxyl,
R3 is hydrogen, 1-4C-alkyl, halogen, 1-4C-alkoxy, or completely or predominantly fluorine-substituted 1-4C-alkoxy,
R4 is hydrogen or halogen,
R5 is hydrogen,
or a salt, stereoisomer or a salt of a stereoisomer thereof.

7. Compound according to claim 1 or 6, in which
R1 is 2-7C-alkyl substituted by R11, in which
R11 is -N(R111)R112, or halogen, in which
R111 is hydrogen, 1-4C-alkyl, 2-4C-alkenyl, 2-4C-alkinyl, 3-7C-cycloalkyl, 3-7C-cycloalkyl-1-4C-alkyl, hydroxy-2-4C-alkyl, 1-4C-alkoxy-2-4C-alkyl, 1N-(1-4C-alkyl)-pyrazolyl, 1 N-(H)-pyrazolyl, 2N-(1-4C-alkyl)-pyrazolyl, isoxazolyl, or completely or partially fluorine-substituted 1-4C-alkyl,
R112 is hydrogen or 1-4C-alkyl
or R111 and R112 together and with inclusion of the nitrogen atom, to which they are bonded, form a ring Het, in which
Het is optionally substituted by one or two substituents independently selected from 1-4C-alkyl and fluorine, and is piperidin-1-yl, morpholin-4-yl, thiomorpholin-4-yl, S-oxo-thiomorpholin-4-yl, S,S-dioxo-thiomorpholin-4-yl, pyrrolidin-1-yl, azetidin-1-yl, azepan-1-yl, homopiperidin-1-yl, 4N-(R113)-piperazin-1-yl, 4N-(R113)-homopiperazin-1-yl, 2,5-dihydro-pyrrol-1-yl, 3,6-dihydro-2H-pyridin-1-yl, 1,2,3,6-tetrahydropyridin-1-yl, pyrrol-1-yl, pyrazol-1-yl, imidazol-1-yl, triazol-1-yl, or tetrazol-1-yl, in which
R113 is hydrogen, 1-4C-alkyl or 1-4C-alkylcarbonyl, and
R2 is hydroxyl,
R3 is hydrogen, 1-4C-alkyl, halogen, 1-4C-alkoxy, or completely or predominantly fluorine-substituted 1-4C-alkoxy,
R4 is hydrogen or halogen,
R5 is hydrogen,
or a salt, stereoisomer or a salt of a stereoisomer thereof.

8. Compound according to any of claims 1 to 7, in which
R1 is 2-7C-alkyl substituted by -N(R111)R112, in which
R111 is 1-4C-alkyl,
R112 is hydrogen or 1-4C-alkyl,
or R111 and R112 together and with inclusion of the nitrogen atom, to which they are bonded, form a ring Het, in which
Het is piperidin-1-yl, morpholin-4-yl or pyrrolidin-1-yl,
R3 is hydrogen, chloro or methoxy,
R4 is hydrogen,
either
R2 is hydroxyl, and
R5 is hydrogen,
or
R2 is hydroxyl, and
R5 is 1-4C-alkyl,
or
R2 is hydrogen, and
R5 is hydrogen,
or
R2 is hydrogen, and
R5 is 1-4C-alkyl,
or a salt, stereoisomer or a salt of a stereoisomer thereof.

9. Compound according to any of claims 1 to 8, in which the stereochemistry is as shown in formulae I* 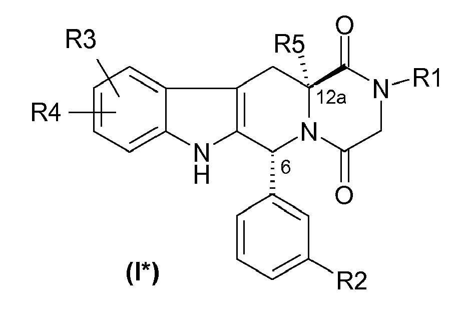 or a salt thereof.

10. A compound according to claim 1, which is from any of the formulae I-AD*, I-AE*, I-BD*, I-BE*, I-AD**, I-AE**, I-BD** or I-BE**, 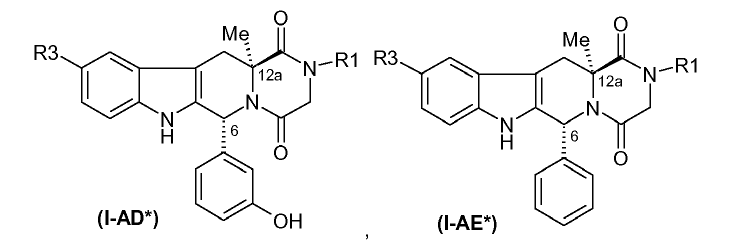 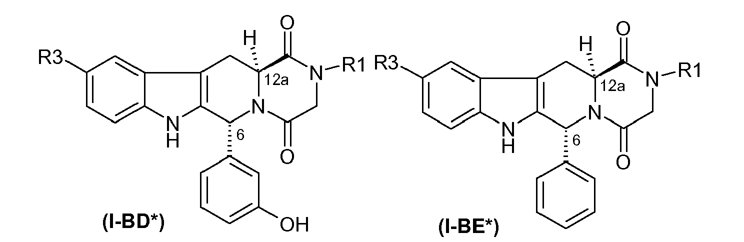 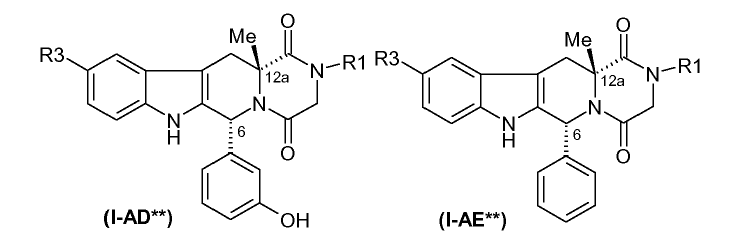 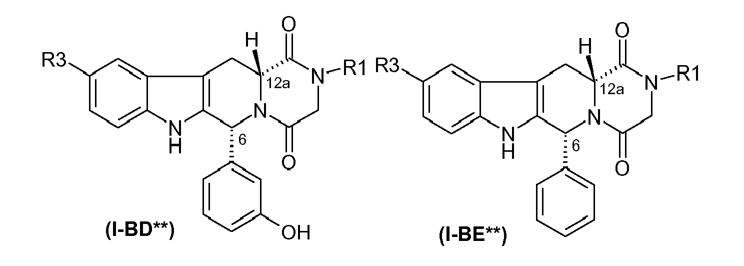 and in which R1 and R3 have any of the following meanings 1.1 to 1.390:
| **No.** | **R1** | **R3** |
|---|---|---|
| 1.1 | 2-bromo-ethyl | methoxy |
| 1.2 | 2-amino-ethyl | methoxy |
| 1.3 | 2-methylamino-ethyl | methoxy |
| 1.4 | 2-ethylamino-ethyl | methoxy |
| 1.5 | 2-(2,2-difluoroethyl)-amino-ethyl | methoxy |
| 1.6 | 2-isopropylamino-ethyl | methoxy |
| 1.7 | 2-isobutylamino-ethyl | methoxy |
| 1.8 | 2-cyclopropylmethylamino-ethyl | methoxy |
| 1.9 | 2-(2-hydroxy-ethylamino)-ethyl | methoxy |
| 1.10 | 2-(2-methoxy-ethylamino)-ethyl | methoxy |
| 1.11 | 2-cyclopropylamino-ethyl | methoxy |
| 1.12 | 2-cyclobutylamino-ethyl | methoxy |
| 1.13 | 2-*tert*-butylamino-ethyl | methoxy |
| 1.14 | 2-allylam ino-ethyl | methoxy |
| 1.15 | 2-prop-2-ynylamino-ethyl | methoxy |
| 1.16 | 2-(1-methyl-1H-pyrazol-2-ylamino)-ethyl | methoxy |
| 1.17 | 2-(2-methyl-2H-pyrazol-3-ylamino)-ethyl | methoxy |
| 1.18 | 2-dimethylamino-ethyl | methoxy |
| 1.19 | 2-(ethyl-methyl-am ino)-ethyl | methoxy |
| 1.20 | 2-[(2-hydroxy-ethyl)-methyl-amino]-ethyl | methoxy |
| 1.21 | 2-diethylamino-ethyl | methoxy |
| 1.22 | 2-[(2-hyd roxy-ethyl)-ethyl-am ino]-ethyl | methoxy |
| 1.23 | 2-[(2-methoxy-ethyl)-ethyl-am ino]-ethyl | methoxy |
| 1.24 | 2-(allyl-methyl-am ino)-ethyl | methoxy |
| 1.25 | 2-(methyl-prop-2-ynyl-am ino)-ethyl | methoxy |
| 1.26 | 2-(isopropyl-methyl-am ino)-ethyl | methoxy |
| 1.27 | 2-azetidin-1-yl-ethyl | methoxy |
| 1.28 | 2-morpholin-4-yl-ethyl | methoxy |
| 1.29 | 2-pyrrolidin-1-yl-ethyl | methoxy |
| 1.30 | 2-(3.3-difluoro-pyrrolidin-1-yl)-ethyl | methoxy |
| 1.31 | 2-(2,5-dihydro-pyrrol-1-yl)-ethyl | methoxy |
| 1.32 | 2-piperidin-1-yl-ethyl | methoxy |
| 1.33 | 2-(4-methyl-piperidin-1-yl)-ethyl | methoxy |
| 1.34 | 2-(3,6-dihydro-2H-pyridin-1-yl)-ethyl | methoxy |
| 1.35 | 2-(4-methyl-piperazin-1-yl)-ethyl | methoxy |
| 1.36 | 2-(4-acetyl-piperazin-1-yl)-ethyl | methoxy |
| 1.37 | 2-azepan-1-yl-ethyl | methoxy |
| 1.38 | 2-imidazol-1-yl-ethyl | methoxy |
| 1.39 | 3-chloro-n-propyl | methoxy |
| 1.40 | 3-amino-n-propyl | methoxy |
| 1.41 | 3-methylamino-n-propyl | methoxy |
| 1.42 | 3-ethylamino-n-propyl | methoxy |
| 1.43 | 3-(2,2-difluoroethyl)-amino-n-propyl | methoxy |
| 1.44 | 3-isopropylamino-n-propyl | methoxy |
| 1.45 | 3-isobutylamino-n-propyl | methoxy |
| 1.46 | 3-cyclopropylmethylamino-n-propyl | methoxy |
| 1.47 | 3-(2-hydroxy-ethylamino)-n-propyl | methoxy |
| 1.48 | 3-(2-hydroxy-ethylamino)-n-propyl | methoxy |
| 1.49 | 3-cyclopropylamino-n-propyl | methoxy |
| 1.50 | 3-cyclobutylamino-n-propyl | methoxy |
| 1.51 | 3-*tert*-butylamino-n-propyl | methoxy |
| 1.52 | 3-allylam ino-n-propyl | methoxy |
| 1.53 | 3-prop-2-ynylamino-n-propyl | methoxy |
| 1.54 | 3-(1-methyl-1H-pyrazol-2-ylamino)-n-propyl | methoxy |
| 1.55 | 3-(2-methyl-2H-pyrazol-3-ylamino)-n-propyl | methoxy |
| 1.56 | 3-dimethylamino-n-propyl | methoxy |
| 1.57 | 3-(ethyl-methyl-am ino)-n-propyl | methoxy |
| 1.58 | 3-[(2-hydroxy-ethyl)-methyl-amino]-n-propyl | methoxy |
| 1.59 | 3-diethylamino-n-propyl | methoxy |
| 1.60 | 3-[(2-hydroxy-ethyl)-ethyl-amino]-n-propyl | methoxy |
| 1.61 | 3-[(2-methoxy-ethyl)-ethyl-amino]-n-propyl | methoxy |
| 1.62 | 3-(allyl-methyl-am ino)-n-propyl | methoxy |
| 1.63 | 3-(methyl-prop-2-ynyl-am ino)-n-propyl | methoxy |
| 1.64 | 3-(isopropyl-methyl-am ino)-n-propyl | methoxy |
| 1.65 | 3-azetidin-1-yl-n-propyl | methoxy |
| 1.66 | 3-morpholin-4-yl-n-propyl | methoxy |
| 1.67 | 3-pyrrolidin-1-yl-n-propyl | methoxy |
| 1.68 | 3-(3.3-difluoro-pyrrolidin-1-yl)-n-propyl | methoxy |
| 1.69 | 3-(2,5-dihydro-pyrrol-1-yl)-n-propyl | methoxy |
| 1.70 | 3-piperidin-1-yl-n-propyl | methoxy |
| 1.71 | 3-(4-methyl-piperidin-1-yl)-n-propyl | methoxy |
| 1.72 | 3-(3,6-dihydro-2H-pyridin-1-yl)-n-propyl | methoxy |
| 1.73 | 3-(4-methyl-piperazin-1-yl)-n-propyl | methoxy |
| 1.74 | 3-(4-acetyl-piperazin-1-yl)-n-propyl | methoxy |
| 1.75 | 3-azepan-1-yl-n-propyl | methoxy |
| 1.76 | 3-imidazol-1-yl-n-propyl | methoxy |
| 1.77 | 2-bromo-ethyl | ethoxy |
| 1.78 | 2-amino-ethyl | ethoxy |
| 1.79 | 2-methylamino-ethyl | ethoxy |
| 1.80 | 2-ethylamino-ethyl | ethoxy |
| 1.81 | 2-(2,2-difluoroethyl)-amino-ethyl | ethoxy |
| 1.82 | 2-isopropylamino-ethyl | ethoxy |
| 1.83 | 2-isobutylamino-ethyl | ethoxy |
| 1.84 | 2-cyclopropylmethylamino-ethyl | ethoxy |
| 1.85 | 2-(2-hydroxy-ethylamino)-ethyl | ethoxy |
| 1.86 | 2-(2-methoxy-ethylamino)-ethyl | ethoxy |
| 1.87 | 2-cyclopropylamino-ethyl | ethoxy |
| 1.88 | 2-cyclobutylamino-ethyl | ethoxy |
| 1.89 | 2-*tert*-butylamino-ethyl | ethoxy |
| 1.90 | 2-allylamino-ethyl | ethoxy |
| 1.91 | 2-prop-2-ynylamino-ethyl | ethoxy |
| 1.92 | 2-(1-methyl-1H-pyrazol-2-ylamino)-ethyl | ethoxy |
| 1.93 | 2-(2-methyl-2H-pyrazol-3-ylamino)-ethyl | ethoxy |
| 1.94 | 2-dimethylamino-ethyl | ethoxy |
| 1.95 | 2-(ethyl-methyl-amino)-ethyl | ethoxy |
| 1.96 | 2-[(2-hydroxy-ethyl)-methyl-amino]-ethyl | ethoxy |
| 1.97 | 2-diethylamino-ethyl | ethoxy |
| 1.98 | 2-[(2-hydroxy-ethyl)-ethyl-amino]-ethyl | ethoxy |
| 1.99 | 2-[(2-methoxy-ethyl)-ethyl-amino]-ethyl | ethoxy |
| 1.100 | 2-(allyl-methyl-amino)-ethyl | ethoxy |
| 1.101 | 2-(methyl-prop-2-ynyl-amino)-ethyl | ethoxy |
| 1.102 | 2-(isopropyl-methyl-amino)-ethyl | ethoxy |
| 1.103 | 2-azetidin-1-yl-ethyl | ethoxy |
| 1.104 | 2-morpholin-4-yl-ethyl | ethoxy |
| 1.105 | 2-pyrrolidin-1-yl-ethyl | ethoxy |
| 1.106 | 2-(3.3-difluoro-pyrrolidin-1-yl)-ethyl | ethoxy |
| 1.107 | 2-(2,5-dihydro-pyrrol-1-yl)-ethyl | ethoxy |
| 1.108 | 2-piperidin-1-yl-ethyl | ethoxy |
| 1.109 | 2-(4-methyl-piperidin-1-yl)-ethyl | ethoxy |
| 1.110 | 2-(3,6-dihydro-2H-pyridin-1-yl)-ethyl | ethoxy |
| 1.111 | 2-(4-methyl-piperazin-1-yl)-ethyl | ethoxy |
| 1.112 | 2-(4-acetyl-piperazin-1-yl)-ethyl | ethoxy |
| 1.113 | 2-azepan-1-yl-ethyl | ethoxy |
| 1.114 | 2-imidazol-1-yl-ethyl | ethoxy |
| 1.115 | 3-chloro-n-propyl | ethoxy |
| 1.116 | 3-amino-n-propyl | ethoxy |
| 1.117 | 3-methylamino-n-propyl | ethoxy |
| 1.118 | 3-ethylamino-n-propyl | ethoxy |
| 1.119 | 3-(2,2-difluoroethyl)-amino-n-propyl | ethoxy |
| 1.120 | 3-isopropylamino-n-propyl | ethoxy |
| 1.121 | 3-isobutylamino-n-propyl | ethoxy |
| 1.122 | 3-cyclopropylmethylamino-n-propyl | ethoxy |
| 1.123 | 3-(2-hydroxy-ethylamino)-n-propyl | ethoxy |
| 1.124 | 3-(2-hydroxy-ethylamino)-n-propyl | ethoxy |
| 1.125 | 3-cyclopropylamino-n-propyl | ethoxy |
| 1.126 | 3-cyclobutylamino-n-propyl | ethoxy |
| 1.127 | 3-*tert*-butylamino-n-propyl | ethoxy |
| 1.128 | 3-allylamino-n-propyl | ethoxy |
| 1.129 | 3-prop-2-ynylamino-n-propyl | ethoxy |
| 1.130 | 3-(1-methyl-1H-pyrazol-2-ylamino)-n-propyl | ethoxy |
| 1.131 | 3-(2-methyl-2H-pyrazol-3-ylamino)-n-propyl | ethoxy |
| 1.132 | 3-dimethylamino-n-propyl | ethoxy |
| 1.133 | 3-(ethyl-methyl-am ino)-n-propyl | ethoxy |
| 1.134 | 3-[(2-hydroxy-ethyl)-methyl-amino]-n-propyl | ethoxy |
| 1.135 | 3-diethylamino-n-propyl | ethoxy |
| 1.136 | 3-[(2-hydroxy-ethyl)-ethyl-amino]-n-propyl | ethoxy |
| 1.137 | 3-[(2-methoxy-ethyl)-ethyl-amino]-n-propyl | ethoxy |
| 1.138 | 3-(allyl-methyl-am ino)-n-propyl | ethoxy |
| 1.139 | 3-(methyl-prop-2-ynyl-am ino)-n-propyl | ethoxy |
| 1.140 | 3-(isopropyl-methyl-amino)-n-propyl | ethoxy |
| 1.141 | 3-azetid in-1-yl-n-propyl | ethoxy |
| 1.142 | 3-morpholin-4-yl-n-propyl | ethoxy |
| 1.143 | 3-pyrrolidin-1-yl-n-propyl | ethoxy |
| 1.144 | 3-(3.3-difluoro-pyrrolidin-1-yl)-n-propyl | ethoxy |
| 1.145 | 3-(2,5-dihydro-pyrrol-1-yl)-n-propyl | ethoxy |
| 1.146 | 3-piperidin-1-yl-n-propyl | ethoxy |
| 1.147 | 3-(4-methyl-piperidin-1-yl)-n-propyl | ethoxy |
| 1.148 | 3-(3,6-dihydro-2H-pyridin-1-yl)-n-propyl | ethoxy |
| 1.149 | 3-(4-methyl-piperazin-1-yl)-n-propyl | ethoxy |
| 1.150 | 3-(4-acetyl-piperazin-1-yl)-n-propyl | ethoxy |
| 1.151 | 3-azepan-1-yl-n-propyl | ethoxy |
| 1.152 | 3-imidazol-1-yl-n-propyl | ethoxy |
| 1.153 | 2-bromo-ethyl | difluoromethoxy |
| 1.154 | 2-amino-ethyl | difluoromethoxy |
| 1.155 | 2-methylamino-ethyl | difluoromethoxy |
| 1.156 | 2-ethylamino-ethyl | difluoromethoxy |
| 1.157 | 2-(2,2-difluoroethyl)-amino-ethyl | difluoromethoxy |
| 1.158 | 2-isopropylamino-ethyl | difluoromethoxy |
| 1.159 | 2-isobutylamino-ethyl | difluoromethoxy |
| 1.160 | 2-cyclopropylmethylamino-ethyl | difluoromethoxy |
| 1.161 | 2-(2-hyd roxy-ethylam ino)-ethyl | d ifluoromethoxy |
| 1.162 | 2-(2-methoxy-ethylamino)-ethyl | difluoromethoxy |
| 1.163 | 2-cyclopropylamino-ethyl | difluoromethoxy |
| 1.164 | 2-cyclobutylamino-ethyl | difluoromethoxy |
| 1.165 | 2-*tert*-butylamino-ethyl | difluoromethoxy |
| 1.166 | 2-allylamino-ethyl | difluoromethoxy |
| 1.167 | 2-prop-2-ynylamino-ethyl | difluoromethoxy |
| 1.168 | 2-(1-methyl-1H-pyrazol-2-ylamino)-ethyl | difluoromethoxy |
| 1.169 | 2-(2-methyl-2H-pyrazol-3-ylamino)-ethyl | difluoromethoxy |
| 1.170 | 2-dimethylamino-ethyl | difluoromethoxy |
| 1.171 | 2-(ethyl-methyl-am ino)-ethyl | d ifluoromethoxy |
| 1.172 | 2-[(2-hydroxy-ethyl)-methyl-amino]-ethyl | difluoromethoxy |
| 1.173 | 2-diethylamino-ethyl | difluoromethoxy |
| 1.174 | 2-[(2-hydroxy-ethyl)-ethyl-amino]-ethyl | difluoromethoxy |
| 1.175 | 2-[(2-methoxy-ethyl)-ethyl-amino]-ethyl | difluoromethoxy |
| 1.176 | 2-(allyl-methyl-am ino)-ethyl | difluoromethoxy |
| 1.177 | 2-(methyl-prop-2-ynyl-am ino)-ethyl | difluoromethoxy |
| 1.178 | 2-(isopropyl-methyl-am ino)-ethyl | difluoromethoxy |
| 1.179 | 2-azetidin-1-yl-ethyl | difluoromethoxy |
| 1.180 | 2-morpholin-4-yl-ethyl | difluoromethoxy |
| 1.181 | 2-pyrrolidin-1-yl-ethyl | difluoromethoxy |
| 1.182 | 2-(3.3-difluoro-pyrrolidin-1-yl)-ethyl | difluoromethoxy |
| 1.183 | 2-(2,5-dihydro-pyrrol-1-yl)-ethyl | difluoromethoxy |
| 1.184 | 2-piperidin-1-yl-ethyl | difluoromethoxy |
| 1.185 | 2-(4-methyl-piperidin-1-yl)-ethyl | difluoromethoxy |
| 1.186 | 2-(3,6-dihydro-2H-pyridin-1-yl)-ethyl | difluoromethoxy |
| 1.187 | 2-(4-methyl-piperazin-1-yl)-ethyl | difluoromethoxy |
| 1.188 | 2-(4-acetyl-piperazin-1-yl)-ethyl | difluoromethoxy |
| 1.189 | 2-azepan-1-yl-ethyl | difluoromethoxy |
| 1.190 | 2-imidazol-1-yl-ethyl | difluoromethoxy |
| 1.191 | 3-chloro-n-propyl | d ifluoromethoxy |
| 1.192 | 3-amino-n-propyl | difluoromethoxy |
| 1.193 | 3-methylamino-n-propyl | difluoromethoxy |
| 1.194 | 3-ethylamino-n-propyl | difluoromethoxy |
| 1.195 | 3-(2,2-difluoroethyl)-amino-n-propyl | difluoromethoxy |
| 1.196 | 3-isopropylamino-n-propyl | difluoromethoxy |
| 1.197 | 3-isobutylamino-n-propyl | difluoromethoxy |
| 1.198 | 3-cyclopropylmethylamino-n-propyl | difluoromethoxy |
| 1.199 | 3-(2-hyd roxy-ethylam ino)-n-propyl | difluoromethoxy |
| 1.200 | 3-(2-hydroxy-ethylamino)-n-propyl | difluoromethoxy |
| 1.201 | 3-cyclopropylamino-n-propyl | difluoromethoxy |
| 1.202 | 3-cyclobutylamino-n-propyl | difluoromethoxy |
| 1.203 | 3-*tert*-butylamino-n-propyl | difluoromethoxy |
| 1.204 | 3-allylamino-n-propyl | difluoromethoxy |
| 1.205 | 3-prop-2-ynylamino-n-propyl | difluoromethoxy |
| 1.206 | 3-(1-methyl-1H-pyrazol-2-ylamino)-n-propyl | difluoromethoxy |
| 1.207 | 3-(2-methyl-2H-pyrazol-3-ylamino)-n-propyl | difluoromethoxy |
| 1.208 | 3-dimethylamino-n-propyl | difluoromethoxy |
| 1.209 | 3-(ethyl-methyl-amino)-n-propyl | difluoromethoxy |
| 1.210 | 3-[(2-hydroxy-ethyl)-methyl-amino]-n-propyl | difluoromethoxy |
| 1.211 | 3-diethylamino-n-propyl | difluoromethoxy |
| 1.212 | 3-[(2-hydroxy-ethyl)-ethyl-amino]-n-propyl | difluoromethoxy |
| 1.213 | 3-[(2-methoxy-ethyl)-ethyl-am ino]-n-propyl | difluoromethoxy |
| 1.214 | 3-(allyl-methyl-amino)-n-propyl | difluoromethoxy |
| 1.215 | 3-(methyl-prop-2-ynyl-am ino)-n-propyl | difluoromethoxy |
| 1.216 | 3-(isopropyl-methyl-amino)-n-propyl | difluoromethoxy |
| 1.217 | 3-azetidin-1-yl-n-propyl | difluoromethoxy |
| 1.218 | 3-morpholin-4-yl-n-propyl | difluoromethoxy |
| 1.219 | 3-pyrrolidin-1-yl-n-propyl | difluoromethoxy |
| 1.220 | 3-(3.3-difluoro-pyrrolidin-1-yl)-n-propyl | difluoromethoxy |
| 1.221 | 3-(2,5-dihydro-pyrrol-1-yl)-n-propyl | d ifluoromethoxy |
| 1.222 | 3-piperidin-1-yl-n-propyl | difluoromethoxy |
| 1.223 | 3-(4-methyl-piperidin-1-yl)-n-propyl | difluoromethoxy |
| 1.224 | 3-(3,6-dihydro-2H-pyridin-1-yl)-n-propyl | difluoromethoxy |
| 1.225 | 3-(4-methyl-piperazin-1-yl)-n-propyl | difluoromethoxy |
| 1.226 | 3-(4-acetyl-piperazin-1-yl)-n-propyl | difluoromethoxy |
| 1.227 | 3-azepan-1-yl-n-propyl | difluoromethoxy |
| 1.228 | 3-imidazol-1-yl-n-propyl | difluoromethoxy |
| 1.229 | 2-bromo-ethyl | chloro |
| 1.230 | 2-amino-ethyl | chloro |
| 1.231 | 2-methylamino-ethyl | chloro |
| 1.232 | 2-ethylamino-ethyl | chloro |
| 1.233 | 2-(2,2-difluoroethyl)-amino-ethyl | chloro |
| 1.234 | 2-isopropylamino-ethyl | chloro |
| 1.235 | 2-isobutylamino-ethyl | chloro |
| 1.236 | 2-cyclopropylmethylamino-ethyl | chloro |
| 1.237 | 2-(2-hydroxy-ethylamino)-ethyl | chloro |
| 1.238 | 2-(2-methoxy-ethylamino)-ethyl | chloro |
| 1.239 | 2-cyclopropylamino-ethyl | chloro |
| 1.240 | 2-cyclobutylamino-ethyl | chloro |
| 1.241 | 2-*tert*-butylamino-ethyl | chloro |
| 1.242 | 2-allylamino-ethyl | chloro |
| 1.243 | 2-prop-2-ynylamino-ethyl | chloro |
| 1.244 | 2-(1-methyl-1H-pyrazol-2-ylamino)-ethyl | chloro |
| 1.245 | 2-(2-methyl-2H-pyrazol-3-ylamino)-ethyl | chloro |
| 1.246 | 2-dimethylamino-ethyl | chloro |
| 1.247 | 2-(ethyl-methyl-am ino)-ethyl | chloro |
| 1.248 | 2-[(2-hydroxy-ethyl)-methyl-amino]-ethyl | chloro |
| 1.249 | 2-diethylamino-ethyl | chloro |
| 1.250 | 2-[(2-hydroxy-ethyl)-ethyl-amino]-ethyl | chloro |
| 1.251 | 2-[(2-methoxy-ethyl)-ethyl-am ino]-ethyl | chloro |
| 1.252 | 2-(allyl-methyl-am ino)-ethyl | chloro |
| 1.253 | 2-(methyl-prop-2-ynyl-am ino)-ethyl | chloro |
| 1.254 | 2-(isopropyl-methyl-am ino)-ethyl | chloro |
| 1.255 | 2-azetidin-1-yl-ethyl | chloro |
| 1.256 | 2-morpholin-4-yl-ethyl | chloro |
| 1.257 | 2-pyrrolidin-1-yl-ethyl | chloro |
| 1.258 | 2-(3.3-difluoro-pyrrolidin-1-yl)-ethyl | chloro |
| 1.259 | 2-(2,5-dihydro-pyrrol-1-yl)-ethyl | chloro |
| 1.260 | 2-piperidin-1-yl-ethyl | chloro |
| 1.261 | 2-(4-methyl-piperidin-1-yl)-ethyl | chloro |
| 1.262 | 2-(3,6-dihydro-2H-pyridin-1-yl)-ethyl | chloro |
| 1.263 | 2-(4-methyl-piperazin-1-yl)-ethyl | chloro |
| 1.264 | 2-(4-acetyl-piperazin-1-yl)-ethyl | chloro |
| 1.265 | 2-azepan-1-yl-ethyl | chloro |
| 1.266 | 2-imidazol-1-yl-ethyl | chloro |
| 1.267 | 3-chloro-n-propyl | chloro |
| 1.268 | 3-amino-n-propyl | chloro |
| 1.269 | 3-methylamino-n-propyl | chloro |
| 1.270 | 3-ethylamino-n-propyl | chloro |
| 1.271 | 3-(2,2-difluoroethyl)-amino-n-propyl | chloro |
| 1.272 | 3-isopropylamino-n-propyl | chloro |
| 1.273 | 3-isobutylamino-n-propyl | chloro |
| 1.274 | 3-cyclopropylmethylamino-n-propyl | chloro |
| 1.275 | 3-(2-hydroxy-ethylamino)-n-propyl | chloro |
| 1.276 | 3-(2-hydroxy-ethylamino)-n-propyl | chloro |
| 1.277 | 3-cyclopropylamino-n-propyl | chloro |
| 1.278 | 3-cyclobutylamino-n-propyl | chloro |
| 1.279 | 3-*tert*-butylamino-n-propyl | chloro |
| 1.280 | 3-allylamino-n-propyl | chloro |
| 1.281 | 3-prop-2-ynylamino-n-propyl | chloro |
| 1.282 | 3-(1-methyl-1H-pyrazol-2-ylamino)-n-propyl | chloro |
| 1.283 | 3-(2-methyl-2H-pyrazol-3-ylamino)-n-propyl | chloro |
| 1.284 | 3-dimethylamino-n-propyl | chloro |
| 1.285 | 3-(ethyl-methyl-amino)-n-propyl | chloro |
| 1.286 | 3-[(2-hydroxy-ethyl)-methyl-amino]-n-propyl | chloro |
| 1.287 | 3-diethylamino-n-propyl | chloro |
| 1.288 | 3-[(2-hydroxy-ethyl)-ethyl-amino]-n-propyl | chloro |
| 1.289 | 3-[(2-methoxy-ethyl)-ethyl-amino]-n-propyl | chloro |
| 1.290 | 3-(allyl-methyl-amino)-n-propyl | chloro |
| 1.291 | 3-(methyl-prop-2-ynyl-am ino)-n-propyl | chloro |
| 1.292 | 3-(isopropyl-methyl-amino)-n-propyl | chloro |
| 1.293 | 3-azetidin-1-yl-n-propyl | chloro |
| 1.294 | 3-morpholin-4-yl-n-propyl | chloro |
| 1.295 | 3-pyrrolidin-1-yl-n-propyl | chloro |
| 1.296 | 3-(3.3-difluoro-pyrrolidin-1-yl)-n-propyl | chloro |
| 1.297 | 3-(2,5-dihydro-pyrrol-1-yl)-n-propyl | chloro |
| 1.298 | 3-piperidin-1-yl-n-propyl | chloro |
| 1.299 | 3-(4-methyl-piperidin-1-yl)-n-propyl | chloro |
| 1.300 | 3-(3,6-dihydro-2H-pyridin-1-yl)-n-propyl | chloro |
| 1.301 | 3-(4-methyl-piperazin-1-yl)-n-propyl | chloro |
| 1.302 | 3-(4-acetyl-piperazin-1-yl)-n-propyl | chloro |
| 1.303 | 3-azepan-1-yl-n-propyl | chloro |
| 1.304 | 3-imidazol-1-yl-n-propyl | chloro |
| 1.305 | 2-bromo-ethyl | bromo |
| 1.306 | 2-amino-ethyl | bromo |
| 1.307 | 2-methylamino-ethyl | bromo |
| 1.308 | 2-ethylamino-ethyl | bromo |
| 1.309 | 2-(2,2-difluoroethyl)-amino-ethyl | bromo |
| 1.310 | 2-isopropylamino-ethyl | bromo |
| 1.311 | 2-isobutylamino-ethyl | bromo |
| 1.312 | 2-cyclopropylmethylamino-ethyl | bromo |
| 1.313 | 2-(2-hydroxy-ethylamino)-ethyl | bromo |
| 1.314 | 2-(2-methoxy-ethylamino)-ethyl | bromo |
| 1.315 | 2-cyclopropylamino-ethyl | bromo |
| 1.316 | 2-cyclobutylamino-ethyl | bromo |
| 1.317 | 2-*tert*-butylamino-ethyl | bromo |
| 1.318 | 2-allylamino-ethyl | bromo |
| 1.319 | 2-prop-2-ynylamino-ethyl | bromo |
| 1.320 | 2-(1-methyl-1H-pyrazol-2-ylamino)-ethyl | bromo |
| 1.321 | 2-(2-methyl-2H-pyrazol-3-ylamino)-ethyl | bromo |
| 1.322 | 2-dimethylamino-ethyl | bromo |
| 1.323 | 2-(ethyl-methyl-am ino)-ethyl | bromo |
| 1.324 | 2-[(2-hydroxy-ethyl)-methyl-amino]-ethyl | bromo |
| 1.325 | 2-diethylamino-ethyl | bromo |
| 1.326 | 2-[(2-hydroxy-ethyl)-ethyl-amino]-ethyl | bromo |
| 1.327 | 2-[(2-methoxy-ethyl)-ethyl-amino]-ethyl | bromo |
| 1.328 | 2-(allyl-methyl-am ino)-ethyl | bromo |
| 1.329 | 2-(methyl-prop-2-ynyl-am ino)-ethyl | bromo |
| 1.330 | 2-(isopropyl-methyl-am ino)-ethyl | bromo |
| 1.331 | 2-azetidin-1-yl-ethyl | bromo |
| 1.332 | 2-morpholin-4-yl-ethyl | bromo |
| 1.333 | 2-pyrrolidin-1-yl-ethyl | bromo |
| 1.334 | 2-(3.3-difluoro-pyrrolidin-1-yl)-ethyl | bromo |
| 1.335 | 2-(2,5-dihydro-pyrrol-1-yl)-ethyl | bromo |
| 1.336 | 2-piperidin-1-yl-ethyl | bromo |
| 1.337 | 2-(4-methyl-piperidin-1-yl)-ethyl | bromo |
| 1.338 | 2-(3,6-dihydro-2H-pyridin-1-yl)-ethyl | bromo |
| 1.339 | 2-(4-methyl-piperazin-1-yl)-ethyl | bromo |
| 1.340 | 2-(4-acetyl-piperazin-1-yl)-ethyl | bromo |
| 1.341 | 2-azepan-1-yl-ethyl | bromo |
| 1.342 | 2-imidazol-1-yl-ethyl | bromo |
| 1.343 | 3-chloro-n-propyl | bromo |
| 1.344 | 3-amino-n-propyl | bromo |
| 1.345 | 3-methylamino-n-propyl | bromo |
| 1.346 | 3-ethylamino-n-propyl | bromo |
| 1.347 | 3-(2,2-difluoroethyl)-amino-n-propyl | bromo |
| 1.348 | 3-isopropylamino-n-propyl | bromo |
| 1.349 | 3-isobutylamino-n-propyl | bromo |
| 1.350 | 3-cyclopropylmethylamino-n-propyl | bromo |
| 1.351 | 3-(2-hyd roxy-ethylam ino)-n-propyl | bromo |
| 1.352 | 3-(2-hydroxy-ethylamino)-n-propyl | bromo |
| 1.353 | 3-cyclopropylamino-n-propyl | bromo |
| 1.354 | 3-cyclobutylamino-n-propyl | bromo |
| 1.355 | 3-*tert*-butylamino-n-propyl | bromo |
| 1.356 | 3-allylamino-n-propyl | bromo |
| 1.357 | 3-prop-2-ynylamino-n-propyl | bromo |
| 1.358 | 3-(1-methyl-1H-pyrazol-2-ylamino)-n-propyl | bromo |
| 1.359 | 3-(2-methyl-2H-pyrazol-3-ylamino)-n-propyl | bromo |
| 1.360 | 3-dimethylamino-n-propyl | bromo |
| 1.361 | 3-(ethyl-methyl-am ino)-n-propyl | bromo |
| 1.362 | 3-[(2-hydroxy-ethyl)-methyl-amino]-n-propyl | bromo |
| 1.363 | 3-diethylamino-n-propyl | bromo |
| 1.364 | 3-[(2-hydroxy-ethyl)-ethyl-amino]-n-propyl | bromo |
| 1.365 | 3-[(2-methoxy-ethyl)-ethyl-amino]-n-propyl | bromo |
| 1.366 | 3-(allyl-methyl-amino)-n-propyl | bromo |
| 1.367 | 3-(methyl-prop-2-ynyl-amino)-n-propyl | bromo |
| 1.368 | 3-(isopropyl-methyl-amino)-n-propyl | bromo |
| 1.369 | 3-azetidin-1-yl-n-propyl | bromo |
| 1.370 | 3-morpholin-4-yl-n-propyl | bromo |
| 1.371 | 3-pyrrolidin-1-yl-n-propyl | bromo |
| 1.372 | 3-(3.3-difluoro-pyrrolidin-1-yl)-n-propyl | bromo |
| 1.373 | 3-(2,5-dihydro-pyrrol-1-yl)-n-propyl | bromo |
| 1.374 | 3-piperidin-1-yl-n-propyl | bromo |
| 1.375 | 3-(4-methyl-piperidin-1-yl)-n-propyl | bromo |
| 1.376 | 3-(3,6-dihydro-2H-pyridin-1-yl)-n-propyl | bromo |
| 1.377 | 3-(4-methyl-piperazin-1-yl)-n-propyl | bromo |
| 1.378 | 3-(4-acetyl-piperazin-1-yl)-n-propyl | bromo |
| 1.379 | 3-azepan-1-yl-n-propyl | bromo |
| 1.380 | 3-imidazol-1-yl-n-propyl | bromo |
| 1.381 | methyl | methoxy |
| 1.382 | methyl | ethoxy |
| 1.383 | methyl | difluoromethoxy |
| 1.384 | methyl | chloro |
| 1.385 | methyl | bromo |
| 1.386 | ethyl | methoxy |
| 1.387 | ethyl | ethoxy |
| 1.388 | ethyl | difluoromethoxy |
| 1.389 | ethyl | chloro |
| 1.390 | ethyl | bromo |
or a salt, stereoisomer or a salt of a stereoisomer thereof.

11. A compound according to claim 1, which is selected from
1. (6RS,12aRS)-2-(2-Dimethylamino-ethyl)-6-(3-hydroxy-phenyl)-2,3,6,7,12,12a-hexahydro-pyrazino[1',2':1,6]pyrido[3,4-b]indole-1,4-dione and (6RS,12aSR)-2-(2-Dimethylamino-ethyl)-6-(3-hydroxy-phenyl)-2,3,6,7,12,12a-hexahydro-pyrazino[1',2':1,6]pyrido[3,4-b]indole-1,4-dione
2. (6RS,12aRS)-6-(3-Hydroxy-phenyl)-2-methyl-2,3,6,7,12,12a-hexahydro-pyrazino[1',2':1,6]pyrido[3,4-b]indole-1,4-dione,
3. (6RS,12aSR)-6-(3-Hydroxy-phenyl)-2-methyl-2,3,6,7,12,12a-hexahydro-pyrazino[1',2':1,6]pyrido[3,4-b]indole-1,4-dione,
4. (6RS,12aRS)-6-(3-Hydroxy-phenyl)-2-(2-piperidin-1-yl-ethyl)-2,3,6,7,12,12a-hexahydro-pyrazino[1',2':1,6]pyrido[3,4-b]indole-1,4-dione,
5. (6RS,12aRS)-6-(3-Hydroxy-phenyl)-2-(2-pyrrolidin-1-yl-ethyl)-2,3,6,7,12,12a-hexahydro-pyrazino[1',2':1,6]pyrido[3,4-b]indole-1,4-dione,
6. (6RS,12aSR)-6-(3-Hydroxy-phenyl)-2-(2-pyrrolidin-1-yl-ethyl)-2,3,6,7,12,12a-hexahydro-pyrazino[1',2':1,6]pyrido[3,4-b]indole-1,4-dione,
7. (6RS,12aSR)-6-(3-Hydroxy-phenyl)-2-(2-piperidin-1-yl-ethyl)-2,3,6,7,12,12a-hexahydro-pyrazino[1',2':1,6]pyrido[3,4-b]indole-1,4-dione,
8. (6RS,12aSR)-2-(2-Dimethylamino-ethyl)-6-(3-hydroxy-phenyl)-10-methoxy-12a-methyl-2,3,6,7,12,12a-hexahydro-pyrazino[1',2':1,6]pyrido[3,4-b]indole-1,4-dione,
9. (6RS,12aSR)-6-(3-Hydroxy-phenyl)-10-methoxy-12a-methyl-2-(2-pyrrolidin-1-yl-ethyl)-2,3,6,7,12,12a-hexahydro-pyrazino[1',2':1,6]pyrido[3,4-b]indole-1,4-dione,
10. (6RS,12aSR)-6-(3-Hydroxy-phenyl)-10-methoxy-2,12a-dimethyl-2,3,6,7,12,12a-hexahydro-pyrazino[1',2':1,6]pyrido[3,4-b]indole-1,4-dione,
11. (6RS,12aSR)-6-(3-Hydroxy-phenyl)-10-methoxy-12a-methyl-2-(2-piperidin-1-yl-ethyl)-2,3,6,7,12,12a-hexahydro-pyrazino[1',2':1,6]pyrido[3,4-b]indole-1,4-dione,
12. (6RS,12aSR)-2-(3-Dimethylamino-n-propyl)-6-(3-hydroxy-phenyl)-10-methoxy-12a-methyl-2,3,6,7,12,12a-hexahydro-pyrazino[1',2':1,6]pyrido[3,4-b]indole-1,4-dione,
13. (6RS,12aSR)-6-(3-Hydroxy-phenyl)-10-methoxy-12a-methyl-2-(2-morpholin-4-yl-ethyl)-2,3,6,7,12,12a-hexahydro-pyrazino[1',2':1,6]pyrido[3,4-b]indole-1,4-dione,
14. (6RS,12aSR)-6-(3-Hydroxy-phenyl)-10-methoxy-12a-methyl-2-(3-morpholin-4-yl-n-propyl)-2,3,6,7,12,12a-hexahydro-pyrazino[1',2':1,6]pyrido[3,4-b]indole-1,4-dione,
15. (6RS,12aSR)-2-(2-Diethylamino-ethyl)-6-(3-hydroxy-phenyl)-10-methoxy-12a-methyl-2,3,6,7,12,12a-hexahydro-pyrazino[1',2':1,6]pyrido[3,4-b]indole-1,4-dione,
16. (6RS,12aSR)-2-(3-Diethylamino-n-propyl)-6-(3-hydroxy-phenyl)-10-methoxy-12a-methyl-2,3,6,7,12,12a-hexahydro-pyrazino[1',2':1,6]pyrido[3,4-b]indole-1,4-dione,
17. (6RS,12aSR)-10-Chloro-6-(3-hydroxy-phenyl)-2-(2-morpholin-4-yl-ethyl)-2,3,6,7,12,12a-hexahydro-pyrazino[1',2':1,6]pyrido[3,4-b]indole-1,4-dione,
18. (6RS,12aSR)-10-Chloro-6-(3-hydroxy-phenyl)-2-(3-morpholin-4-yl-n-propyl)-2,3,6,7,12,12a-hexahydro-pyrazino[1',2':1,6]pyrido[3,4-b]indole-1,4-dione,
19. (6RS,12aSR)-2-(2-Diisopropylamino-ethyl)-6-(3-hydroxy-phenyl)-10-methoxy-12a-methyl-2,3,6,7,12,12a-hexahydro-pyrazino[1',2':1,6]pyrido[3,4-b]indole-1,4-dione,
20. (6RS,12aSR)-10-Chloro-2-(3-diethylamino-n-propyl)-6-(3-hydroxy-phenyl)-2,3,6,7,12,12a-hexahydro-pyrazino[1',2':1,6]pyrido[3,4-b]indole-1,4-dione,
21. (6RS,12aSR)-10-Chloro-6-(3-hydroxy-phenyl)-2-(2-pyrrolidin-1-yl-ethyl)-2,3,6,7,12,12a-hexahydro-pyrazino[1',2':1,6]pyrido[3,4-b]indole-1,4-dione,
22. (6RS,12aSR)-10-Chloro-2-(2-dimethylamino-ethyl)-6-(3-hydroxy-phenyl)-2,3,6,7,12,12a-hexahydro-pyrazino[1',2':1,6]pyrido[3,4-b]indole-1,4-dione,
23. (6RS,12aSR)-10-Chloro-2-(2-diethylamino-ethyl)-6-(3-hydroxy-phenyl)-2,3,6,7,12,12a-hexahydro-pyrazino[1',2':1,6]pyrido[3,4-b]indole-1,4-dione,
24. (6RS,12aSR)-10-Chloro-6-(3-hydroxy-phenyl)-2-(3-piperidin-1-yl-n-propyl)-2,3,6,7,12,12a-hexahydro-pyrazino[1',2':1,6]pyrido[3,4-b]indole-1,4-dione,
25. (6RS,12aSR)-6-(3-Hydroxy-phenyl)-2-(2-isopropylamino-ethyl)-10-methoxy-12a-methyl-2,3,6,7,12,12a-hexahydro-pyrazino[1',2':1,6]pyrido[3,4-b]indole-1,4-dione,
26. (6RS,12aSR)-10-Chloro-2-(2-diisopropylamino-ethyl)-6-(3-hydroxy-phenyl)-2,3,6,7,12,12a-hexahydro-pyrazino[1',2':1,6]pyrido[3,4-b]indole-1,4-dione,
27. (6RS,12aSR)-6-(3-Hydroxy-phenyl)-10-methoxy-12a-methyl-2-(3-piperidin-1-yl-n-propyl)-2,3,6,7,12,12a-hexahydro-pyrazino[1',2':1,6]pyrido[3,4-b]indole-1,4-dione,
28. (6RS,12aSR)-6-(3-Hydroxy-phenyl)-10-methoxy-12a-methyl-2-(3-pyrrolidin-1-yl-n-propyl)-2,3,6,7,12,12a-hexahydro-pyrazino[1',2':1,6]pyrido[3,4-b]indole-1,4-dione,
29. (6RS,12aSR)-6-(3-Hydroxy-phenyl)-10-methoxy-12a-methyl-2-(2-methylamino-ethyl)-2,3,6,7,12,12a-hexahydro-pyrazino[1',2':1,6]pyrido[3,4-b]indole-1,4-dione,
30. (6RS,12aSR)-2-(3-Dimethylamino-n-propyl)-6-(3-hydroxy-phenyl)-10-methoxy-2,3,6,7,12,12a-hexahydro-pyrazino[1',2':1,6]pyrido[3,4-b]indole-1,4-dione, and
31. (6RS,12aSR)-2-(3-Diethylamino-n-propyl)-6-(3-hydroxy-phenyl)-10-methoxy-2,3,6,7,12,12a-hexahydro-pyrazino[1',2':1,6]pyrido[3,4-b]indole-1,4-dione
or a salt thereof.

12. Compounds, or a salt, stereoisomer or a salt of a stereoisomer thereof, according to any of the claims 1 to 11 for use in the treatment of diseases.

13. A pharmaceutical composition comprising one or more compounds, or a salt, stereoisomer or a salt of a stereoisomer thereof, according to any of the claims 1 to 11 together with pharmaceutically acceptable auxiliaries and/or excipients.

14. Use of the compounds, or a salt, stereoisomer or a salt of a stereoisomer thereof, according to any of the claims 1 to 11 in the production of pharmaceutical compositions for the treatment, prevention or amelioration of hyperproliferative diseases and disorders responsive to the induction of apoptosis.

15. A compound or a salt, stereoisomer or a salt of a stereoisomer thereof, according to any of claims 1 to 11, or a pharmaceutical composition comprising a compound or a salt, stereoisomer or a salt of a stereoisomer thereof, according to any of claims 1 to 11, for the treatment, prevention or amelioration of hyperproliferative diseases and disorders responsive to the induction of apoptosis.

16. A method for treating, preventing or ameliorating hyperproliferative diseases and disorders responsive to the induction of apoptosis in mammals, comprising administering to said mammals in need thereof a pharmaceutically active and therapeutically effective and tolerable amount of one or more of the compounds or a salt, stereoisomer or a salt of a stereoisomer thereof, according to any of the claims 1 to 11.

17. A combination comprising
a first active ingredient, which is at least one compound or a salt, stereoisomer or a salt of a stereoisomer thereof, according to any of the claims 1 to 11, and
a second active ingredient, which is at least one anti-cancer agent.

18. The combination according to claim 17, in which said anti-cancer agent is selected from (i) kinase inhibitors; (ii) proteasome inhibitors; (iii) histone deacetylase inhibitors; (iv) heat shock protein 90 inhibitors; (v) vascular targeting agents (VAT), and anti-angiogenic drugs and KDR tyrosine kinase inhibitors; (vi) monoclonal antibodies as well as mutants and conjugates of monoclonal antibodies, and antibody fragments; (vii) oligonucleotide based therapeutics; (viii) Toll-like receptor / TLR 9 agonists, TLR 7 agonists, or TLR 7/8 agonists; (ix) protease inhibitors; (x) hormonal therapeutics;
bleomycin; retinoids; DNA methyltransferase inhibitors; alanosine; cytokines; interferons; and death receptor agonists.

19. The method according to claim 16, in which said cancer is selected from the group consisting of
cancer of the breast, bladder, bone, brain, central and peripheral nervous system, colon, endocrine glands, esophagus, endometrium, germ cells, head and neck, kidney, liver, lung, larynx and hypopharynx, mesothelioma, sarcoma, ovary, pancreas, prostate, rectum, renal, small intestine, soft tissue, testis, stomach, skin, ureter, vagina and vulva;
inherited cancers, retinomblastoma and Wilms tumor;
leukemia, lymphoma, non-Hodgkins disease, chronic and acute myeloid leukaemia, acute lymphoblastic leukemia, Hodgkins disease, multiple myeloma and T-cell lymphoma;
myelodysplastic syndrome, plasma cell neoplasia, paraneoplastic syndromes, cancers of unknown primary site and AIDS related malignancies.
